# EUROPEAN PATENT APPLICATION

(11) **EP 3 418 351 A1**
(43) Date of publication of application: **26.12.2018**
(21) Application number: 17753033.4
(22) Date of filing: 07.02.2017
(51) Int. Cl.: C10M 105/70, C07C 215/40, C07D 207/06, C07D 233/60, C07D 295/088, C07D 487/04, C10M 105/72, G11B 5/725, C10N 20/00, C10N 30/06, C10N 40/18

(54) **IONIC LIQUID, LUBRICANT, AND MAGNETIC RECORDING MEDIUM**

(30) Priority: 15.02.2016 JP 2016026086; 23.03.2016 JP 2016057813; 18.05.2016 JP 2016099590
(71) Applicant: Dexerials Corporation, Tokyo 141-0032 (JP)
(72) Inventor: KONDO, Hirofumi, Tokyo 141-0032 (JP); HATSUDA, Kouki, Tokyo 141-0032 (JP); TANO, Nobuo, Tokyo 141-0032 (JP); BAGHEL, Pankaj, Tokyo 141-0032 (JP)
(74) Representative: Graf von Stosch Patentanwaltsgesellschaft mbH
(86) International application number: PCT/JP2017/004451
(87) International publication number: WO 2017/141775

(57) **Abstract**

A lubricant including an ionic liquid including a conjugate base and a conjugate acid, wherein the conjugate acid includes a group including a hydroxyl group and a group including a straight-chain hydrocarbon group having 6 or greater carbon atoms, and a pKa of an acid that is a source of the conjugate base in acetonitrile is 10 or less.

## Description

### Technical Field

The present invention relates to an ionic liquid, a lubricant including the ionic liquid, and a magnetic recording medium using the lubricant.

### Background Art

Conventionally, in a thin film magnetic recording medium, a lubricant is applied onto a surface of a magnetic layer for the purpose of reducing frictions between a magnetic head and the surface of the magnetic recording medium, or reducing abrasion. In order to avoid adhesion, such as sticktion, an actual film thickness of the lubricant is of a molecular order. Accordingly, it is not exaggeration to say that the most important thing for a thin film magnetic recording medium is to select a lubricant having excellent abrasion resistance in any environment.

During a life of a magnetic recording medium, it is important that a lubricant is present on a surface of the medium without causing desorption, spin-off, and chemical deteriorations. Making the lubricant present on a surface of a medium is more difficult, as the surface of the thin film magnetic recording medium is smoother. This is because the thin film magnetic recording medium does not have an ability of replenishing a lubricant as with a coating-type magnetic recording medium.

In the case where an adhesion force between a lubricant and a protective film disposed at a surface of a magnetic layer is weak, moreover, a film thickness of the lubricant is reduced during heating or sliding hence accelerating abrasion. Therefore, a large amount of the lubricant is required. The large amount of the lubricant is the mobile lubricant, and therefore a function of replenishing the lost lubricant can be provided. However, an excessive amount of the lubricant makes the film thickness of the lubricant larger than the surface roughness. Therefore, a problem associated with adhesion arises, and in a crucial case, sticktion arises to cause driving failures.

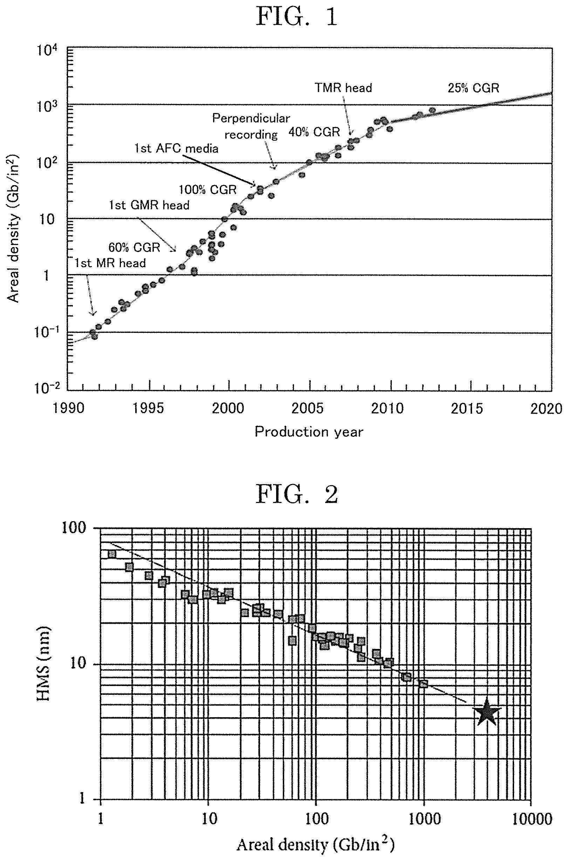

As illustrated in FIG. 1, moreover, an increase rate of an areal density of a hard disk drive of a product has been decreased in the last several years in NPL 1, but the year rate of 25% has been achieved. The areal density is nearly reaching 4 Tb/in², which is one of the targets. It can be found that a distance of a head-disk interface relative to an increase of the recording density has been reduced as illustrated in FIG. 2. There is always a need for improving reliability with the decrease in the head-disk interface distance, which has been described in NPLs 2 to 4.

A current recording density is about 1 Tb/in², spacing is about 6 nm, a thickness of a lubricant is 0.8 nm, and the thickness of the lubricant needs to be reduced for a future recording density of 4 Tb/in². A molecular weight of a conventional PFPE lubricant needs to be made small in order to reduce a film thickness of the lubricant, but thermal stability may be deteriorated when the molecular weight is small. It can be understood that the problems in reliability cannot be sufficiently solved with a conventional perfluoropolyether (PFPE)-based lubricant.

Particularly for a thin film magnetic recording medium having high surface smoothness, a novel lubricant is designed at a molecular level, and synthesized to solve the above-described trade-off. Moreover, there are a number of reports regarding lubricity of PFPE. As described, lubricants are very important in magnetic recording media.

Chemical structures of typical PFPE-based lubricants are depicted in Table 1.

**Table 1**

| Fomblin-based lubricants | |
|---|---|
| | X-CF₂(OCF₂CF₂)ₙ(OCF₂)ₘOCF₂-X(0.5<n/m<1) |
| Z | X=-OCF₃ |
| Z-DOL | X=-CH₂OH |
| Z-DIAC | X=-COOH |
| Z-Tetraol | 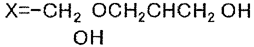 |
| AM2001 | 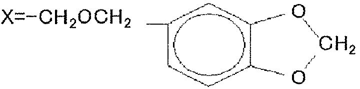 |

| Other lubricants | |
|---|---|
| A20H | 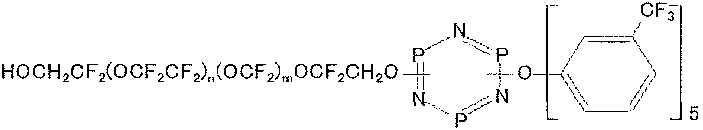 |
| Mono | F-(CF₂CF₂CF₂O)₁-CF₂CF₂CH₂-N(C₃H₇)₂ |

Z-DOL in Table 1 is one of lubricants typically used for thin-film magnetic recording media. Moreover, Z-tetraol (ZTMD) is a lubricant, in which a functional hydroxyl group is further introduced into a main chain of PFPE, and it has been reported that use of Z-tetraol enhances reliability of a drive while reducing a space at an interface between a head and a medium. It has been reported that A20H suppresses decomposition of the PFPE main chain with Lewis acid or Lewis base, and improves tribological properties. On the other hand, it has been reported that Mono has a different polymer main chain and different polar groups to those of the PFPE, and the polymer main chain and polar groups of Mono are respectively poly-n-propyloxy, and amine, and Mono reduces adhesion interactions at near contact.

However, a typical solid lubricant, which has a high melting point and is considered thermally stable, disturbs an electromagnetic conversion process that is extremely highly sensitive, and moreover, an abrasion powder scraped by a head is generated on a running track. Therefore, abrasion properties are deteriorated. As described above, the liquid lubricant has mobility that enables to move the adjacent lubricant layer to replenish the lubricant removed due to abrasion by the head. However, the lubricant is span-off from a surface of the disk especially at a high temperature during driving of the disk, because of the mobility of the lubricant, and thus the lubricant is reduced. As a result, a protection function is lost. Accordingly, a lubricant having a high viscosity and low volatility is suitably used, and use of such a lubricant enables to prolong a service life of a disk drive with suppressing an evaporation rate.

Considering the above-described lubricating systems, requirements for a low-friction and low-abrasion lubricant used for thin film magnetic recording media are as follows.
(1) Low volatility.
(2) Low surface tension for a surface filling function.
(3) Interaction between terminal polar groups and a surface of a disk.
(4) High thermal and oxidization stability in order to avoid decomposition or reduction over a service period.
(5) Chemically inactive with metals, glass, and polymers, and no abrasion powder generated from a head or a guide.
(6) No toxicity and no flammability.
(7) Excellent boundary lubricating properties.
(8) Soluble with organic solvents.

Recently, an ionic liquid has been attracted attentions as one of solvents for synthesis of organic or inorganic materials and being friendly to the environments in the fields of electricity storage materials, a separation technology, and a catalyst technology. The ionic liquid is roughly classified as a molten salt having a low melting point. The ionic liquid is typically a molten salt having a melting point of 100°C or lower, among the above-mentioned molten salts. The important properties of the ionic liquid used as a lubricant are low volatility, inflammability, thermal stability, and an excellent dissolving performance.

For example, abrasion and wear of a surface of a metal or ceramic may be reduced by using a certain ionic liquid compared to a conventional hydrocarbon-based lubricant. For example, there is a report that, in the case where an imidazole cation-based ionic liquid is synthesized by substituting with a fluoroalkyl group, and tetrafluoroboric acid salt or hexafluorophosphoric acid salt of alkyl imidazolium is used for steel, aluminium, copper, single crystal SiO₂, silicon, or sialon ceramics (Si-Al-O-N), tribological properties more excellent than those of cyclic phosphazene (X-1P) or PFPE are exhibited. Moreover, there is a report that an ammonium-based ionic liquid reduces frictions more than a base oil in the region of elastohydrodynamic to boundary lubrication. Moreover, effects of the ionic liquid as an additive for a base oil have been studied, and a chemical or tribochemical reaction of the ionic liquid has been researched to understand lubricating systems. However, there are a few application examples of the ionic liquid to magnetic recording media. For example, NPL 5 reports an ionic liquid of imidazole-based tris(pentafluoroethyl)trifluorophosphate. However, NPL 5 just indicates its potential but does not refer to specific tribological properties.

Among the ionic liquids, a perfluorooctanoic acid alkyl ammonium salt is a protic ionic liquid (PIL), and has been reported as having a significant effect of reducing frictions of magnetic recording media compared to Z-DOL mentioned above (see, for example, PTLs 1 and 2, and NPLs 6 to 8).

However, the above-mentioned perfluorocarboxylic acid ammonium salts have weak interaction between a cation and an anion in the reaction represented by the following reaction formula (A). According to Le Chatelier's principle, the equilibrium of the reaction is sifted to the left side at a high temperature, and the perfluorocarboxylic acid ammonium salt becomes a dissociated neutral compound and hence thermal stability is deteriorated. Specifically, protons are transferred at a high temperature, the equilibrium is sifted to neutral substance and dissociation occurs (see, for example, NPL 9). In other words, thermal stability at a high temperature will be degraded.

CₙF₂ₙ₊₁COOH + CₙF₂ₙ₊₁NH₂ ⇄ CₙF₂ₙ₊₁COO⁻H₃N⁺CₙH₂ₙ₊₁ (A)

Meanwhile, the limit of a surface recording density of a hard disk is said to be from 1 Tb/in² to 2.5 Tb/in². Currently, the surface recording density is getting close to the limit, but developments of technology for increasing capacities have been actively conducted with a reduction in particle size of magnetic particles as a premise. As the technology for increasing capacities, there are a reduction in an effective flying height and introduction of Shingle Write (BMP).

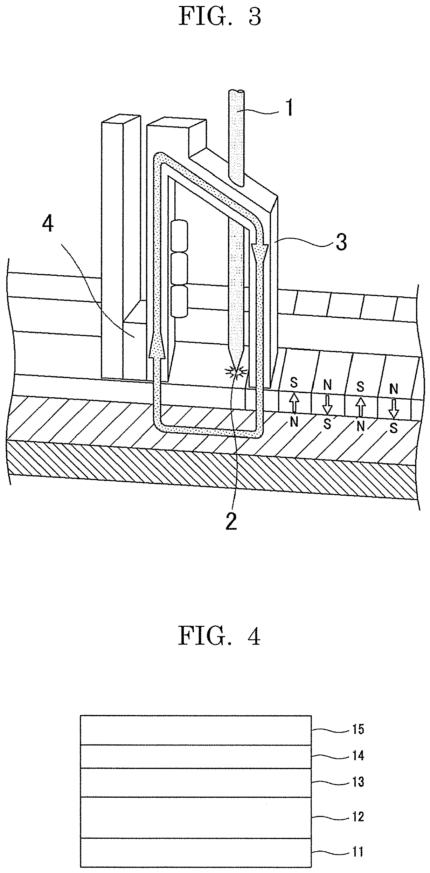

Moreover, there is "thermally-assisted magnetic recording (heat assisted magnetic recording)" as the next-generation recording technology. The thermally-assisted magnetic recording is schematically illustrated in FIG. 3. In FIG. 3, the referential numeral 1 is laser light, the referential numeral 2 is near field light, the referential numeral 3 is a recording head (PMR element), and the referential numeral 4 is a reproducing head (TMR element). The problems of the thermally-assisted magnetic recording include a deterioration of durability due to evaporation or deterioration of a lubricant on a surface of a magnetic layer because a recording area is heated by laser during recording and reproducing. Even though it is a short period, there is a possibility that a thin film magnetic recording medium is exposed to a high temperature, which is 400°C or higher, in thermally-assisted magnetic recording. Therefore, thermal stability of a lubricant generally used for thin film magnetic recording media, such as Z-DOL and Z-TETRAOL, is concerned about.

A protic ionic liquid forms ions as described above, and is typically a material having high thermal stability. The equilibrium of the protic ionic liquid is represented by Scheme 1 below.

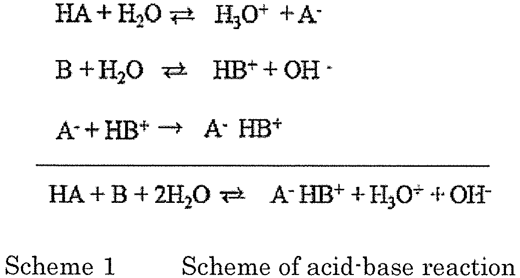

In Scheme 1, HA is Bronsted acid and B is Bronsted base. The acid (HA) and the base (B) are reacted as in Scheme 1 to generate a salt (A⁻HB⁺).

In Scheme 1, a dissociation constant Kₐ₁ of the acid and a dissociation constant K_{b2} of the base can be represented by Scheme 2 below in the form including the density.

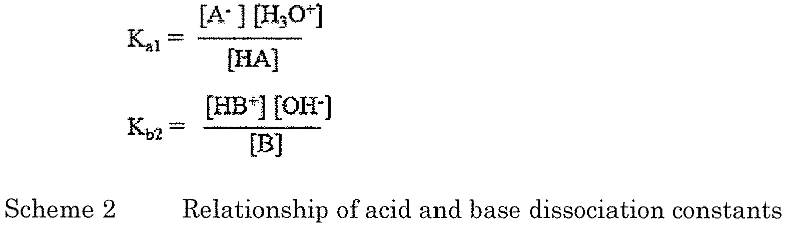

The Kₐ₁ and K_{b2} largely differ depending on a substance. In some cases, the Kₐ₁ and K_{b2} may be large digits, which is inconvenient for handling. Therefore, it is often represented with a negative logarithm. Specifically, the acid dissociation constant is determined as -log₁₀ Kₐ₁ = pKₐ₁ as represented by Scheme 3 below. Clearly, acidity is stronger, as the pKₐ₁ is smaller.

A difference ΔpKa of the acid dissociation constants of the acid and the base is discussed. The acid-base reaction is influenced by both acidity of the acid and basicity of the base (or acidity of conjugate acid of the base), and the difference ΔpKa in the acidity of the acid and base is represented by Scheme 3 below.

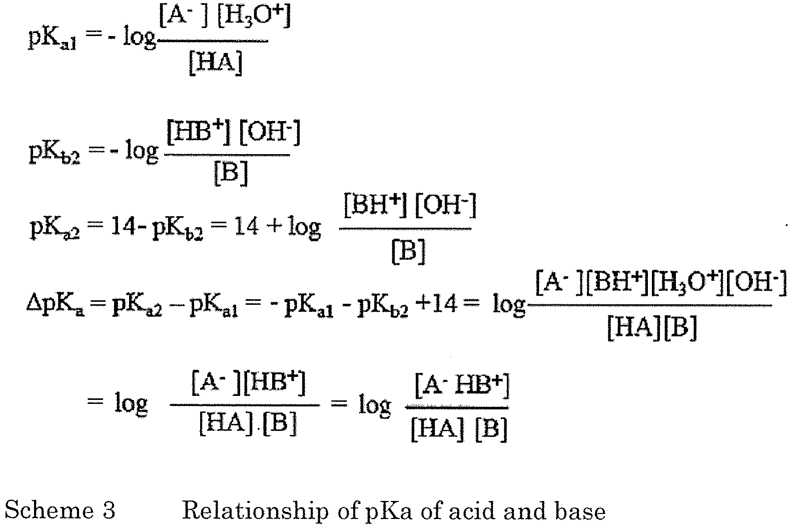

It is indicated that the ΔpKa increases, as the base concentration [A⁻HB⁺] increases relative to the acid concentration and the base concentration.

Meanwhile, Yoshizawa et al. have reported that proton transfer tends to occur when a difference (ΔpKa) of pKa of acid and base is 10 or greater,

[AH]+[B]↔[A⁻HB⁺]

the equilibrium of the formula above is sifted to the ion side (right side), and stability is enhanced further (see, for example, NPL 9). Moreover, Watanabe et al. has reported that proton transfer and thermal stability of a protic ionic liquid largely depend on ΔpKa, and thermal stability of the ionic liquid is significantly improved by using the acid with which the difference in pKa between the acid and the base (ΔpKa) is 15 or greater when DBU (1,8-diazabicyclo[5,4,0]undec-7-ene) is used as a base (see, for example, NPL 10). Moreover, Kondo et al. have reported that a perfluorooctanesulfonic acid octadecyl ammonium salt-based protic ionic liquid having large ΔpKa improves durability of a magnetic recording medium (see, for example, NPL 11 and PTL 3). In the recent report of Kondo et al. related to thermal resistance of an ionic liquid, it has been reported that a decomposition temperature increases with up to certain degree of ΔpKa, and the decomposition temperature is not increased any further even when ΔpKa is increased from the above-described point (see, for example, NPLs 12 and 13). Moreover, it is reported that a pyrrolidinium-based ionic liquid including germinal dication may improve thermal resistance more than a typical ionic liquid of monocation (see NPL 13). As described in NPL 14, however, a relationship between a molecular structure constituting the pyrrolidinium-based ionic liquid and physical or chemical characteristics has not been fully understood yet. A combination of a cation and an anion largely influences on physical or chemical characteristics of an ionic liquid. A variety of the anion site is many, but the relationship could not be clarified unless the cation is a cation structurally similar to the anion (see, for example, NPL 15). For example, viscosity of the liquid increases, as hydrogen bonding strength of halogen is stronger (Cl > Br > I). However, the method for increasing the viscosity is not limited to the increase in the hydrogen bonding strength. For example, the viscosity can be increased by varying an alkyl chain of imidazole. Similarly, the combination of the anion and cation influences on a melting point, surface tension, and thermal stability, but a wide range of researches has not be conducted on an effect of the anion. Accordingly, it is possible to change physical or chemical characteristics of an ionic liquid by with a combination of cations or anions, but it is difficult to predict.

In the case where the lubricants for hard disks presented in Table 1 are considered, a polar group such as a hydroxyl group is introduced to their end for increasing interactions with the media surface. Such a hydroxyl group is reacted with and fixed on the media surface by a heating treatment, whereby thermal stability is improved. Moreover, there is also an effect of reducing the polar term component of the surface energy through binding of the hydroxyl group (NPL 16).

Also, ionic liquid-type lubricants are preferably lower in melting point when considering other applications than the applications in hard discs.

### Citation List

### Patent Literatures

PTL 1: Japanese Patent No. 2581090
PTL 2: Japanese Patent No. 2629725
PTL 3: International Publication No. WO 2014/104342

### Non-Patent Literatures

NPL 1: Advances in Tribology Volume 2013, ArticleID 521086
NPL 2: C. M. Mate, Q. Dai, R. N. Payne, B. E. Knigge, and P. Baumgart, "Will the numbers add up for sub-7-nm magnetic spacings? Futuremetrology issues for disk drive lubricants, overcoats, and topographies," IEEE Transactions onMagnetics, vol. 41, no. 2, pp. 626-631, 2005.
NPL 3: B. Marchon and T. Olson, "Magnetic spacing trends: from LMR to PMR and beyond," IEEE Transactions on Magnetics, vol. 45, no. 10, pp. 3608-3611, 2009.
NPL 4: J. Gui, "Tribology challenges for head-disk interface toward 1Tb/in2," IEEE Transactions on Magnetics, vol. 39, no. 2, pp. 716-721, 2003.
NPL 5: Gong, X., Kozbial, A., Rose, F., Li, L.,Effect of π-π+ Stacking on the Layering of Ionic Liquids Confined to an Amorphous Carbon Surface., Applied Mater. Interfaces 2015, vol. 7, pp. 7078-7081
NPL 6: Kondo, H., Seto, J., Haga. S., Ozawa, K., (1989) Novel Lubricants for Magnetic Thin Film Media, Magnetic Soc. Japan, Vol. 13, Suppl. No. S1, pp. 213-218
NPL 7: Kondo, H., Seki, A., Watanabe, H., & Seto, J., (1990). Frictional Properties of Novel Lubricants for Magnetic Thin Film Media, IEEE Trans. Magn. Vol. 26, No. 5, (Sep. 1990), pp. 2691-2693, ISSN:0018-9464
NPL 8: Kondo, H., Seki, A., & Kita, A., (1994a). Comparison of an Amide and Amine Salt as Friction Modifiers for a Magnetic Thin Film Medium. Tribology Trans. Vol. 37, No. 1, (Jan. 1994), pp. 99-105, ISSN: 0569-8197
NPL 9: Yoshizawa, M., Xu, W., Angell, C. A., Ionic Liquids by Proton Transfer: Vapor pressure, Conductivity, and the Relevance of ΔpKa from Aqueous Solutions, J. Am. Chem. Soc., Vol. 125, pp. 15411-15419 (2003)
NPL 10: Miran, M.S., Kinoshita, H., Yasuda, T., Susan, M.A.B.H., Watanabe, M., Physicochemical Properties Determined by ΔpKa for Protic Ionic Liquids Based on an Organic Super-strong Base with Various Bronsted Acids, Phys. Chem. Chem. Phys., Vol 14, pp. 5178-5186 (2012)
NPL 11: Hirofumi Kondo, Makiya Ito, Koki Hatsuda, KyungSung Yun and Masayoshi Watanabe, "Novel Ionic Lubricants for Magnetic Thin Film" Media,IEEE TRANSACTIONS ON MAGNETICS, VOL. 49, NO. 7, pp. 3756-3759, JULY (2013)
NPL 12: Hirofumi Kondo, Makiya Ito, Koki Hatsuda, Nobuo Tano, KyungSung Yun and Masayoshi Watanabe, IEEE International magnetic conference Dresden, Germany, May 4-8, 2014
NPL 13: Hirofumi Kondo, Makiya Ito, Koki Hatsuda, Nobuo Tano, KyungSung Yun and Masayoshi Watanabe, IEEE Trans. Magn., 2014, Vol. 50, Issue 11, Article#: 3302504
NPL 14: Anderson, J. L., Ding R., Ellern A., Armstrong D. W., "Structure and Properties of High Stability Geminal DicationicIonic Liquids", J. Am. Chem. Soc., 2005, 127, 593-604.
NPL 15: Dzyuba, S. V.; Bartsch, R. A. ,"Influence of Structural Variations in 1-Alkyl(aralkyl)-3-Methylimidazolium Hexafluorophosphates and Bis(trifluoromethylsulfonyl)imides on Physical Properties of the Ionic Liquids, Chem. Phys. Phys. Chem. 2002, 3, 161-166
NPL 16: R.J. Waltman, D. J. Pocker, G.W. Tyndall, Studies on the interactions between ZDOL perfluoropolyether lubricant and the carbon overcoat of rigid magnetic media ,Tribology Letters 1998, Volume 4, Issue 3, pp 267-275

### Summary of Invention

### Technical Problem

The present invention has been proposed based on the above-described situations in the art, and the present invention provides an ionic liquid having excellent lubricity even at a high temperature and being usable for many applications because of being able to be lowered in melting point, a lubricant having excellent lubricity even at a high temperature and being usable for many applications because of being able to be lowered in melting point, and a magnetic recording medium having excellent practical properties.

### Solution to Problem

<1> A lubricant including:
   an ionic liquid including a conjugate base and a conjugate acid,
   wherein the conjugate acid includes a group including a hydroxyl group and a group including a straight-chain hydrocarbon group having 6 or greater carbon atoms, and a pKa of an acid that is a source of the conjugate base in acetonitrile is 10 or less.
<2> The lubricant according to <1>, wherein the conjugate acid is represented by General Formula (A) below, General Formula (B) below, General Formula (C) below, or General Formula (D) below: 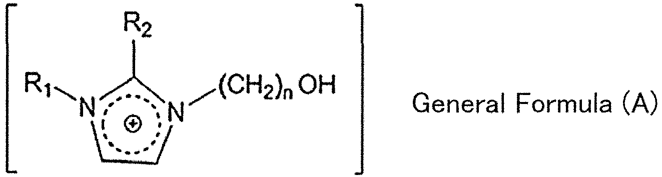 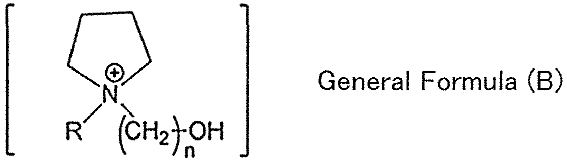 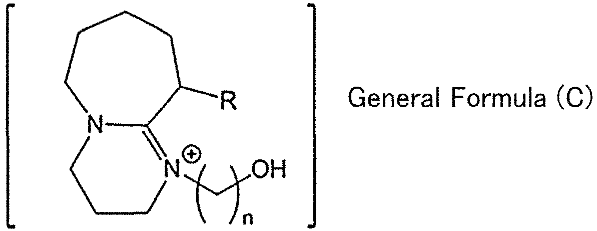 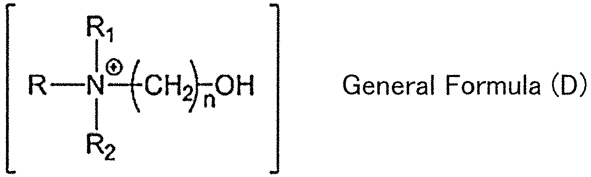 where in the General Formula (A), R₁ and R₂ each independently represent a hydrogen atom or a group including a straight-chain hydrocarbon group having 6 or greater carbon atoms, n is an integer of 1 or greater, and at least one of R₁ and R₂ is a group including a straight-chain hydrocarbon group having 6 or greater carbon atoms,
   in the General Formula (B), R represents a group including a straight-chain hydrocarbon group having 6 or greater carbon atoms, and n is an integer of 1 or greater,
   in the General Formula (C), R represents a group including a straight-chain hydrocarbon group having 6 or greater carbon atoms, and n is an integer of 1 or greater, and
   in the General Formula (D), R represents a group including a straight-chain hydrocarbon group having 6 or greater carbon atoms, R₁ and R₂ each independently represent a hydrogen atom or a hydrocarbon group, and n is an integer of 1 or greater.
<3> The lubricant according to <1> or <2>, wherein the conjugate base is represented by General Formula (X) below or General Formula (Y) below:

   CₗF₂ₗ₊₁-SO₃^{Θ} General Formula (X)

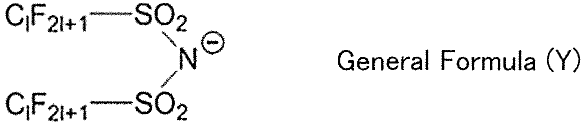

   where in the General Formula (X), l is an integer of 1 or greater but 12 or less, and
   in the General Formula (Y), l is an integer of 1 or greater but 12 or less.
<4> A magnetic recording medium including:
   a non-magnetic support;
   a magnetic layer disposed on the non-magnetic support; and
   the lubricant according to any one of <1> to <3>, disposed on the magnetic layer.
<5> An ionic liquid including:
   a conjugate base; and
   a conjugate acid,
   wherein the conjugate acid includes a group including a hydroxyl group and a group including a straight-chain hydrocarbon group having 6 or greater carbon atoms, and a pKa of an acid that is a source of the conjugate base in acetonitrile is 10 or less.
<6> The ionic liquid according to <5>, wherein the conjugate acid is represented by General Formula (A) below, General Formula (B) below, General Formula (C) below, or General Formula (D) below: 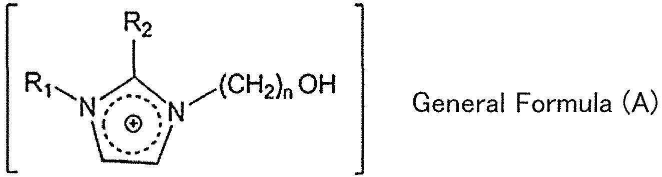 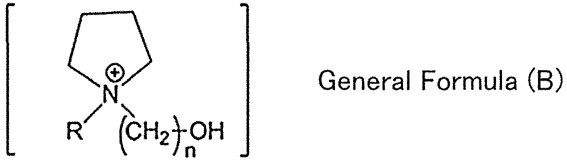 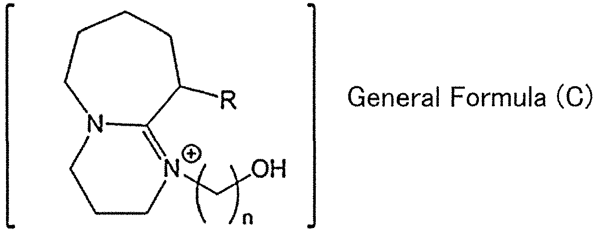 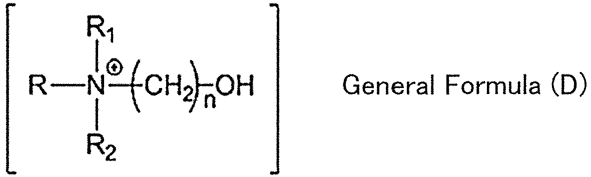 where in the General Formula (A), R₁ and R₂ each independently represent a hydrogen atom or a group including a straight-chain hydrocarbon group having 6 or greater carbon atoms, n is an integer of 1 or greater, and at least one of R₁ and R₂ is a group including a straight-chain hydrocarbon group having 6 or greater carbon atoms,
   in the General Formula (B), R represents a group including a straight-chain hydrocarbon group having 6 or greater carbon atoms, and n is an integer of 1 or greater,
   in the General Formula (C), R represents a group including a straight-chain hydrocarbon group having 6 or greater carbon atoms, and n is an integer of 1 or greater, and
   in the General Formula (D), R represents a group including a straight-chain hydrocarbon group having 6 or greater carbon atoms, R₁ and R₂ each independently represent a hydrogen atom or a hydrocarbon group, and n is an integer of 1 or greater.
<7> The ionic liquid according to <5> or <6>, wherein the conjugate base is represented by General Formula (X) below or General Formula (Y) below:

   CₗF₂ₗ₊₁-SO₃^{Θ} General Formula (X)

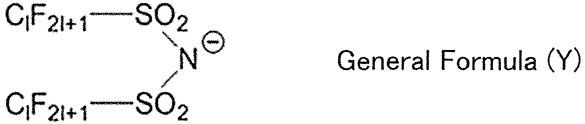

   where in the General Formula (X), 1 is an integer of 1 or greater but 12 or less, and
   in the General Formula (Y), 1 is an integer of 1 or greater but 12 or less.

### Advantageous Effects of Invention

According to the present invention, it is possible to provide an ionic liquid having excellent lubricity even at a high temperature and being usable for many applications because of being able to be lowered in melting point, a lubricant having excellent lubricity even at a high temperature and being usable for many applications because of being able to be lowered in melting point, and a magnetic recording medium having excellent practical properties.

### Brief Description of Drawings

FIG. 1 is a graph depicting transition and prediction of an areal recording density of a hard disk drive.
FIG. 2 is a roadmap of head-medium spacing (HMS) relative to an areal recording density of a hard disk.
FIG. 3 is a schematic view illustrating thermally-assisted magnetic recording.
FIG. 4 is a cross-sectional view illustrating one example of a hard disk according to one aspect of the present invention.
FIG. 5 is a cross-sectional view illustrating one example of a magnetic tape according to one aspect of the present invention.

### Description of Embodiments

Embodiments of the present invention are specifically described with reference to drawing hereinafter in the following order.
1. Lubricant and ionic liquid
2. Magnetic recording medium
3. Examples

### <1. Lubricant and ionic liquid>

The lubricant, which is one embodiment of the present invention, includes an ionic liquid including a conjugate acid and a conjugate base.

The ionic liquid, which is one embodiment of the present invention, includes a conjugate acid and a conjugate base.

In the ionic liquid, the conjugate acid includes a group including a hydrocarbon group. The hydrocarbon group is a straight-chain hydrocarbon group having 6 or greater carbon atoms. Here, the "straight-chain hydrocarbon group having 6 or greater carbon atoms" may be a partially fluorinated hydrocarbon group obtained by substituting part of the hydrogen atoms bonded to carbon with a fluorine atom.

In the ionic liquid, the conjugate acid includes a group including a hydroxyl group.

In the ionic liquid, a pKa of an acid that is a source of the conjugate base in acetonitrile is 10 or less.

Since the ionic liquid of the present embodiment includes a conjugate acid a conjugate base, and a pKa of an acid that is a source of the conjugate base in acetonitrile is 10 or less, excellent thermal stability can be exhibited. Since the cation site has a group including a hydrocarbon group having 6 or greater carbon atoms, moreover, excellent lubricity can be obtained in combination with the thermal stability. Also, since a hydroxyl group is introduced to the cation site, thermal resistance and solubility to a fluorine-based solvent are improved, and the melting point is decreased. Some of the compounds are improved in solubility to CF₃(CHF)₂CF₂CF₃ that is often used as a fluorine-based solvent in a lubricant applying step of hard discs, and as a result, production lines of magnetic recording media do not need to be explosion proof.

Here, a lubricant containing an ionic liquid may be used in a state where the concentration of the ionic liquid is about 0.05% by mass. Therefore, solubility of the ionic liquid to fluorine-based solvents is preferably 0.05% by mass or more. Also, depending on the situations where it is used, more solubility may be required. Furthermore, considering, for example, changes in use situations and storage conditions, solubility of 0.1% by mass or more may be required [i.e., the ionic liquid is 0.1 parts by mass or more relative to 100 parts by mass of CF₃(CHF)₂CF₂CF₃].

The pKa is 10 or less, which is a strong acid, and is preferably 6.0 or less.

The lower limit of the pKa is not particularly limited and may be appropriately selected depending on the intended purpose, but the pKa is preferably -5.0 or greater.

In the present specification, "pKa" is an acid dissociation constant, and is an acid dissociation constant in acetonitrile.

### <<Conjugate base>>

The conjugate base is not particularly limited and may be appropriately selected depending on the intended purpose, as long as pKa of an acid that is a source of the conjugate base in acetonitrile is 10 or less. Examples of the conjugate base include a conjugate base represented by General Formula (X) below, a conjugate base represented by General Formula (Y) below, a conjugate base represented by General Formula (U) below, a conjugate base represented by General Formula (V) below, and a conjugate base represented by General Formula (W) below. Among the above-listed examples, a conjugate base represented by General Formula (X) below and a conjugate base represented by General Formula (Y) below are preferable because solubility of the ionic liquid to a solvent becomes high.

CₗF₂ₗ₊₁-SO₃^{Θ} General Formula (X)

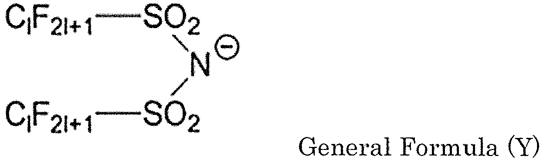

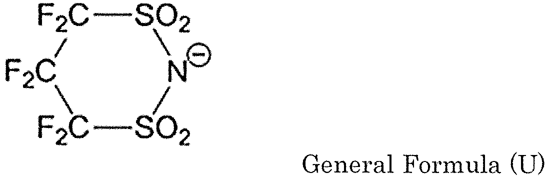

^{Θ}C(CN)₃ General Formula (V)

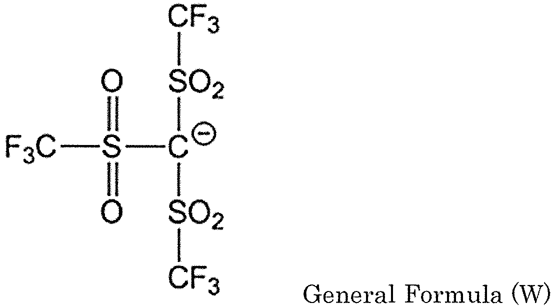

In General Formula (X), 1 is an integer of 1 or greater but 12 or less, and is preferably an integer of 1 or greater but 6 or less.

In General Formula (Y), 1 is an integer of 1 or greater but 12 or less, and is preferably an integer of 1 or greater but 6 or less.

As the acid that is a source of the conjugate base (HA), Bronsted acids regarded as super acid, such as bis((perfluoroalkyl)sulfonyl)imide [(CₗF₂ₗ₊₁SO₂)₂NH] (pKa = 0 to 0.3), perfluorocyclopropane sulfoimide (pKa = -0.8), perfluoroalkyl sulfonic acid (CₘF₂ₘ₊₁SO₃H) (pKa = 0.7), tris(perfluoroalkanesulfonyl)methide compounds [(CF₃SO₂)₃CH] (pKa = -3.7), tricyanomethane (pKa = 5.1), inorganic acids [e.g., nitric acid (pKa = 9.4) and sulfuric acid (pKa = 8.7)], tetrafluoroboric acid (pKa = 1.8), and hexafluorophosphate, are preferable. The pKa of the above-listed acids are introduced, for example, in J. Org. Chem. Vol. 76, No. 2, p. 394.

### <<Conjugate acid>>

The conjugate acid includes a group including a straight-chain hydrocarbon group having 6 or greater carbon atoms, and further includes a group including a hydroxyl group.

A method for introducing a hydroxyl group to the conjugate acid is not particularly limited. An exemplary method is introducing it via a straight-chain alkylene chain. One example of this method is a method of alkylating the nitrogen atom of the conjugate acid using a halide, as described in the following Examples. This introduction method is totally different from the introduction methods for Z-DOL and Z-Tetraol, which are general perfluoropolyether-based lubricants. Therefore, these methods are not referred to in any case.

The conjugate acid includes a group including a straight-chain hydrocarbon group having 6 or greater carbon atoms.

The number of carbon atoms in the hydrocarbon group is not particularly limited and may be appropriately selected depending on the intended purpose as long as it is 6 or greater. It is preferably 10 or greater.

The upper limit of the number of carbon atoms in the straight-chain hydrocarbon group having 6 or greater carbon atoms is not particularly limited and may be appropriately selected depending on the intended purpose. The number of carbon atoms is preferably 30 or less, more preferably 25 or less, and particularly preferably 20 or less, in view of availability of raw materials. When the hydrocarbon group has a long chain, lubricity can be improved, with reducing a coefficient of friction.

The group including a straight-chain hydrocarbon group having 6 or greater carbon atoms is preferably a straight-chain hydrocarbon group having 6 or greater carbon atoms.

Solubility to a solvent tends to decrease if they have too many carbon atoms. Thus, the number of carbon atoms in the hydrocarbon group is determined in consideration of the effect of reducing a coefficient of friction and solubility to a solvent.

The hydrocarbon group is not limited as long as it is a straight chain. The hydrocarbon group may be a saturated hydrocarbon group, an unsaturated hydrocarbon group having a double bond at part, or an unsaturated branched hydrocarbon group having a branched chain at part. Among the above-listed examples, the hydrocarbon group is preferably an alkyl group that is a saturated hydrocarbon group in view of abrasion resistance. Moreover, the hydrocarbon group is also preferably a straight-chain hydrocarbon group that does not have a branched chain at any part. Needless to say, the hydrocarbon group may be a hydrocarbon group that has a branched chain at part.

The hydrocarbon group is, for example, a group represented by General Formula (I) below or a group represented by General Formula (II) below.

-(CH₂)ₗ-CH₃ General Formula (I)

-(CH₂)ₘ-(CF₂)ₙ-CF₃ General Formula (II)

In the General Formula (I), 1 is an integer of 5 or greater, preferably an integer of 9 or greater but 29 or less, further preferably an integer of 9 or greater but 24 or less, particularly preferably an integer of 9 or greater but 19 or less.

In the General Formula (II), m is an integer of 1 or greater but 6 or less and n is an integer of 3 or greater but 20 or less, where m+n is 7 or greater, m is preferably an integer of 1 or greater but 3 or less and n is preferably an integer of 5 or greater but 10 or less.

The conjugate acid includes a group including a hydroxyl group. The group including a hydroxyl group is, for example, a group represented by General Formula (IV) below.

-(CH₂)ₙ-OH General Formula (IV)

In the General Formula (IV), n is an integer of 1 or greater, preferably an integer of 1 or greater but 10 or less, more preferably an integer of 1 or greater but 6 or less.

The conjugate acid is preferably a conjugate acid represented by General Formula (A) below, a conjugate acid represented by General Formula (B) below, a conjugate acid represented by General Formula (C) below, or a conjugate acid represented by General Formula (D) below, from the viewpoints of thermal resistance and lubricity.

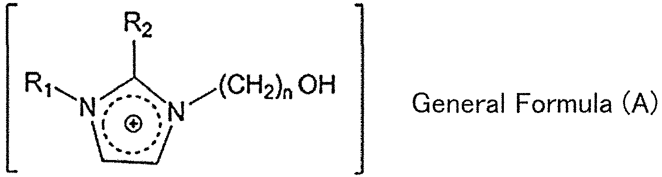

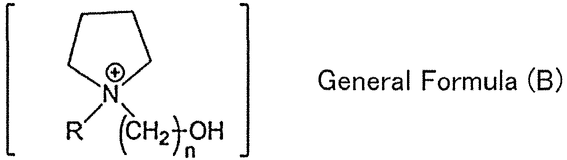

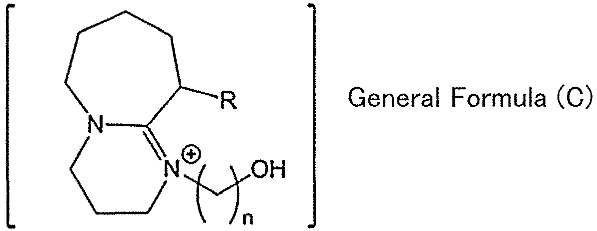

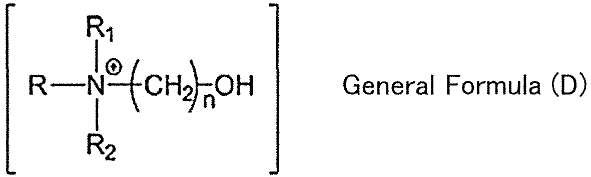

In the General Formula (A), R₁ and R₂ each independently represent a hydrogen atom or a group including a straight-chain hydrocarbon group having 6 or greater carbon atoms, n is an integer of 1 or greater, and at least one of R₁ and R₂ is a group including a straight-chain hydrocarbon group having 6 or greater carbon atoms,
in the General Formula (B), R represents a group including a straight-chain hydrocarbon group having 6 or greater carbon atoms, and n is an integer of 1 or greater,
in the General Formula (C), R represents a group including a straight-chain hydrocarbon group having 6 or greater carbon atoms, and n is an integer of 1 or greater, and
in the General Formula (D), R represents a group including a straight-chain hydrocarbon group having 6 or greater carbon atoms, R₁ and R₂ each independently represent a hydrogen atom or a hydrocarbon group, and n is an integer of 1 or greater.

The number of carbon atoms in the hydrocarbon groups of R₁ and R₂ in the General Formula (A), R in the General Formula (B), R in the General Formula (C), and R in the General Formula (D) is not particularly limited and may be appropriately selected depending on the intended purpose as long as it is 6 or greater. It is preferably 10 or greater.

The upper limit of the number of carbon atoms in the hydrocarbon groups is not particularly limited and may be appropriately selected depending on the intended purpose. The upper limit of the number of carbon atoms therein is preferably 30 or less, more preferably 25 or less, and particularly preferably 20 or less, in view of availability of raw materials. When the hydrocarbon group has a long chain, lubricity can be improved, with reducing a coefficient of friction.

R₁, R₂, and R are preferably a straight-chain hydrocarbon group having 6 or greater carbon atoms.

Solubility to a solvent tends to decrease if they have too many carbon atoms. Thus, the number of carbon atoms in the hydrocarbon group is determined in consideration of the effect of reducing a coefficient of friction and solubility to a solvent.

The hydrocarbon groups of R₁ and R₂ in the General Formula (A), R in the General Formula (B), R in the General Formula (C), and R in the General Formula (D) are not limited as long as the hydrocarbon groups are a straight chain. The hydrocarbon groups may be a saturated hydrocarbon group, an unsaturated hydrocarbon group having a double bond at part, or an unsaturated branched hydrocarbon group having a branched chain at part. Among the above-listed examples, the hydrocarbon group is preferably an alkyl group that is a saturated hydrocarbon group in view of abrasion resistance. Moreover, the hydrocarbon group is also preferably a straight-chain hydrocarbon group that does not have a branched chain at any part.

R₁ and R₂ in the General Formula (A), R in the General Formula (B), R in the General Formula (C), and R in the General Formula (D) are, for example, a group represented by General Formula (III) below.

-(CH₂)ₗ-CH₃ General Formula (III)

In the General Formula (III), 1 is an integer of 5 or greater, preferably an integer of 9 or greater but 29 or less, more preferably an integer of 9 or greater but 19 or less.

When R₁ and R₂ in the General Formula (D) are a hydrocarbon group, the number of carbon atoms in the hydrocarbon group is not particularly limited and may be appropriately selected depending on the intended purpose. The number of carbon atoms in the hydrocarbon group is preferably 1 to 10, more preferably 1 to 6.

The n is an integer of 1 or greater, preferably an integer of 1 or greater but 10 or less, more preferably an integer of 1 or greater but 6 or less.

The conjugate acid represented by the General Formula (A) is, for example, a conjugate acid represented by General Formula (A-1) below.

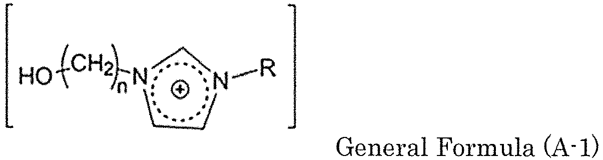

In the General Formula (A-1), R represents a straight-chain hydrocarbon group having 6 or greater carbon atoms, and n is an integer of 1 or greater.

### <<Suitable examples of ionic liquid>>

The ionic liquid is preferably an ionic liquid represented by General Formula (1) below, an ionic liquid represented by General Formula (2) below, an ionic liquid represented by General Formula (3) below, and an ionic liquid represented by General Formula (4) below.

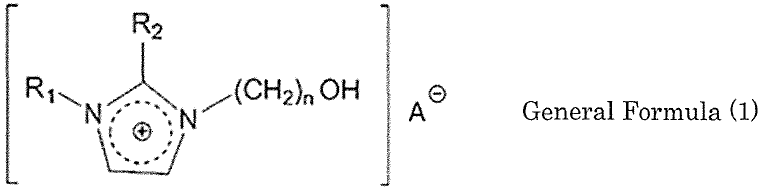

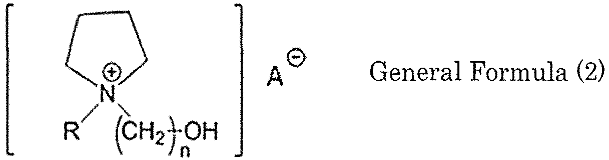

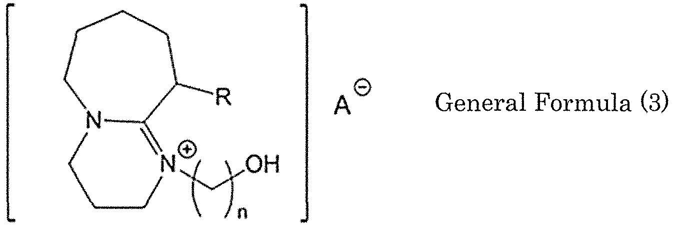

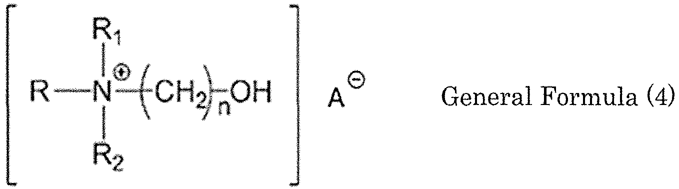

In the General Formula (1), A⁻ represents a conjugate base, R₁ and R₂ each independently represent a hydrogen atom or a group including a straight-chain hydrocarbon group having 6 or greater carbon atoms, n is an integer of 1 or greater, and at least one of R₁ and R₂ is a group including a straight-chain hydrocarbon group having 6 or greater carbon atoms.

In the General Formula (2), A⁻ represents a conjugate base, R represents a group including a straight-chain hydrocarbon group having 6 or greater carbon atoms, and n is an integer of 1 or greater,

In the General Formula (3), A⁻ represents a conjugate base, R represents a group including a straight-chain hydrocarbon group having 6 or greater carbon atoms, and n is an integer of 1 or greater.

In the General Formula (4), A⁻ represents a conjugate base, R represents a group including a straight-chain hydrocarbon group having 6 or greater carbon atoms, R₁ and R₂ each independently represent a hydrogen atom or a hydrocarbon group, and n is an integer of 1 or greater.

The ionic liquid represented by the General Formula (1) is preferably an ionic liquid represented by General Formula (1-1) below and an ionic liquid represented by General Formula (1-2) below.

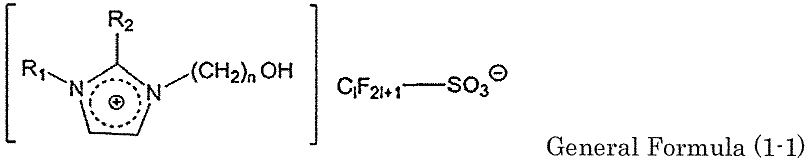

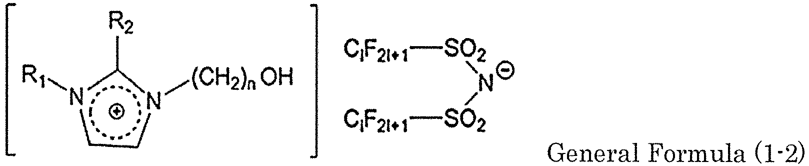

In the General Formula (1-1), R₁ and R₂ each independently represent a hydrogen atom or a group including a straight-chain hydrocarbon group having 6 or greater carbon atoms, n is an integer of 1 or greater, 1 is an integer of 1 or greater but 12 or less, and at least one of R₁ and R₂ is a group including a straight-chain hydrocarbon group having 6 or greater carbon atoms.

In the General Formula (1-2), R₁ and R₂ each independently represent a hydrogen atom or a group including a straight-chain hydrocarbon group having 6 or greater carbon atoms, n is an integer of 1 or greater, 1 is an integer of 1 or greater but 12 or less, and at least one of R₁ and R₂ is a group including a straight-chain hydrocarbon group having 6 or greater carbon atoms.

The ionic liquid represented by the General Formula (2) is preferably an ionic liquid represented by General Formula (2-1) below and an ionic liquid represented by General Formula (2-2) below. Among them, an ionic liquid represented by General Formula (2-2) below is more preferable because it has excellent solubility to a fluorine-based solvent.

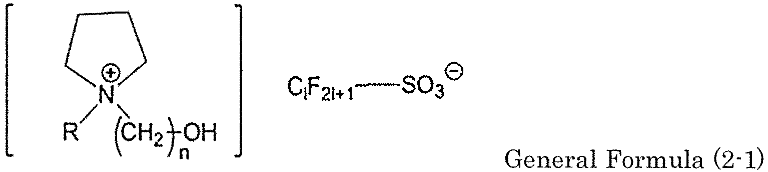

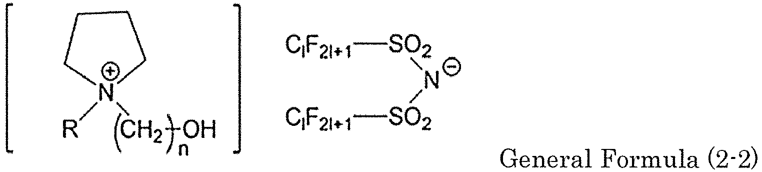

In the General Formula (2-1), R represents a group including a straight-chain hydrocarbon group having 6 or greater carbon atoms, n is an integer of 1 or greater, and 1 is an integer of 1 or greater but 12 or less.

In the General Formula (2-2), R represents a group including a straight-chain hydrocarbon group having 6 or greater carbon atoms, n is an integer of 1 or greater, and 1 is an integer of 1 or greater but 12 or less.

The ionic liquid represented by the General Formula (3) is preferably an ionic liquid represented by General Formula (3-1) below and an ionic liquid represented by General Formula (3-2) below, because they have excellent solubility to a fluorine-based solvent.

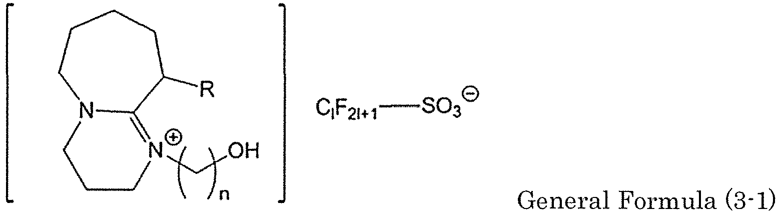

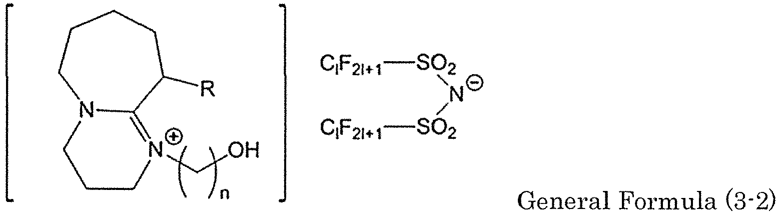

In the General Formula (3-1), R represents a group including a straight-chain hydrocarbon group having 6 or greater carbon atoms, n is an integer of 1 or greater, and 1 is an integer of 1 or greater but 12 or less.

In the General Formula (3-2), R represents a group including a straight-chain hydrocarbon group having 6 or greater carbon atoms, n is an integer of 1 or greater, and 1 is an integer of 1 or greater but 12 or less.

The ionic liquid represented by the General Formula (4) is preferably an ionic liquid represented by General Formula (4-1) below and an ionic liquid represented by General Formula (4-2) below, because they have excellent solubility to a fluorine-based solvent.

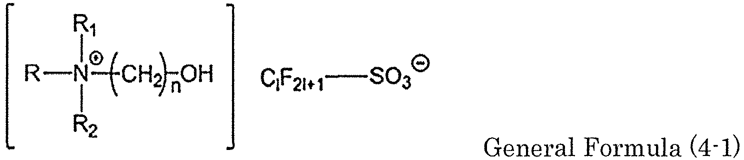

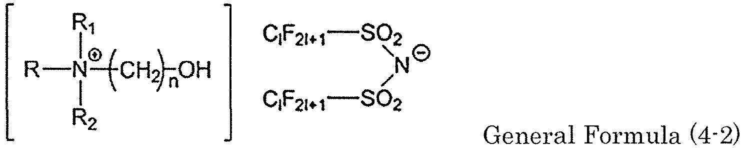

In the General Formula (4-1), R represents a group including a straight-chain hydrocarbon group having 6 or greater carbon atoms, R₁ and R₂ each independently represent a hydrogen atom or a hydrocarbon group, n is an integer of 1 or greater, and 1 is an integer of 1 or greater but 12 or less.

In the General Formula (4-2), R represents a group including a straight-chain hydrocarbon group having 6 or greater carbon atoms, R₁ and R₂ each independently represent a hydrogen atom or a hydrocarbon group, n is an integer of 1 or greater, and 1 is an integer of 1 or greater but 12 or less.

Preferable ranges of R in the general formulas of the ionic liquids are identical to the preferable ranges of R in the general formulas of the corresponding conjugate acids.

Preferable ranges of R₁ and R₂ in the ionic liquid represented by General Formula (1), the ionic liquid represented by General Formula (1-1), and the ionic liquid represented by General Formula (1-2) are identical to the preferable ranges of R₁ and R₂ in the corresponding conjugate acid represented by the General Formula (A).

Preferable ranges of R₁ and R₂ in the ionic liquid represented by General Formula (4), the ionic liquid represented by General Formula (4-1), and the ionic liquid represented by General Formula (4-2) are identical to the preferable ranges of R₁ and R₂ in the corresponding conjugate acid represented by the General Formula (D).

Preferable ranges of n in the general formulas of the ionic liquids are identical to the preferable ranges of n in the general formulas of the corresponding conjugate acids.

Preferable ranges of l in the general formulas of the ionic liquids are identical to the preferable ranges of 1 in the general formulas of the corresponding conjugate bases.

A synthesis method of the ionic liquid is not particularly limited and may be appropriately selected depending on the intended purpose. For example, various types of the ionic liquid can be synthesized with reference to the method disclosed in Examples below.

The ionic liquid of the present embodiment may be used alone as the lubricant, or the ionic liquid may be used in combination with a conventional lubricant. Examples of the lubricant used in combination include long-chain carboxylic acid, long-chain carboxylic acid ester, perfluoroalkyl carboxylic acid ester, perfluoroalkyl carboxylate, perfluoroalkyl perfluoroalkylcarboxylate, and a perfluoropolyether derivative.

Moreover, an extreme pressure agent may be used in combination at a mass ratio of about 30:70 to about 70:30 in a mass ratio in order to maintain a lubricating effect under severe conditions. The extreme pressure agent reacts with a surface of a metal with friction heat generated when the lubricant is partially in contact with the metal in a boundary lubrication region, and forms a coating film of a reaction product. As a result, friction and abrasion are prevented. As the extreme pressure agent, for example, any of a phosphorus-based extreme pressure agent, a sulfur-based extreme pressure agent, a halogen-based extreme pressure agent, an organic metal-based extreme pressure agent, or a complex extreme pressure agent can be used.

Moreover, an anti-rust agent may be optionally used in combination. The anti-rust agent may be any anti-rust agent typically used for this kind of magnetic recording media. Examples of the anti-rust agent include phenols, naphthols, quinones, heterocyclic compounds containing a nitrogen atom, heterocyclic compounds containing an oxygen atom, and heterocyclic compounds containing a sulfur atom. Moreover, the anti-rust agent may be mixed with the lubricant. Alternatively, the anti-rust agent and the lubricant may be deposited as two or more layers by forming a magnetic layer on a non-magnetic support, and applying an anti-rust agent layer on the upper part of the magnetic layer, followed by applying a lubricant layer.

As a solvent of the lubricant, for example, a single use or a combination of alcoholic solvents, such as isopropyl alcohol (IPA), and ethanol, can be used. For example, a mixture of a hydrocarbon-based solvent, such as normal-hexane, and a fluorine-based solvent can be used.

The solvent is preferably a fluorine-based solvent. Examples of the fluorine-based solvent include hydrofluoroethers [e.g., C₃F₇OCH₃, C₄F₉OCH₃, C₄F₉OC₂H₅, C₂F₅CF(OCH₃)C₃F₇, and CF₃(CHF)₂CF₂CF₃]. The hydrofluoroether may be used by mixing with alcohol, such as IPA, ethanol, and methanol.

The fluorine-based solvent may be a commercially available product. Examples of the commercially available product include: Novec™ 7000, 7100, 7200, 7300, and 71IPA, available from 3M; and Vertrel XF, and X-P10 available from Du Pont-Mitsui Fluorochemicals Company, Ltd.

### <2. Magnetic recording medium>

Next, a magnetic recording medium using the above-described lubricant is described. A magnetic recording medium described as one embodiment of the present invention includes at least a magnetic layer on a non-magnetic support, and the above-described lubricant is held on the magnetic layer.

The lubricant of the present embodiment can be applied for so-called a thin film-metal-type magnetic recording medium, in which a magnetic layer formed on a non-magnetic support by a method, such as vapor deposition and sputtering. Moreover, the lubricant can be also applied for a magnetic recording medium having a structure, in which a base layer is disposed between a non-magnetic support and a magnetic layer. Examples of such a magnetic recording medium include a magnetic disk, and a magnetic tape.

FIG. 4 is a cross-sectional view illustrating one example of a hard disk. The hard disk has a structure, in which a substrate 11, a base layer 12, a magnetic layer 13, a protective carbon layer 14, and a lubricant layer 15 are sequentially laminated.

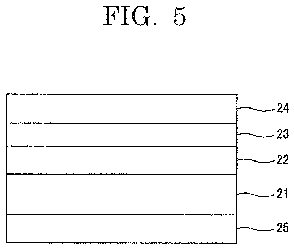

Moreover, FIG. 5 is a cross-sectional view illustrating one example of a magnetic tape. The magnetic tape has a structure, in which a back-coating layer 25, a substrate 21, a magnetic layer 22, a protective carbon layer 23, and a lubricant layer 24 are sequentially laminated.

In the magnetic disk illustrated in FIG. 4, each of the substrate 11 and the base layer 12 corresponds to the non-magnetic support. In the magnetic tape illustrated in FIG. 5, the substrate 21 corresponds to the non-magnetic support. In the case where a rigid substrate, such as an Al alloy plate, and a glass plate, is used as the non-magnetic support, a surface of the substrate may be made hard by forming an oxidized film, such as anodizing or a Ni-P coating on the surface of the substrate.

Each of the magnetic layers 13 and 22 is formed as a continuous film by a method, such as plating, sputtering, vacuum deposition, and plasma CVD. Examples of the magnetic layers 13 and 22 include: longitudinal magnetic recording metal magnetic films formed of metals (e.g., Fe, Co, and Ni), Co-Ni-based alloys, Co-Pt-based alloys, Co-Ni-Pt-based alloys, Fe-Co-based alloys, Fe-Ni-based alloys, Fe-Co-Ni-based alloys, Fe-Ni-B-based alloys, Fe-Co-B-based alloys, or Fe-Co-Ni-B-based alloys; and perpendicular magnetic recording metal magnetic thin films, such as Co-Cr-based alloy thin films, and Co-O-based thin films.

In the case where a longitudinal magnetic recording metal magnetic thin film is formed, particularly, a non-magnetic material, such as Bi, Sb, Pb, Sn, Ga, In, Ge, Si, and Tl, is formed as a base layer 12 on a non-magnetic support in advance, and a metal magnetic material is deposited through vapor deposition or sputtering in a perpendicular direction to diffuse the non-magnetic material into the magnetic metal thin film, to thereby improve a coercive force as well as eliminating orientation to assure in-plane isotropy.

Moreover, a hard protective layer 14 or 23, such as a carbon film, a diamond-formed carbon film, a chromium oxide film, and SiO₂ film, may be formed on a surface of the magnetic layer 13 or 22.

Examples of a method for applying the above-mentioned lubricant to such a metal thin film magnetic recording medium include a method for top-coating a surface of the magnetic layer 13 or 22, or a surface of the protective layer 14 or 23 with the lubricant, as illustrated in FIGs. 4 and 5. A coating amount of the lubricant is preferably from 0.1 mg/m² to 100 mg/m², more preferably from 0.5 mg/m² to 30 mg/m², and particularly preferably from 0.5 mg/m² to 20 mg/m².

As illustrated in FIG. 5, moreover, a metal thin film magnetic tape may optionally have a back-coating layer 25, other than a metal magnetic thin film, which is the magnetic layer 22.

The back-coating layer 25 is formed by adding a carbon-based powder for imparting conductivity, or an inorganic pigment for controlling a surface roughness to a resin binder, and applying the resin binder mixture. In the present embodiment, the above-described lubricant may be internally added to the back-coating layer 25, or applied to the back-coating layer 25 as top coating. Moreover, the above-described lubricant may be internally added to both the magnetic layer 22 and the back-coating layer 25, or applied to both the magnetic layer 22 and the back-coating layer 25 as top coating.

As another embodiment, moreover, the lubricant can be applied for a so-called coating-type magnetic recording medium, in which a magnetic coating film is formed as a magnetic layer by applying a magnetic coating material onto a surface of a non-magnetic support. In the coating-type magnetic recording medium, the non-magnetic support, a magnetic powder constituting the magnetic coating film, and the resin binder for use can be selected from any of those known in the art.

Examples of the non-magnetic support include: polymer substrates formed of polymer materials, such as polyesters, polyolefins, cellulose derivatives, vinyl-based resins, polyimides, polyamides, and polycarbonate; metal substrates formed of aluminium alloys, or titanium alloys; ceramic substrates formed of alumina glass; and glass substrates. Moreover, a shape of the non-magnetic support is not particularly limited, and may be any form, such as a tape, a sheet, and a drum. Furthermore, the non-magnetic support may be subjected to a surface treatment to form fine irregularities in order to control surface properties of the non-magnetic support.

Examples of the magnetic powder include: ferromagnetic iron oxide-based particles, such as γ-Fe₂O₃, cobalt-coated γ-Fe₂O₃; ferromagnetic chromium dioxide; ferromagnetic metal-based particles formed of a metal, such as Fe, Co, and Ni, or an alloy containing any of the above-listed metals; and hexagonal ferrite particles in the form of hexagonal plates.

Examples of the resin binder include: polymers, such as vinyl chloride, vinyl acetate, vinyl alcohol, vinylidene chloride, acrylic acid ester, methacrylic acid ester, styrene, butadiene, and acrylonitrile; copolymers combining two or more selected from the above-listed polymers; polyurethane resins; polyester resins; and epoxy resins. In order to improve dispersibility of the magnetic powder, a hydrophilic polar group, such as a carboxylic acid group, a carboxyl group, and a phosphoric acid group, may be introduced into any of the above-listed binders.

Other than the magnetic powder and the resin binder, additives, such as a dispersing agent, an abrasive, an antistatic agent, and an anti-rust agent, may be added to the magnetic coating film.

As a method for retaining the above-described lubricant in the coating-type magnetic recording medium, there are a method where the lubricant is internally added to the magnetic layer constituting the magnetic coating film formed on the non-magnetic support, a method where the lubricant is applied on a surface of the magnetic layer as top coating, and a combination of the above-listed methods. In the case where the lubricant is internally added into the magnetic coating film, the lubricant is added in an amount of from 0.2 parts by mass to 20 parts by mass relative to 100 parts by mass of the resin binder.

In the case where a surface of the magnetic layer is top-coated with the lubricant, moreover, a coating amount of the lubricant is preferably from 0.1 mg/m² to 100 mg/m², and more preferably from 0.5 mg/m² to 20 mg/m². As a deposition method in the case where the lubricant is applied as top coating, the ionic liquid is dissolved in a solvent, and the obtained solution may be applied or sprayed, or a magnetic recording medium may be dipped in the solution.

The magnetic recording medium, to which the lubricant of the present embodiment is applied, exhibits excellent running performances, abrasion resistance, and durability because of a lubricating effect, and can further improve thermal stability.

### Examples

### <3. Examples>

Specific examples of the present invention are explained below. In the present examples, an ionic liquid was synthesized, and a lubricant including the ionic liquid was produced. First, solubility to Vertrel [CF₃(CHF)₂CF₂CF₃] that is a fluorine-based solvent was examined. The lubricant solution was used to be coated on the surface of a magnetic disk and the surface of a magnetic tape. The disk durability and the tape durability were each evaluated. Production of a magnetic disk, a durability test of the disk, production of a magnetic tape, and a durability test of the tape were performed as described below. Note that, the present invention is not limited to these examples.

### <Production of magnetic disk>

A magnetic thin film was formed on a glass substrate to produce a magnetic disk as illustrated in FIG. 4, for example, according to International Patent Publication No. WO2005/068589. Specifically, a chemically reinforced glass disk, which was formed of aluminium silicate glass and had an outer diameter of 65 mm, an inner diameter of 20 mm, and a disk thickness of 0.635 mm, was prepared, and a surface of the glass disk was polished so that Rmax of the surface was 4.8 nm and Ra of the surface was 0.43 nm. The glass substrate was subjected to ultrasonic cleaning for 5 minutes in pure water, and then in isopropyl alcohol (IPA) having a purity of 99.9% or greater. The washed glass substrate was left to stand in saturated IPA steam for 1.5 minutes, followed by drying. The resultant glass substrate was provided as a substrate 11.

On the substrate 11, a NiAl alloy (Ni: 50 mol%, Al: 50 mol%) thin film having a thickness of 30 nm as a seed layer, a CrMo alloy (Cr: 80 mol%, Mo: 20 mol%) thin film having a thickness of 8 nm as a base layer 12, and a CoCrPtB alloy (Co: 62 mol%, Cr: 20 mol%, Pt: 12 mol%, B: 6 mol%) thin film having a thickness of 15 nm as a magnetic layer 13 were sequentially formed by DC magnetron sputtering.

Subsequently, a 5 nm-thick protective carbon layer 14 formed of amorphous diamond-like carbon was formed by plasma CVD, and the resultant disk sample was subjected to ultrasonic cleaning for 10 minutes in isopropyl alcohol (IPA) having a purity of 99.9% or greater in a cleaner to remove impurities on the surface of the disk, followed by drying. Thereafter, an ionic liquid in a mixture solvent of n-hexane and ethanol was applied on the surface of the disk by dip coating in the environment of 25°C and 50% in relative humidity (RH), to form a lubricant layer 15 (about 1 nm).

### <Measurement of thermal stability>

In the TG/DTA measurement, while air being introduced at a flow rate of 200 mL/min, the measurement was performed by means of EXSTAR6000 available from Seiko Instruments Inc. at a temperature range of from 30°C to 600°C at a heating rate of 10°C/min.

An endothermic peak temperature in the measurement was defined as a melting point.

### <Disk durability test>

A CSS durability test was performed by means of a commercially available strain-gauge-type disk friction-abrasion tester in the following manner. A hard disk was mounted on a rotatable spindle with tightening torque (14.7 Ncm). Thereafter, a head slider was attached on the hard disk in a manner that a center of an air bearing surface at the inner circumference side of the head slider relative to the hard disk was 17.5 mm from a center of the hard disk. The head used for the measurement was an IBM3370-type inline head, a material of the slider was Al₂ O₃ -TiC, and the head load was 63.7 mN. In the test, the maximum value of friction force was monitored per CSS (contact, start, and stop) in the environment of cleanliness 100, 25°C, and 60%RH. The number of times when a coefficient of friction was greater than 1.0 was considered as a result of the CSS durability test. When a result of the CSS durability test was greater than 50,000 times, the result was represented as "> 50,000." Moreover, a CSS durability test was similarly performed after performing a heating test for 3 minutes at a temperature of 200°C, in order to examine heat resistance.

### <Production of magnetic tape>

A magnetic tape having a cross-sectional structure as illustrated in FIG. 5 was produced. First, Co was deposited on a substrate 21 formed of a 5 µm-thick MICTRON (aromatic polyamide) film available from TORAY INDUSTRIES, INC. by oblique deposition to form a magnetic layer 22 formed of a ferromagnetic metal thin film having a film thickness 100 nm. Next, a protective carbon layer 23 formed of a 10 nm-thick diamond-like carbon was formed on a surface of the ferromagnetic metal thin film by plasma CVD, followed by cutting the resultant into a strip having a width of 6 mm. An ionic liquid dissolved in IPA was applied onto the protective carbon layer 23 in a manner that a film thickness was about 1 nm. In this manner, a lubricant layer 24 was formed on the magnetic layer to thereby produce a sample tape.

### <Tape durability test>

Each sample tape was subjected to a measurement of still durability in an environment of a temperature of -5°C and in an environment of a temperature of 40°C and 30%RH, and measurements of a coefficient of friction and shuttle durability in an environment of a temperature of -5°C and in an environment of a temperature of 40°C and 90%RH. The still durability was evaluated by a decay time of an output in a paused state decayed by -3 dB. The shuttle durability was evaluated by the number of shuttles taken until an output was reduced by 3 dB when repeated shuttle run was performed for 2 minutes per time. Moreover, a durability test was similarly performed after performing a heating test for 10 minutes at a temperature of 100°C, in order to examine heat resistance.

The ionic liquid in the present embodiments includes a conjugate base and a conjugate acid, and a pKa of an acid that is a source of the conjugate base in acetonitrile is 10 or less. Moreover, the conjugate acid (cation portion) preferably has a group including a hydroxyl group and a group including a straight-chain hydrocarbon group having 6 or greater carbon atoms. The thermal stability of such an ionic liquid and the durability of a magnetic recording medium using the ionic liquid were examined. Moreover, solubility to a fluorine-based solvent was examined.

### (Example 1A)

### <Synthesis of nonafluorobutanesulfonic acid-1-3'hydroxypropyl-3-octadecylimidazolium>

Synthesis of nonafluorobutanesulfonic acid-1-3'hydroxypropyl-3-octadecylimidazolium was performed according to the following scheme.

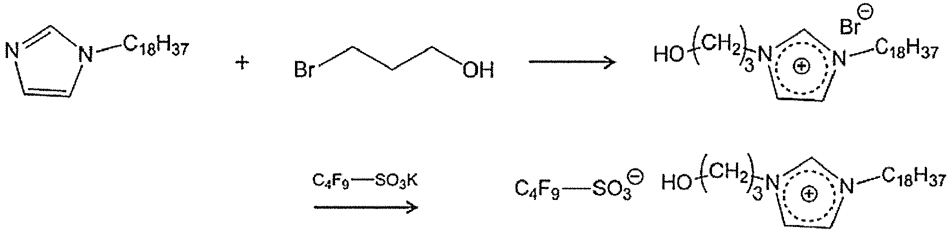

Imidazole (3 g) was dissolved in acetonitrile (100 mL). Octadecylbromide (14.9 g) and potassium hydroxide (2.51 g) were added thereto. The resultant was heated under stirring and was refluxed for 4 hours to obtain 1-octadecylimidazole. After the solvent was removed, the resultant was extracted with dichloromethane and was purified through column chromatography. When the resultant was analyzed through gas chromatography, it had a purity of 98.5% or more.

To a flask, 1-octadecylimidazole (6.13 g) and 3-bromopropanol (3.66 g) were added, and were heated at 120°C for 5.5 hours. After the temperature was returned to normal temperature, the resultant was crystalized by addition of ethyl acetate. The crystal was recrystallized from ethyl acetate to obtain 1-3'hydroxypropyl-3-octadecylimidazolium bromide (7.66 g) that was a colorless crystal. Yield: 87.1%.

Peaks of the obtained compound in CDCl₃ determined through the proton (¹H)NMR and the carbon (¹³C)NMR are presented below.
¹H-NMR(CDCl₃,δppm);0.843(t/J=6.8Hz,3H), 1.160-1.350(m,30H), 1.830-1.940(m,2H), 2.095-2.140(m,2H), 3.626(t/J=5.6Hz,2H), 4.276(t/J=7.6Hz,2H), 4.510(t/J=6.4Hz,2H), 7.262-7.271(m,1H), 7.566-7.575(m,1H), 10.225(s,1H)
¹³C-NMR(CDCl₃,δppm);14.080, 22.647, 26.259, 28.962, 29.326, 29.479, 29.623, 29.671, 30.208, 31.884, 32.392, 47.179, 50.245, 57.135, 121.502, 122.480, 137.381

From these spectra, the product was identified as 1-3'hydroxypropyl-3-octadecylimidazolium bromide.

Through heating, 1-3'hydroxypropyl-3-octadecylimidazolium bromide (3.16 g) was dissolved in water. Then, an aqueous solution of a potassium nonafluorobutanesulfonate salt (2.33 g) was added thereto. The resultant was stirred at normal temperature for 1 hour and was heated to reflux for 1 hour. After cooling, the reaction liquid was extracted with dichloromethane. The organic layer was sufficiently washed with water until a result of the silver nitrate test became negative. After the organic layer was dried with anhydrous sodium sulfate, the solvent was removed to obtain nonafluorobutanesulfonic acid-1-3'hydroxypropyl-3-octadecylimidazolium (4.32 g) that was a colorless crystal. Yield: 92.6%.

The FTIR absorption of the product and the assignment are presented below.

Symmetric stretching vibrations of a SO₂ bond were observed at 1,133 cm⁻¹, symmetric stretching vibrations of CF₂ were observed at 1,255 cm⁻¹, asymmetric stretching vibrations of a SO₂ bond were observed at 1,352 cm⁻¹, bending vibrations of CH₂ were observed at 1,469 cm⁻¹, symmetric stretching vibrations of C=N were observed at 1,566 cm⁻¹, symmetric stretching vibrations of CH₂ were observed at 2,851 cm⁻¹, asymmetric stretching vibrations of CH₂ were observed at 2,920 cm⁻¹, stretching vibrations of CH of an imidazole ring were observed at 3,150 cm⁻¹, and stretching vibrations of OH were observed at 3,481 cm⁻¹.

Peaks of the obtained compound in CDCl₃ determined through the proton (¹H)NMR and the carbon (¹³C)NMR are presented below.
¹H-NMR(CDCl₃,δppm);0.852(t/J=6.8Hz,3H), 1.170-1.320(m,30H), 1.780-1.880(m,2H), 2.058-2.088(m,2H), 3.613(t/J=6.0Hz,2H), 4.158(t/J=7.6Hz,2H), 4.373(t/J=6.0Hz,2H), 7.218-7.226(m,1H), 7.422-7.431(m,1H), 9.091(s,1H)
¹³C-NMR(CDCl₃,δppm);14.070, 22.656, 26.173, 28.885, 29.326, 29.470, 29.671, 30.064, 31.894, 32.153, 47.265, 50.149, 57.950, 121.780, 122.643, 136.461

From these spectra, the product was identified as nonafluorobutanesulfonic acid-1-3'hydroxypropyl-3-octadecylimidazolium.

Note that, a pKa of an acid (nonafluorobutanesulfonic acid) that is a source of a conjugate base in nonafluorobutanesulfonic acid-1-3'hydroxypropyl-3-octadecylimidazolium in acetonitrile is 0.7.

### (Example 2A)

### <Synthesis of bis(nonafluorobutanesulfonyl)imide1-3'hydroxypropyl-3-octadecylimidazolium>

Synthesis of bis(nonafluorobutanesulfonyl)imide1-3'hydroxypropyl-3-octadecylimidazolium was performed according to the following scheme.

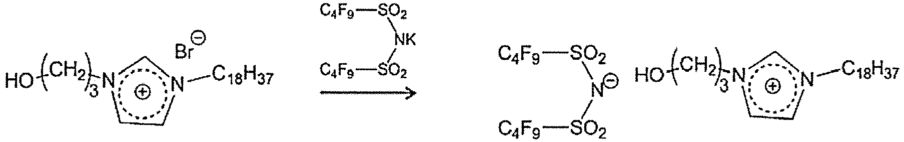

In pure water that had been heated, 1-3'hydroxypropyl-3-octadecylimidazolium bromide (3.07 g) synthesized in Example 1A was dissolved. Then, a product obtained by dissolving potassium bis(nonafluorobutanesulfonyl)imide (4.22 g) in a mixture solvent of water and ethanol was added thereto. The resultant was heated to reflux for 1 hour. After cooling, it was extracted with dichloromethane. The organic layer was washed with pure water until a result of the AgNO₃ test became negative. After the organic layer was dried with anhydrous sodium sulfate, the solvent was removed to obtain bis(nonafluorobutanesulfonyl)imide1-3'hydroxypropyl-3-octadecylimidazolium (5.20 g) that was a colorless liquid. Yield: 81.1%.

The FTIR absorption of the product and the assignment are presented below. Symmetric stretching vibrations of a SO₂ bond were observed at 1,077 cm⁻¹, symmetric stretching vibrations of CF₂ were observed at 1,236 cm⁻¹, asymmetric stretching vibrations of a SO₂ bond were observed at 1,354 cm⁻¹, bending vibrations of CH₂ were observed at 1,466 cm⁻¹, symmetric stretching vibrations of C=N were observed at 1,565 cm⁻¹, symmetric stretching vibrations of CH₂ were observed at 2,856 cm⁻¹, asymmetric stretching vibrations of CH₂ were observed at 2,927 cm⁻¹, stretching vibrations of CH of an imidazole ring were observed at 3,152 cm⁻¹, and stretching vibrations of OH were observed at 3,553 cm⁻¹.

Peaks of the obtained compound in CDCl₃ determined through the proton (¹H)NMR and the carbon (¹³C)NMR are presented below.
¹H-NMR(CDCl₃,δppm);0.855(t/J=6.8Hz,3H), 1.180-1.330(m,30H), 1.780-1.880(m,2H), 2.057-2.086(m,2H), 3.655(t/J=6.0Hz,2H), 4.138(t/J=7.6Hz,2H), 4.345(t/J=6.0Hz,2H), 7.211-7.235(m,1H), 7.357-7.366(m,1H), 8.794(s,1H)
¹³C-NMR(CDCl₃,δppm);14.070, 22.666, 26.106, 28.847, 29.288, 29.335, 29.450, 29.556, 29.671, 30.073, 31.904, 31.980, 47.341, 50.235, 58.323, 121.838, 122.777, 135.857

From these spectra, the product was identified as bis(nonafluorobutanesulfonyl)imide1-3'hydroxypropyl-3-octadecylimidazolium.

Note that, a pKa of an acid [bis(nonafluorobutanesulfonyl)imide] that is a source of a conjugate base in bis(nonafluorobutanesulfonyl)imide1-3'hydroxypropyl-3-octadecylimidazolium in acetonitrile is 0.0.

### (Example 3A)

### <Synthesis of bis(nonafluorobutanesulfonyl)imide-N-3'hydroxypropyl-N-octadecylpyrrolidinium>

Synthesis of bis(nonafluorobutanesulfonyl)imide-N-3'hydroxypropyl-N-octadecylpyrrolidinium was performed according to the following scheme.

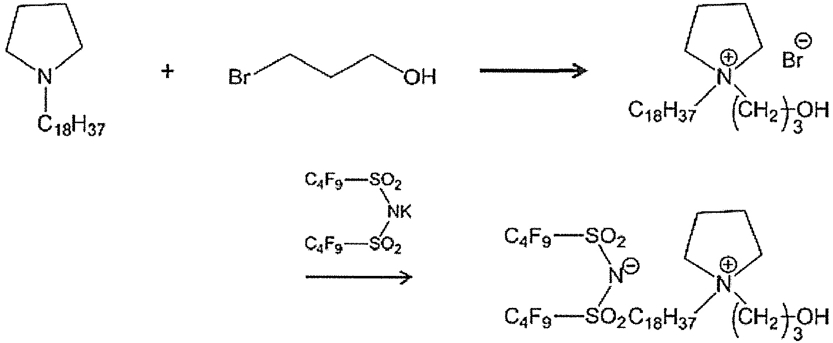

Bromooctadecane (52.4 g) and potassium hydroxide (8.75 g) were added to acetonitrile, and pyrrolidine (11.09 g) was added thereto. Then, the resultant was heated to reflux for 24 hours. A crystal was obtained through filtration. After the solvent of the organic layer was removed, silica gel column chromatography using a mixture solvent of hexane and ethyl acetate was performed for purification to obtain octadecyl pyrrolidine (44.05 g). The purity determined through gas chromatography was 99.0% or more.

Octadecyl pyrrolidine (6.00 g) and 3-bromopropanol (3.23 g) were charged into a flask and were heated at 120°C for 3.0 hours. After the temperature was returned to normal temperature, ethyl acetate was added thereto for crystallization to obtain N-3'hydroxypropyl-N-octadecylpyrrolidinium bromide (7.77 g). Yield: 90.5%.

Peaks of the obtained compound in deuterated methanol determined through proton (¹H) NMR and carbon (¹³C) NMR are presented below.
¹H-NMR(CD₃OD,δppm);0.875(t/J=7.0Hz,3H), 1.190-1.440(m,30H), 1.670-1.790(m,2H), 1.860-1.950(m,2H), 2.130-2.230(m,4H), 3.250-3.292(m,2H), 3.356-3.397(m,2H), 3.537(t/J=7.2Hz,4H), 3.637(t/J=5.8Hz,2H)
¹³C-NMR(CD₃OD,δppm);14.454, 22.830, 23.740, 24.219, 27.333, 27.506, 30.266, 30.477, 30.592, 30.649, 30.793, 33.073, 55.104, 58.477, 59.301, 61.132, 64.122

From these spectra, the product was identified as N-3'hydroxypropyl-N-octadecylpyrrolidinium bromide.

Through heating, N-3'hydroxypropyl-N-octadecylpyrrolidinium bromide (2.77 g) was dissolved in pure water. A product obtained by heating and dissolving bis(nonafluorobutanesulfonyl)imide potassium (3.88 g) in a mixture solvent of pure water and ethanol was added thereto. The resultant was heated to reflux for 1 hour. The precipitated product was extracted with dichloromethane and the organic layer was sufficiently washed with pure water until a result of the AgNO₃ test became negative. After the organic layer was dried with anhydrous sodium sulfate, the solvent was removed to obtain bis(nonafluorobutanesulfonyl)imide-N-3'hydroxypropyl-N-octadecylpyrrolidinium (5.62 g) that was a colorless liquid. Yield: 97.4%.

The FTIR absorption of the product and the assignment are presented below.

Symmetric stretching vibrations of a SO₂ bond were observed at 1,140 cm⁻¹, symmetric stretching vibrations of CF₂ were observed at 1,237 cm⁻¹, asymmetric stretching vibrations of a SO₂ bond were observed at 1,354 m⁻¹, bending vibrations of a CH₂ bond were observed at 1,467 cm⁻¹, symmetric stretching vibrations of a CH₂ bond were observed at 2,857 cm⁻¹, asymmetric stretching vibrations of a CH₂ bond were observed at 2,928 cm⁻¹, and stretching vibrations of a OH bond were observed at 3,553 cm⁻¹.

Peaks of the obtained compound in deuterated chloroform determined through the proton (¹H)NMR and the carbon (¹³C)NMR are presented below.
¹H-NMR(CDCl₃,δppm);0.855(t/J=7.0Hz,3H), 1.170-1.360(m,30H), 1.640-1.740(m,2H), 1.850-1.940(m,2H), 2.140-2.290(m,4H), 2.623(brs, 1H), 3.110-3.190(m,2H), 3.340-3.410(m,2H), 3.420-3.580(m,4H), 3.707(t/J=5.4Hz,2H)
¹³C-NMR(CDCl₃,δppm);14.080, 21.708, 22.666, 23.289, 26.173, 26.240, 29.010, 29.335, 29.393, 29.546, 29.680, 31.904, 57.643, 58.333, 60.384, 63.095

From these spectra, the product was identified as bis(nonafluorobutanesulfonyl)imide-N-3'hydroxypropyl-N-octadecylpyrrolidinium.

Note that, a pKa of an acid [bis(nonafluorobutanesulfonyl)imide] that is a source of a conjugate base in bis(nonafluorobutanesulfonyl)imide-N-3'hydroxypropyl-N-octadecylpyrrolidinium in acetonitrile is 0.0.

### (Example 4A)

### <Synthesis of nonafluorobutanesulfonic acid-6-octadecyl-8-3'-hydroxypropyl-1,8-diazabicyclo[5.4.0]-7-undecenium>

Synthesis of nonafluorobutanesulfonic acid-6-octadecyl-8-3'-hydroxypropyl-1,8-diazabicyclo[5.4.0]-7-undecenium was performed according to the following scheme.

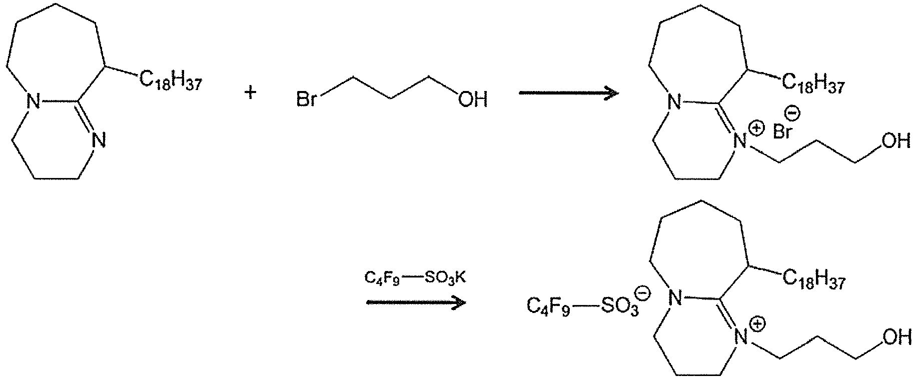

According to Non-Patent Literature [N. Matsumura, H. Nishiguchi, M. Okada, and S. Yoneda, J. Heterocyclic Chem. pp. 885 to 887, Vol/23. Issue 3 (1986)] by Matsumura et al., 6-octadecyl-1,8-diazabicyclo[5.4.0]-7-undecene was synthesized.

Into a flask, 6-octadecyl-1,8-diazabicyclo[5.4.0]-7-undecenium (5.71 g) and 3-bromopropanol (3.23 g) were charged and were heated at 120°C for 3.0 hours. The temperature was returned to normal temperature. Then, the reaction product was dissolved by addition of ethyl acetate and was crystallized in a refrigerator. The resultant was promptly filtrated at a low temperature and the thus-obtained crystal was subjected to vacuum drying to obtain 6-octadecyl-8-3'-hydroxypropyl-1,8-diazabicyclo[5.4.0]-7-undecenium bromide (6.60 g). Yield: 81.9%.

Through heating, 6-octadecyl-8-3'-hydroxypropyl-1,8-diazabicyclo[5.4.0]-7-undecenium bromide (4.30 g) was dissolved in pure water. Then, an aqueous solution obtained by dissolving potassium nonafluorobutanesulfonate (2.99 g) in pure water through heating was added thereto. The resultant was allowed to react at normal temperature for 1 hour and was heated to reflux for 1 hour. After cooling, the reaction solution was extracted with dichloromethane. Then, the organic layer was washed with pure water until a result of the AgNO₃ test became negative. The solvent was removed to obtain nonafluorobutanesulfonic acid-6-octadecyl-8-3'-hydroxypropyl-1,8-diazabicyclo[5.4.0]-7-undecenium (6.00 g) that was a light yellow liquid. Yield: 99.4%.

The FTIR absorption of the product and the assignment are presented below.

Symmetric stretching vibrations of a SO₂ bond were observed at 1,134 cm⁻¹, symmetric stretching vibrations of CF₂ were observed at 1,255 cm⁻¹, asymmetric stretching vibrations of a SO₂ bond were observed at 1,352 cm⁻¹, bending vibrations of CH₂ were observed at 1,466 cm⁻¹, stretching vibrations of C=N were observed at 1,609 cm⁻¹, symmetric stretching vibrations of CH₂ were observed at 2,855 cm⁻¹, asymmetric stretching vibrations of CH₂ were observed at 2,926 cm⁻¹, and stretching vibrations of a hydroxyl group were observed at 3,479 cm⁻¹.

Peaks of the obtained compound in deuterated chloroform determined through the proton (¹H)NMR and the carbon (¹³C)NMR are presented below.
¹H-NMR(CDCl₃,δppm);0.849(t/J=6.8Hz,3H), 1.140-1.480(m,34H), 1.520-1.940(m,8H), 1.960-2.140(m,2H), 2.423(brs,1H), 2.770-2.860(m,1H), 3.160-3.610(m,6H), 3.610-3.710(m,2H) 3.760-3.940(m,2H)
¹³C-NMR(CDCl₃,δppm);14.089, 20.433, 22.033, 22.656, 25.646, 26.528, 27.179, 27.419, 29.335, 29.412, 29.680, 30.945, 31.894, 38.631, 47.562, 50.983, 51.337, 54.059, 58.534, 168.122

From these spectra, the product was identified as nonafluorobutanesulfonic acid-6-octadecyl-8-3'-hydroxypropyl-1,8-diazabicyclo[5.4.0]-7-undecenium.

Note that, a pKa of an acid (nonafluorobutanesulfonic acid) that is a source of a conjugate base in nonafluorobutanesulfonic acid-6-octadecyl-8-3'-hydroxypropyl-1,8-diazabicyclo [5.4.0]-7-undecenium in acetonitrile is 0.7.

### (Example 5A)

### <Synthesis of bis(nonafluorobutanesulfonyl)imide-6-octadecyl-8-3'-hydroxypropyl-1,8-diazabicyclo[ 5.4.0]-7-undecenium>

Synthesis of bis(nonafluorobutanesulfonyl)imide-6-octadecyl-8-3'-hydroxypropyl-1,8-diazabicyclo[ 5.4.0]-7-undecenium was performed according to the following scheme.

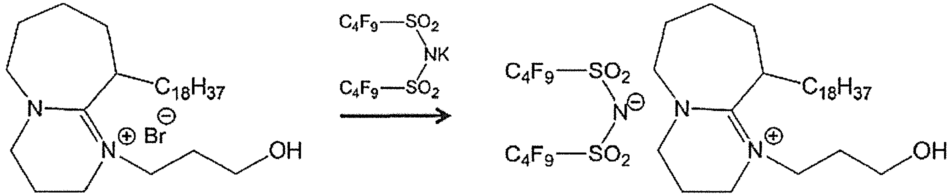

Through heating, 6-octadecyl-8-3'-hydroxypropyl-1,8-diazabicyclo[5.4.0]-7-undecenium bromide (2.04 g) synthesized in Example 4A was dissolved in pure water. Then, a solution obtained by dissolving bis(nonafluorobutanesulfonyl)imide potassium (3.07 g) in pure water and ethanol through heating was added thereto. The resultant was allowed to react at normal temperature for 1 hour and was heated to reflux for 1 hour. After cooling, the reaction solution was extracted with dichloromethane. Then, the organic layer was washed with pure water until a result of the AgNO₃ test became negative. The solvent was removed to obtain bis(nonafluorobutanesulfonyl)imide-6-octadecyl-8-3'-hydroxypropyl-1,8-diazabicyclo[ 5.4.0]-7-undecenium (3.42 g) that was a light yellow liquid. Yield: 87.3%.

The FTIR absorption of the product and the assignment are presented below.

Asymmetric stretching vibrations of a SNS bond were observed at 1,074 cm⁻¹, symmetric stretching vibrations of CF₂ were observed at 1,232 cm⁻¹, asymmetric stretching vibrations of a SO₂ bond were observed at 1,352 cm⁻¹, bending vibrations of CH₂ were observed at 1,468 cm⁻¹, stretching vibrations of C=N were observed at 1,608 cm⁻¹, symmetric stretching vibrations of CH₂ were observed at 2,854 cm⁻¹, asymmetric stretching vibrations of CH₂ were observed at 2,924 cm⁻¹, and stretching vibrations of a hydroxyl group were observed at 3,479 cm⁻¹.

Peaks of the obtained compound in deuterated chloroform determined through the proton (¹H)NMR and the carbon (¹³C)NMR are presented below.
¹H-NMR(CDCl₃,δppm);0.854(t/J=7.2Hz,3H), 1.130-1.340(m,34H), 1.520-1.910(m,8H), 1.960-2.140(m,2H), 2.770-2.860(m,1H), 3.380-3.600(m,6H), 3.610-3.715(m,2H) 3.740-3.940(m,2H)
¹³C-NMR(CDCl₃,δppm);14.080, 19.197, 20.299, 21.947, 22.666, 26.518, 27.409, 29.307, 29.345, 29.412, 29.508, 29.537, 29.585, 29.642, 29.690, 30.715, 31.904, 38.640, 47.389, 50.858, 51.069, 54.011, 58.592, 168.228

From these spectra, the product was identified as bis(nonafluorobutanesulfonyl)imide-6-octadecyl-8-3'-hydroxypropyl-1,8-diazabicyclo[ 5.4.0]-7-undecenium.

Note that, a pKa of an acid [bis(nonafluorobutanesulfonyl)imide] that is a source of a conjugate base in bis(nonafluorobutanesulfonyl)imide-6-octadecyl-8-3'-hydroxypropyl-1,8-diazabicyclo[ 5.4.0]-7-undecenium in acetonitrile is 0.0.

### (Example 6A)

### <Synthesis of nonafluorobutanesulfonic acid-1-dodecyl-2-undecyl-3-3'hydroxypropylimidazolium>

Synthesis of nonafluorobutanesulfonic acid-1-dodecyl-2-undecyl-3-3'hydroxypropylimidazolium was performed according to the following scheme.

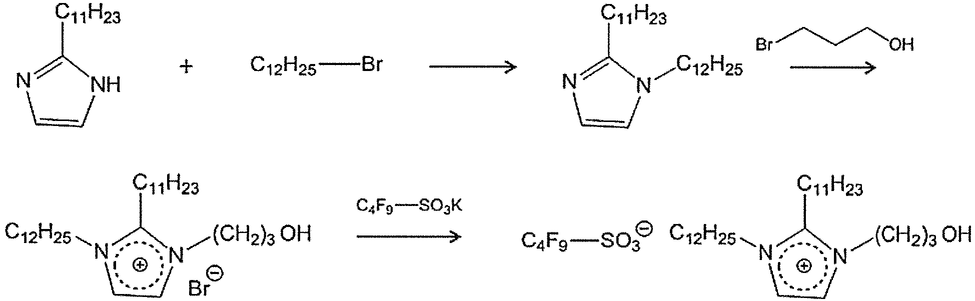

In toluene (100 mL), 2-undecylimidazole (17.76 g) was dissolved. Then, dodecyl bromide (20.00 g) and potassium hydroxide (7.75 g) were added and were heated under stirring. The resultant was heated to reflux for 8 hours to obtain 1-dodecyl-2-undecylimidazole. After the solvent was removed, the resultant was extracted with dichloromethane. Then, a solution of n-hexane:ethyl acetate=9:1 (volume ratio) was used to perform purification through silica gel column chromatography. When the resultant was analyzed through gas chromatography, it had a purity of 98.9% or more.

To a flask, 1-dodecyl-2-undecylimidazole (7.04 g) and 3-bromopropanol (3.84 g) were added and were heated at 120°C for 4 hours. After the temperature was returned to normal temperature, the precipitated crystal was recrystallized from ethyl acetate to obtain 1-dodecyl-2-undecyl-3-3'hydroxypropylimidazolium bromide (8.00 g) that was a colorless crystal. Yield: 83.8%.

The FTIR absorption of the product and the assignment are presented below.

Bending vibrations of CH₂ were observed at 1,470 cm⁻¹, symmetric stretching vibrations of C=N were observed at 1,526 cm⁻¹, symmetric stretching vibrations of CH₂ were observed at 2,851 cm⁻¹, asymmetric stretching vibrations of CH₂ were observed at 2,919 cm⁻¹, stretching vibrations of CH of an imidazole ring were observed at 3,086 cm⁻¹ and 3,123 cm⁻¹, and stretching vibrations of OH were observed at 3,317 cm⁻¹.

Peaks of the obtained compound in CDCl₃ determined through the proton (¹H)NMR and the carbon (¹³C)NMR are presented below.
¹H-NMR(CDCl₃,δppm);0.835(t/J=6.8Hz,6H), 1.160-1.440(m,34H), 1.571(quint/J=8.0Hz,2H), 1.760-1.860(m,2H), 2.115(quint/J=6.0Hz,2H), 3.083(t/J=8.2Hz,2H), 3.596(t/J=5.4Hz,2H), 4.094(t/J=7.8Hz,2H), 4.347(t/J=7.0Hz,2H), 7.434(d/J=2.0Hz,1H), 7.831(d/J=2.0Hz,1H)
¹³C-NMR(CDCl₃,δppm);14.051, 22.608, 26.422, 27.888, 29.000, 29.067, 29.259, 29.297, 29.460, 29.498, 29.527, 30.006, 31.808, 32.038, 45.348, 48.606, 57.020, 121.138, 122.154, 146.609

From these spectra, the product was identified as 1-dodecyl-2-undecyl-3-3'hydroxypropylimidazolium bromide.

Through heating, 1-dodecyl-2-undecyl-3-3'hydroxypropylimidazolium bromide (3.60 g) was dissolved in water and a small amount of ethanol, and an aqueous solution of a potassium nonafluorobutanesulfonate salt (2.80 g) was added thereto. The resultant was stirred at normal temperature for 1 hour and was heated to reflux for 1 hour. After cooling, the reaction liquid was extracted with dichloromethane. Then, the organic layer was sufficiently washed with water until a result of the silver nitrate test became negative. After the organic layer was dried with anhydrous sodium sulfate to remove the solvent, vacuum drying at 100°C was performed for 20 hours to obtain nonafluorobutanesulfonic acid-1-dodecyl-2-undecyl-3-3'hydroxypropylimidazolium (4.80 g) that was a colorless liquid. Yield: 94.3%.

The FTIR absorption of the product and the assignment are presented below.

Symmetric stretching vibrations of a SO₂ bond were observed at 1,054 cm⁻¹, symmetric stretching vibrations of CF₂ were observed at 1,134 cm⁻¹ and 1,255 cm⁻¹, asymmetric stretching vibrations of a SO₂ bond were observed at 1,351 cm⁻¹, bending vibrations of CH₂ were observed at 1,466 cm⁻¹, symmetric stretching vibrations of C=N were observed at 1,525 cm⁻¹, symmetric stretching vibrations of CH₂ were observed at 2,857 cm⁻¹, asymmetric stretching vibrations of CH₂ were observed at 2,927 cm⁻¹, stretching vibrations of CH of an imidazole ring were observed at 3,134 cm⁻¹, and stretching vibrations of OH were observed at 3,478 cm⁻¹.

Peaks of the obtained compound in CDCl₃ determined through the proton (¹H)NMR and the carbon (¹³C)NMR are presented below.
¹H-NMR(CDCl₃,δppm);0.852(t/J=6.8Hz,6H), 1.160-1.440(m,34H), 1.573(quint/J=7.8Hz,2H), 1.740-1.850(m,2H), 2.063(quint/J=5.8Hz,2H), 2.427(brs,1H), 3.009(t/J=8.4Hz,2H), 3.588(t/J=5.8Hz,2H), 4.022(t/J=7.8Hz,2H), 4.242(t/J=7.0Hz,2H), 7.237(d/J=2.8Hz,1H), 7.470(d/J=2.0Hz,1H)
¹³C-NMR(CDCl₃,δppm);14.070, 22.647, 26.422, 27.745, 28.990, 29.038, 29.268, 29.297, 29.326, 29.479, 29.537, 29.565, 29.901, 31.846, 31.865, 45.204, 48.453, 57.518, 120.994, 121.684, 146.791

From these spectra, the product was identified as nonafluorobutanesulfonic acid-1-dodecyl-2-undecyl-3-3'hydroxypropylimidazolium.

Note that, a pKa of an acid (nonafluorobutanesulfonic acid) that is a source of a conjugate base in nonafluorobutanesulfonic acid-1-dodecyl-2-undecyl-3-3'hydroxypropylimidazolium in acetonitrile is 0.7.

### (Example 7A)

### <Synthesis of bis(nonafluorobutanesulfonyl)imide-1-dodecyl-2-undecyl-3-3'hydroxypropyloctadecyl imidazolium>

Synthesis of bis(nonafluorobutanesulfonyl)imide-1-dodecyl-2-undecyl-3-3'hydroxypropyloctadecyl imidazolium was performed according to the following scheme.

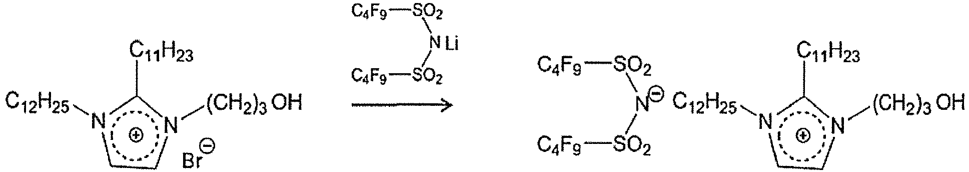

In a solution of pure water and a small amount of ethanol that had been heated, 1-dodecyl-2-undecyl-3-3'hydroxypropyloctadecylimidazolium bromide (3.64 g) synthesized in Example 6A was dissolved, and a product obtained by dissolving lithium bis(nonafluorobutanesulfonyl)imide (4.13 g) in water was added thereto. The resultant was heated to reflux for 1 hour. After cooling, the resultant was extracted with dichloromethane. The organic layer was washed with pure water until a result of the AgNO₃ test became negative. The organic layer was dried with anhydrous sodium sulfate, and the solvent was removed. The resultant was subjected to vacuum drying at 100°C for 20 hours to obtain bis(nonafluorobutanesulfonyl)imide-1-dodecyl-2-undecyl-3-3'hydroxypropylimidazoli um (6.80 g) that was a colorless liquid. Yield: 96.6%.

The FTIR absorption of the product and the assignment are presented below.

Symmetric stretching vibrations of SNS were observed at 1,054 cm⁻¹, symmetric stretching vibrations of a SO₂ bond were observed at 1,134 cm⁻¹, symmetric stretching vibrations of CF₂ were observed at 1,236 cm⁻¹ and 1,255 cm⁻¹, asymmetric stretching vibrations of a SO₂ bond were observed at 1,352 cm⁻¹, bending vibrations of CH₂ were observed at 1,466 cm⁻¹, symmetric stretching vibrations of C=N were observed at 1,525 cm⁻¹, symmetric stretching vibrations of CH₂ were observed at 2,857 cm⁻¹, asymmetric stretching vibrations of CH₂ were observed at 2,927 cm⁻¹, stretching vibrations of CH of an imidazole ring were observed at 3,134 cm⁻¹, and stretching vibrations of OH were observed at 3,478 cm⁻¹.

Peaks of the obtained compound in CDCl₃ determined through the proton (¹H)NMR and the carbon (¹³C)NMR are presented below.
¹H-NMR(CDCl₃,δppm);0.852(t/J=7.0Hz,6H), 1.140-1.440(m,34H), 1.586(quint/J=8.0Hz,2H),1.750-1.850(m,2H), 2.045(quint/J=6.2Hz,2H), 2.963(t/J=8.2Hz,2H), 3.607(t/J=5.4Hz,2H), 4.000(t/J=7.8Hz,2H), 4.220(t/J=7.0Hz,2H), 7.195(d/J=2.0Hz,1H), 7.335(d/J=2.0Hz,1H)
¹³C-NMR(CDCl₃,δppm);14.061, 22.647, 26.374, 27.659, 28.943, 28.971, 29.268, 29.297, 29.470, 29.527, 29.556, 29.882, 31.616, 31.846, 45.109, 48.472, 57.691, 121.071, 121.531, 146.657

From these spectra, the product was identified as bis(nonafluorobutanesulfonyl)imide-1-dodecyl-2-undecyl-3-3'hydroxypropylimidazoli um.

Note that, a pKa of an acid [bis(nonafluorobutanesulfonyl)imide] that is a source of a conjugate base in bis(nonafluorobutanesulfonyl)imide-1-dodecyl-2-undecyl-3-3'hydroxypropylimidazoli um in acetonitrile is 0.0.

### (Example 8A)

### <Synthesis of bis(nonafluorobutanesulfonyl)imide-1-3'hydroxypropyl-2-heptadecyl-3-octadecylimid azolium>

Synthesis of bis(nonafluorobutanesulfonyl)imide-1-3'hydroxypropyl-2-heptadecyl-3-octadecylimid azolium was performed according to the following scheme.

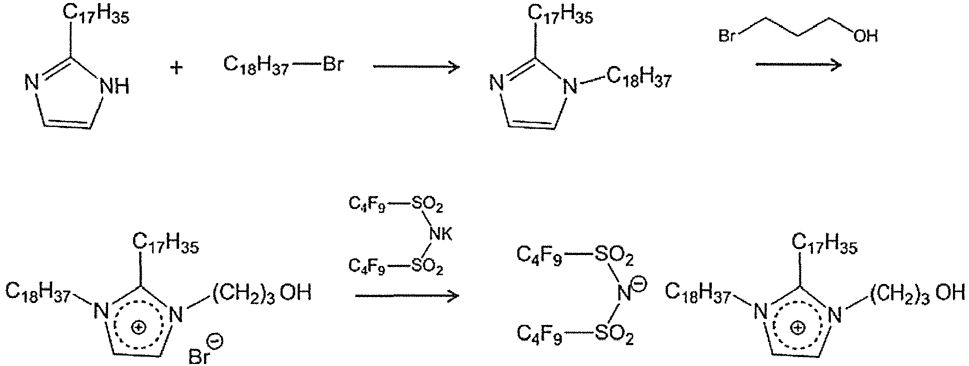

In toluene (100 mL), 2-heptadecylimidazole (15.34 g) was dissolved. Then, octadecylbromide (17.62 g) and potassium hydroxide (4.86 g) were added thereto and were heated under stirring. The resultant was refluxed for 11 hours to obtain 1-octadecyl-2-heptadecylimidazole. After the solvent was removed, the resultant was extracted with dichloromethane. A solution of n-hexane:ethyl acetate 9:1 was used to perform purification through silica gel column chromatography. When the resultant was analyzed through gas chromatography, it had a purity of 99.3% or more.

To a flask, 1-octadecyl-2-heptadecylimidazole (8.42 g) and 3-bromopropanol (3.20 g) were added. With the flask being tightly sealed, the resultant was heated at 120°C for 4 hours. The temperature was returned to normal temperature, the precipitated crystal was recrystallized from ethyl acetate to obtain 1-3'hydroxypropyl-2-heptadecyl-3-octadecylimidazolium bromide (9.75 g) that was a colorless crystal. Yield: 92.7%.

Peaks of the obtained compound in CDCl₃ determined through the proton (¹H)NMR and the carbon (¹³C)NMR are presented below.
¹H-NMR(CDCl₃,δppm);0.848(t/J=7.2Hz,6H), 1.140-1.460(m,58H), 1.580(quint/J=7.2Hz,2H), 1.760-1.860(m,2H), 2.129(quint/J=6.0Hz,2H), 3.083(t/J=8.2Hz,2H), 3.618(t/J=5.4Hz,2H), 4.097(t/J=7.8Hz,2H), 4.356(t/J=6.8Hz,2H), 7.380(d/J=2.0Hz,1H), 7.813(d/J=1.6Hz,1H)
¹³C-NMR(CDCl₃,δppm);14.099, 22.666, 24.007, 26.470, 27.927, 29.038, 29.105, 29.335, 29.518, 29.642, 29.680, 29.968, 31.894, 45.348, 48.683, 56.991, 120.994, 122.087, 146.829

From these spectra, the product was identified as 1-3'hydroxypropyl-2-heptadecyl-3-octadecylimidazolium bromide.

In a solution of pure water and a small amount of ethanol that had been heated, 1-3'hydroxypropyl-2-heptadecyl-3-octadecylimidazolium bromide (3.15 g) was dissolved. Then, a product obtained by dissolving potassium bis(nonafluorobutanesulfonyl)imide (2.90 g) in a solution of pure water and a small amount of ethanol was added thereto. The resultant was heated to reflux for 1 hour. After cooling, the resultant was extracted with dichloromethane. The organic layer was washed with pure water until a result of the AgNO₃ test became negative. After the organic layer was dried with anhydrous sodium sulfate, the solvent was removed. The resultant was subjected to vacuum drying at 100°C for 20 hours to obtain bis(nonafluorobutanesulfonyl)imide 1-3'hydroxypropyl-2-heptadecyl-3-octadecylimidazolium (5.03 g) that was a colorless liquid. Yield: 93.0%.

The FTIR absorption of the product and the assignment are presented below.

Symmetric stretching vibrations of SNS were observed at 1,076 cm⁻¹, symmetric stretching vibrations of a SO₂ bond were observed at 1,140 cm⁻¹, symmetric stretching vibrations of CF₂ were observed at 1,216 cm⁻¹ and 1,237 cm⁻¹, asymmetric stretching vibrations of a SO₂ bond were observed at 1,355 cm⁻¹, bending vibrations of CH₂ were observed at 1,467 cm⁻¹, symmetric stretching vibrations of C=N were observed at 1,525 cm⁻¹, symmetric stretching vibrations of CH₂ were observed at 2,855 cm⁻¹, asymmetric stretching vibrations of CH₂ were observed at 2,927 cm⁻¹, stretching vibrations of CH of an imidazole ring were observed at 3,146 cm⁻¹, and stretching vibrations of OH were observed at 3,555 cm⁻¹.

Peaks of the obtained compound in CDCl₃ determined through the proton (¹H)NMR and the carbon (¹³C)NMR are presented below.
¹H-NMR(CDCl₃,δppm);0.855(t/J=6.6Hz,6H), 1.140-1.440(m,58H), 1.588(quint/J=8.0Hz,2H),1.740-1.850(m,2H), 2.049(quint/J=6.3Hz,2H), 2.966(t/J=8.2Hz,2H), 3.613(t/J=5.6Hz,2H), 4.003(t/J=7.8Hz,2H), 4.224(t/J=6.8Hz,2H), 7.193(d/J=2.0Hz,1H), 7.335(d/J=2.0Hz,1H)
¹³C-NMR(CDCl₃,δppm);14.099, 22.675, 26.384, 27.668, 28.952, 28.990, 29.278, 29.355, 29.508, 29.652, 29.690, 29.882, 31.597, 31.913, 45.118, 48.482, 57.700, 121.061, 121.531, 146.666

From these spectra, the product was identified as bis(nonafluorobutanesulfonyl)imide1-3'hydroxypropyl-2-heptadecyl-3-octadecylimid azolium.

Note that, a pKa of an acid [bis(nonafluorobutanesulfonyl)imide] that is a source of a conjugate base in bis(nonafluorobutanesulfonyl)imide1-3'hydroxypropyl-2-heptadecyl-3-octadecylimid azolium in acetonitrile is 0.0.

### (Example 9A)

### <Synthesis of bis(nonafluorobutanesulfonyl)imide-N-3'hydroxypropyl-N,N-dimethyltetradecylam monium>

Synthesis of bis(nonafluorobutanesulfonyl)imide-N-3'hydroxypropyl-N,N-dimethyltetradecylam monium was performed according to the following scheme.

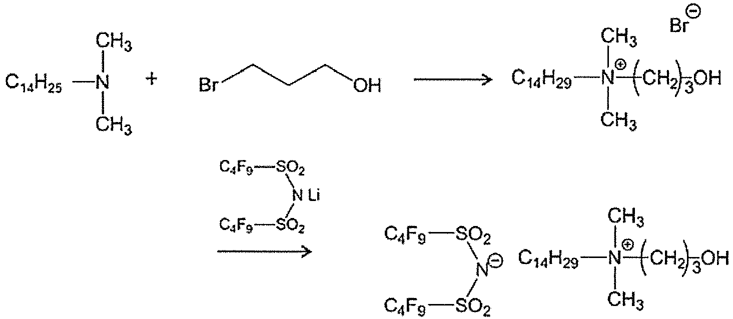

Into a flask that had been tightly sealed, N,N-dimethyltetradecylamine (6.83 g) and 3-bromopropanol (5.80 g) were charged and were allowed to react at 90°C for 5 hours. After reaction was completed, the temperature was returned to normal temperature, the resultant became a viscous liquid. After n-hexane was added to the liquid, a precipitated product was found. A supernatant was removed through decantation. This procedure was repeated for 3 times to purify the product. As a result, N,N-dimethyl-N-3'hydroxypropyltetradecylammonium bromide (9.50 g) was obtained. Yield: 89.2%.

Peaks of the product in deuterated chloroform determined through the proton (¹H)NMR are presented below.
¹H-NMR(CDCl₃,δppm);0.852(t/J=6.4Hz,3H), 1.180-1.400(m,22H), 1.670-1.760(m,2H), 1.871(brs,1H), 2.005-2.090(m,2H), 3.276(s,6H), 3.330-3.410(m,2H) 3.700-3.790(m,4H)

From these spectra, the product was identified as N,N-dimethyl-N-3'hydroxypropyltetradecyl bromide.

In pure water that had been heated, N,N-dimethyl-N-3'hydroxypropyltetradecylammonium bromide (3.50 g) was dissolved. Then, a product obtained by dissolving bis(nonafluorobutanesulfonyl)imide lithium (5.40 g) in pure water was added thereto. The resultant was heated to reflux for 1 hour. After cooling, the resultant was extracted with dichloromethane. The organic layer was washed with pure water until a result of the AgNO₃ test became negative. After the organic layer was dried with anhydrous sodium sulfate and the solvent was removed. The resultant was subjected to vacuum drying at 100°C for 24 hours to obtain bis(nonafluorobutanesulfonyl)imide-N-3'hydroxypropyl-N,N-dimethyltetradecylam monium (7.90 g) that was a colorless liquid. Yield: 97.5%.

The FTIR absorption of the product and the assignment are presented below.

Symmetric stretching vibrations of SNS were observed at 1,075 cm⁻¹, symmetric stretching vibrations of a SO₂ bond were observed at 1,140 cm⁻¹, symmetric stretching vibrations of CF₂ were observed at 1,199 cm⁻¹ and 1,237 cm⁻¹, asymmetric stretching vibrations of a SO₂ bond were observed at 1,354 cm⁻¹, bending vibrations of CH₂ were observed at 1,470 cm⁻¹, symmetric stretching vibrations of CH₂ were observed at 2,858 cm⁻¹, asymmetric stretching vibrations of CH₂ were observed at 2,929 cm⁻¹, and stretching vibrations of OH were observed at 3,549 cm⁻¹.

Peaks of the obtained compound in deuterated chloroform determined through the proton (¹H)NMR and the carbon (¹³C)NMR are presented below.
¹H-NMR(CDCl₃,δppm);0.842(t/J=6.8Hz,3H), 1.170-1.320(m,22H), 1.610-1.710(m,2H), 1.867-1.937(m,2H), 2.538(brs,1H), 2.993(s,6H), 3.149-3.192(m,2H), 3.351-3.392(m,2H), 3.665(t/J=5.4Hz,2H)
¹³C-NMR(CDCl₃,δppm);14.003, 22.628, 25.454, 26.097, 28.990, 29.297, 29.364, 29.527, 29.585, 29.613, 31.865, 50.705, 58.266, 62.358, 64.887

From these spectra, the product was identified as bis(nonafluorobutanesulfonyl)imide-N-3'hydroxypropyl-N,N-dimethyltetradecylam monium.

Note that, a pKa of an acid [bis(nonafluorobutanesulfonyl)imide] that is a source of a conjugate base in bis(nonafluorobutanesulfonyl)imide-N-3'hydroxypropyl-N,N-dimethyltetradecylam monium in acetonitrile is 0.0.

### (Example 10A)

### <Synthesis of bis(nonafluorobutanesulfonyl)imide-N-3'hydroxypropyl-N,N-dimethyloctadecylamm onium>

Synthesis of bis(nonafluorobutanesulfonyl)imide-N-3'hydroxypropyl-N,N-dimethyloctadecylamm onium was performed according to the following scheme.

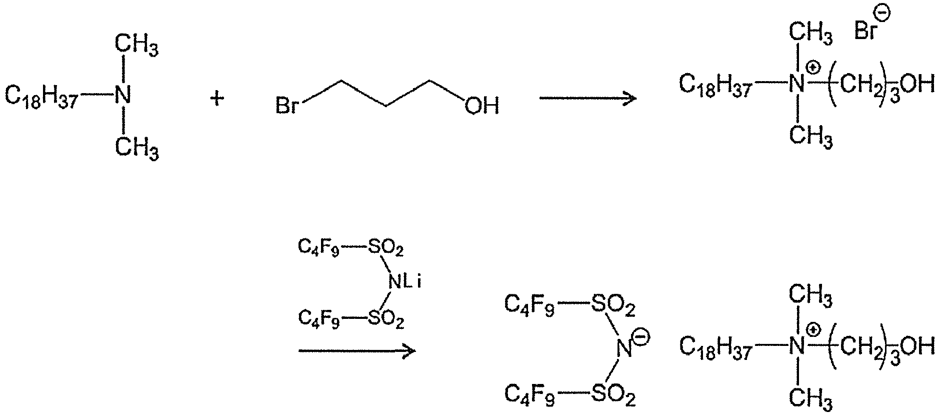

Into a flask that had been tightly sealed, N,N-dimethyloctadecylamine (8.71 g) and 3-bromopropanol (4.19 g) were charged and were allowed to react at 120°C for 2 hours. The temperature was returned to normal temperature to precipitate a crystal. The crystal was recrystallized from a mixture solvent of ethyl acetate and ethanol to obtain N-3'hydroxypropyl-N,N-dimethyloctadecylammonium bromide (11.28 g). Yield: 88.2%.

The FTIR absorption of the product and the assignment are presented below.

Bending vibrations of CH₂ were observed at 1,472 cm⁻¹, symmetric stretching vibrations of CH₂ were observed at 2,851 cm⁻¹, asymmetric stretching vibrations of CH₂ were observed at 2,918 cm⁻¹, and stretching vibrations of a hydroxyl group were observed at 3,296 cm⁻¹.

Peaks of the obtained compound in deuterated chloroform determined through the proton (¹H)NMR and the carbon (¹³C)NMR are presented below.
¹H-NMR(CDCl₃,δppm);0.844(t/J=7.4Hz,3H), 1.170-1.400(m,30H), 1.670-1.760(m,2H), 2.008-2.077(m,2H), 3.277(s,6H), 3.350-3.420(m,2H) 3.700-3.790(m,4H)
¹³C-NMR(CDCl₃,δppm);14.080, 22.656, 22.800, 25.962, 26.298, 29.192, 29.326, 29.393, 29.460, 29.633, 29.671, 31.884, 51.222, 58.208, 62.722, 64.657

From these spectra, the product was identified as N-3'hydroxypropyl-N,N-dimethyloctadecylammonium bromide.

In pure water that had been heated, N,N-dimethyl-N-3'hydroxypropyloctadecylammonium bromide (3.16 g) was dissolved. Then, a product obtained by dissolving lithium bis(nonafluorobutanesulfonyl)imide (4.51 g) in pure water was added thereto. The resultant was heated to reflux for 1 hour. After cooling, the resultant was extracted with dichloromethane. The organic layer was washed with pure water until a result of the AgNO₃ test became negative. After the organic solvent was dried with anhydrous sodium sulfate and the solvent was removed. The resultant was subjected to vacuum drying at 100°C for 60 hours to obtain bis(nonafluorobutanesulfonyl)imide-N-3'hydroxypropyl-N,N-dimethyloctadecylamm onium (6.59 g) that was a colorless liquid. Yield: 97.1%.

The FTIR absorption of the product and the assignment are presented below.

Symmetric stretching vibrations of SNS were observed at 1,076 cm⁻¹, symmetric stretching vibrations of a SO₂ bond were observed at 1,140 cm⁻¹, symmetric stretching vibrations of CF₂ were observed at 1,200 cm⁻¹ and 1,237 cm⁻¹, asymmetric stretching vibrations of a SO₂ bond were observed at 1,354 cm⁻¹, bending vibrations of CH₂ were observed at 1,469 cm⁻¹, symmetric stretching vibrations of CH₂ were observed at 2,856 cm⁻¹, asymmetric stretching vibrations of CH₂ were observed at 2,927 cm⁻¹, and stretching vibrations of OH were observed at 3,551 cm⁻¹.

Peaks of the obtained compound in CDCl₃ determined through the proton (¹H)NMR and the carbon (¹³C)NMR are presented below.
¹H-NMR(CDCl₃,δppm);0.854(t/J=7.0Hz,3H), 1.180-1.340(m,30H), 1.630-1.730(m,2H),1.895-1.963(m,2H), 3.026(s,6H), 3.166-3.209(m,2H), 3.396-3.437(m,2H), 3.700(t/J=6.6Hz,2H)
¹³C-NMR(CDCl₃,δppm);14.089, 22.666, 25.483, 26.010, 29.019, 29.307, 29.345, 29.393, 29.556, 29.680, 31.904, 50.858, 58.323, 62.482, 64.935

From these spectra, the product was identified as bis(nonafluorobutanesulfonyl)imide-N-3'hydroxypropyl-N,N-dimethyloctadecylamm onium.

Note that, a pKa of an acid [bis(nonafluorobutanesulfonyl)imide] that is a source of a conjugate base in bis(nonafluorobutanesulfonyl)imide-N-3'hydroxypropyl-N,N-dimethyloctadecylamm onium in acetonitrile is 0.0.

### (Comparative Example 1A)

### <Synthesis of nonafluorobutanesulfonic acid-1-octadecylimidazolium>

Nonafluorobutanesulfonic acid-1-octadecylimidazolium was synthesized according to the following scheme.

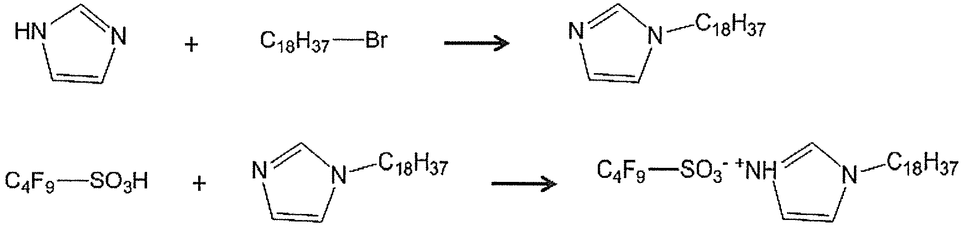

In ethanol (50 mL), 1-octadecylimidazole (3.27 g) synthesized in Example 1A was dissolved. Then, an ethanol solution of nonafluorobutanesulfonic acid (3.05 g) was gradually added dropwise. After the dropping was completed, the resultant was stirred for 30 minutes and was heated to reflux for 1 hour. After the solvent was removed, a mixture solvent of ethanol/n-hexane was used for recrystallization to obtain nonafluorobutanesulfonic acid-1-octadecylimidazolium that was colorless. Yield: 95%.

The assignment of the FTIR spectra of the product is presented below.

Symmetric stretching vibrations of SO₂ were observed at 1,134 cm⁻¹, asymmetric stretching vibrations of a SO₂ bond were observed at 1,355 cm⁻¹, symmetric stretching vibrations of CF₂ were observed at 1,246 cm⁻¹, bending vibrations of a CH bond were observed at 1,470 cm⁻¹, symmetric stretching vibrations of CH₂ were observed at 2,852 cm⁻¹, asymmetric stretching vibrations of CH₂ were observed at 2,920 cm⁻¹, and stretching vibrations of a CH bond of an imidazole ring were observed at 3,158 cm⁻¹.

Peaks of the product in deuterated chloroform determined through the proton (¹H)NMR and the carbon (¹³C)NMR are presented below.
¹H-NMR(CDCl₃,δppm);0.847(t,3H, J=7.2Hz), 1.222-1.282(m,30H), 1.790-1.890(m,2H), 4.181(t/J=7.2Hz,2H), 7.189(dd/J=1.8Hz,3.8Hz,1H), 7.444(dd/J=1.8Hz,3.8Hz,1H), 8.866(d/J=1.8Hz,3.8Hz,1H), 13.200(brs,1H)
¹³C-NMR(CDCl₃,δppm);14.055, 22.648, 26.113, 28.875, 29.272, 29.318, 29.440, 30.142, 31.882, 49.847, 122.500, 122.851, 135.015

As described above, it could be confirmed that nonafluorobutanesulfonic acid-1-octadecylimidazolium was synthesized.

### (Comparative Example 2A)

### <Synthesis of hexafluorocyclopropane-1,3-bis(sulfonyl)imide-1-butyl-3-n-octadecylimidazolium>

For comparison, synthesis of hexafluorocyclopropane-1,3-bis(sulfonyl)imide-1-butyl-3-n-octadecylimidazolium was performed according to the following scheme.

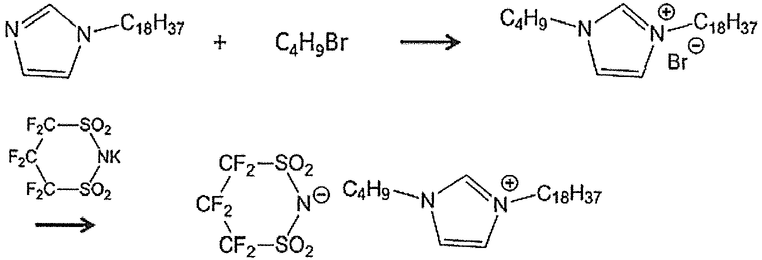

In acetonitrile, 1-octadecylimidazole (10.7 g) synthesized in Example 1A and bromobutane (6.03 g) were dissolved and were heated to reflux for 5 hours. After the solvent was removed, recrystallization was performed from a mixture solvent of n-hexane and ethanol to obtain 1-butyl-3-octadecylimidazolium bromide. This bromide (4.57 g) was dissolved in ethanol and an ethanol solution of potassium hexafluorocyclopropane-1,3-bis(sulfonyl)imide (3.31 g) was added thereto. Upon stirring, a colorless precipitated product formed. The solution was heated to reflux for 1 hour. After cooling, the solvent was removed. Then, dichloromethane was added thereto and a dissolved portion was filtrated. The organic layer was washed with pure water until a result of the AgNO₃ test became negative. The resultant was dried and was recrystallized from a mixture solvent of n-hexane and ethanol to obtain hexafluorocyclopropane-1,3-bis(sulfonyl)imide-1-butyl-3-n-octadecylimidazolium (6.00 g) that was a colorless crystal. Yield: 90%.

The FTIR absorption of the product and the assignment are presented below.

Symmetric stretching vibrations of SO₂ were observed at 1,091 cm⁻¹, symmetric stretching vibrations of CF₂ were observed at 1,161 cm⁻¹, asymmetric stretching vibrations of a SO₂ bond were observed at 1,356 cm⁻¹, bending vibrations of a CH bond were observed at 1,470 cm⁻¹, stretching vibrations specific to imidazole were observed at 1,560 cm⁻¹, symmetric stretching vibrations of CH₂ were observed at 2,850 cm⁻¹, and asymmetric stretching vibrations of CH₂ were observed at 2,919 cm⁻¹.

Peaks of the compound in deuterated chloroform determined through the proton (¹H)NMR and the carbon (¹³C)NMR, and the assignment are presented below.
¹H-NMR(CDCl₃,δppm);0.850(t,3H,J=7.2Hz), 0.941(t,3H,J=7.2Hz), 1.170-1.410(m,32H), 1.835(quint,4H,J=7.2Hz), 4.160(m,4H), 7.267(d,1H,J=2.1Hz), 7.294(d,1H,J=2.1Hz), 8.749(s,1H)
¹³C-NMR(CDCl₃,δppm);13.254, 14.085, 19.351, 22.663, 26.113, 28.853, 29.303, 29.333, 29.448, 29.570, 29.631, 29.677, 30.127, 31.898, 32.004, 49.977, 50.244, 122.179, 122.263, 135.473

From these spectra, the product was identified as hexafluorocyclopropane-1,3-bis(sulfonyl)imide-1-butyl-3-n-octadecylimidazolium.

Note that, a pKa of an acid [hexafluorocyclopropane-1,3-bis(sulfonyl)imide] that is a source of a conjugate base in hexafluorocyclopropane-1,3-bis(sulfonyl)imide-1-butyl-3-n-octadecylimidazolium in acetonitrile is -0.8.

### (Comparative Example 3A)

### <Synthesis of heptadecafluorooctanesulfonic acid-6-octadecyl-1,8-diazabicyclo[5.4.0]-7-undecenium>

For comparison, synthesis of heptadecafluorooctanesulfonic acid-6-octadecyl-1,8-diazabicyclo[5.4.0]-7-undecenium was performed according to the following scheme.

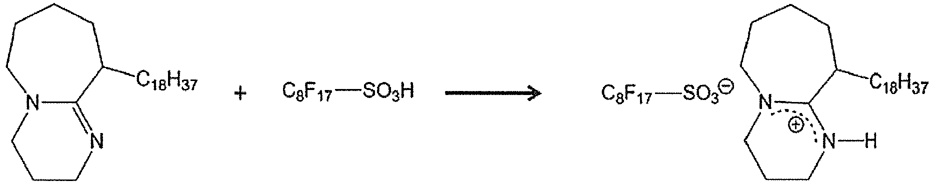

To an ethanol solution of 6-octadecyl-1,8-diazabicyclo[5.4.0]-7-undecene (4.04 g) synthesized in Example 4A, heptadecafluorooctanesulfonic acid (5.00 g) was added. The resultant was stirred at normal temperature for 1 hour and was heated to reflux for 1 hour. After the solvent was removed, the resultant was dissolved in dichloromethane and was sufficiently washed with water. The organic layer was dried with anhydrous sodium sulfate and the solvent was removed. Recrystalization was performed from a mixture solvent of n-hexane and ethanol to obtain heptadecafluorooctanesulfonic acid-6-octadecyl-1,8-diazabicyclo[5.4.0]-7-undecenium (7.86 g). Yield: 86.9%.

The FTIR absorption of the product and the assignment are presented below.

Symmetric stretching vibrations of SO₂ were observed at 1,055 cm⁻¹, symmetric stretching vibrations of CF₂ were observed at 1,252 cm⁻¹, asymmetric stretching vibrations of a SO₂ bond were observed at 1,368 cm⁻¹, bending vibrations of CH₂ were observed at 1,467 cm⁻¹, stretching vibrations of C=N were observed at 1,643 cm⁻¹, symmetric stretching vibrations of CH₂ were observed at 2,851 cm⁻¹, asymmetric stretching vibrations of CH₂ were observed at 2,920 cm⁻¹, and NH stretching vibrations were observed at 3,296 cm⁻¹.

Peaks of the obtained compound in deuterated chloroform determined through the proton (¹H)NMR and the carbon (¹³C)NMR are presented below.
¹H-NMR(CDCl₃,δppm);0.843(t,3H,J=6.6Hz), 1.205-1.287(m,32H), 1.544-1.800(m,8H), 1.975-2.033(m,2H), 2.792-2.816(m,1H), 3.440-3.559(m,6H), 8.713(brs,1H)
¹³C-NMR(CDCl₃,δppm);14.024, 19.336, 22.633, 25.121, 26.311, 27.181, 28.311, 29.028, 29.303, 29.425, 29.532, 29.608, 29.654, 31.882, 38.491, 43.375, 49.725, 53.785, 168.029

From these spectra, the product was identified as heptadecafluorooctanesulfonic acid-6-octadecyl-1,8-diazabicyclo[5.4.0]-7-undecenium.

Note that, a pKa of an acid [heptadecafluorooctanesulfonic acid] that is a source of a conjugate base in heptadecafluorooctanesulfonic acid-6-octadecyl-1,8-diazabicyclo[5.4.0]-7-undecenium in acetonitrile is 0.7.

### (Comparative Example 4A)

### <Synthesis of hexafluorocyclopropane-1,3-bis(sulfonyl)imide-6-octadecyl-1,8-diazabicyclo[5.4.0]-7-undecenium>

For comparison, synthesis of hexafluorocyclopropane-1,3-bis(sulfonyl)imide-6-octadecyl-1,8-diazabicyclo[5.4.0]-7-undecenium was performed according to the following scheme.

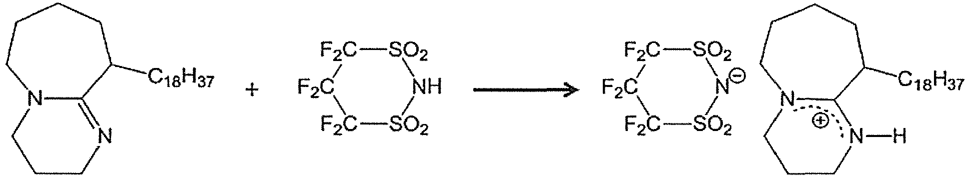

To an ethanol solution of 6-octadecyl-1,8-diazabicyclo[5.4.0]-7-undecene (2.18 g) synthesized in Example 4A, hexafluorocyclopropane-1,3-bis(sulfonyl)imide (3.00 g) was added. The resultant was stirred at normal temperature for 1 hour and was heated to reflux for 1 hour. After the solvent was removed, the resultant was dissolved in dichloromethane and the organic layer was sufficiently washed with water. After the organic layer was dried with anhydrous sodium sulfate, the solvent was removed. The resultant was subjected to vacuum drying at 90°C for 3 days to obtain hexafluorocyclopropane-1,3-bis(sulfonyl)imide-6-octadecyl-1,8-diazabicyclo[5.4.0]-7-undecenium (4.86 g) that was a colorless crystal. Yield: 93.8%.

The FTIR absorption of the product and the assignment are presented below.

Asymmetric stretching vibrations of SNS were observed at 1,042 cm⁻¹, symmetric stretching vibrations of SO₂ were observed at 1,091 cm⁻¹, symmetric stretching vibrations of CF₂ were observed at 1,164 cm⁻¹, asymmetric stretching vibrations of a SO₂ bond were observed at 1,360 cm⁻¹, stretching vibrations of C=N were observed at 1,633 cm⁻¹, symmetric stretching vibrations of CH₂ were observed at 2,848 cm⁻¹, asymmetric stretching vibrations of CH₂ were observed at 2,920 cm⁻¹, and NH stretching vibrations were observed at 3,387 cm⁻¹.

Peaks of the obtained compound in deuterated chloroform determined through the proton (¹H)NMR and the carbon (¹³C)NMR are presented below.
¹H-NMR(CDCl₃,δppm);0.869(t,3H,J=6.6Hz), 1.170-1.340(m,32H), 1.441-1.555(m,2H), 1.600-1.750(m,4H), 1.772-1.832(m,2H), 1.941-2.101(m,2H), 2.670-2.780(m,1H), 3.413(t/J=6.6Hz,2H), 3.508(t/J=6.6Hz,2Hz), 3.550-3.652(m,2H)
¹³C-NMR(CDCl₃,δppm);14.055, 19.260, 22.633, 26.052, 27.090, 28.524, 29.120, 29.226, 29.318, 29.364, 29.486, 29.578, 29.608, 29.669, 31.867, 38.690, 43.177, 49.511, 53.861, 167.922

From these spectra, the product was identified as hexafluorocyclopropane-1,3-bis(sulfonyl)imide-6-octadecyl-1,8-diazabicyclo[5.4.0]-7-undecenium.

Note that, a pKa of an acid [hexafluorocyclopropane-1,3-bis(sulfonyl)imide] that is a source of a conjugate base in hexafluorocyclopropane-1,3-bis(sulfonyl)imide-6-octadecyl-1,8-diazabicyclo[5.4.0]-7-undecenium in acetonitrile is -0.8.

### (Comparative Example 5A)

### <Synthesis of pentadecafluorooctanoic acid octadecylammonium>

For comparison, synthesis of pentadecafluorooctanoic acid octadecylammonium was performed according to the following scheme.

C₇F₁₅-COOH + C₁₈H₃₇-NH₂ → C₇F₁₅-COO⁻ H₃N⁺-C₁₈H₃₇

Pentadecafluorooctanoic acid (4.14 g) and octadecylamine (2.69 g) were added to ethanol and were heated to reflux for 1 hour. After the solvent was removed, recrystallization was performed from a mixture solvent of n-hexane and ethanol to obtain a colorless, platelike crystal (6.23 g). Yield: 92.0%.

The FTIR absorption of the product and the assignment are presented below.

Symmetric stretching vibrations of CF₂ were observed at 1,141 cm⁻¹, 1,201 cm⁻¹, and 1,232 cm⁻¹, bending vibrations of CH₂ were observed at 1,473 cm⁻¹, stretching vibrations of C=O were observed at 1,677 cm⁻¹, symmetric stretching vibrations of CH₂ were observed at 2,851 cm⁻¹, asymmetric stretching vibrations of CH₂ were observed at 2,918 cm⁻¹, and NH₄⁺ stretching vibrations were observed at 3,000 to 3,325 cm⁻¹.

Peaks of the obtained compound in deuterated chloroform determined through the proton (¹H)NMR and the carbon (¹³C)NMR are presented below.
¹H-NMR(CD₃OD,δppm);0.890(t/J=6.6Hz,3H), 1.214-1.408(m,30H), 1.590-1.690(m,2H), 2.896(t/J=7.5Hz,2H), 4.891(brs)
¹³C-NMR(CD₃OD,δppm);14.444, 23.740, 27.464, 28.578, 30.242, 30.486, 30.516, 30.669, 30.791, 33.081, 40.758

From these spectra, the product was identified as pentadecafluorooctanoic acid octadecylammonium.

Note that, a pKa of an acid [pentadecafluorooctanoic acid] that is a source of a conjugate base in pentadecafluorooctanoic acid octadecylammonium in acetonitrile is 12.7.

### (Comparative Example 6A)

### <Synthesis of nonafluorobutanesulfonic acid-1-octadecyl-2-heptadecylimidazolium>

Nonafluorobutanesulfonic acid-1-octadecyl-2-heptadecylimidazolium was synthesized according to the following scheme.

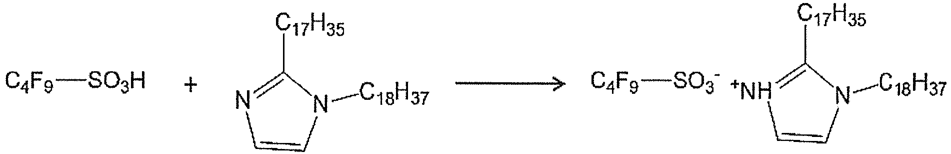

In ethanol, 1-octadecyl-2-heptadecylimidazole (4.45 g) synthesized in Example 8A was dissolved. Then, a product obtained by dissolving nonafluorobutanesulfonic acid (2.40 g) in ethanol was gradually added dropwise. After the dropping was completed, the resultant was stirred for 30 minutes and was heated to reflux for 1 hour. After the solvent was removed, a mixture solvent of ethanol/n-hexane was used for recrystallization to obtain a nonafluorobutanesulfonic acid-1-octadecyl-2-heptadecylimidazole salt (6.43 g) that was colorless. Yield: 93.9%.

The assignment of the FTIR spectra of the product is presented below.

Symmetric stretching vibrations of SO₂ were observed at 1,135 cm⁻¹, symmetric stretching vibrations of CF₂ were observed at 1,279 cm⁻¹, asymmetric stretching vibrations of a SO₂ bond were observed at 1,357 cm⁻¹, bending vibrations of CH₂ were observed at 1,472 cm⁻¹, symmetric stretching vibrations of CH₂ were observed at 2,851 cm⁻¹, asymmetric stretching vibrations of CH₂ were observed at 2,918 cm⁻¹, and NH⁺ stretching vibrations were observed at 3,152 cm⁻¹.

Peaks of the obtained compound in CDCl₃ determined through the proton (¹H)NMR and the carbon (¹³C)NMR are presented below.
¹H-NMR(CDCl₃,δppm);0.848(t/J=6.8Hz,6H), 1.171-1.307(m,58H), 1.702-1.817(m,4H), 2.908(t/J=7.5Hz,2H), 4.003(t/J=7.5Hz,2H), 7.139(t/J=2.0Hz,1H), 7.255(t/J=2.0Hz,1H), 13.285(brs,1H)
¹³C-NMR(CDCl₃,δppm);14.055, 22.648, 24.602, 26.326, 27.196, 28.952, 28.998, 29.333, 29.455, 29.516, 29.562, 29.669, 30.051, 31.882, 47.725, 118.928, 120.866, 144.149

As described above, it could be confirmed that nonafluorobutanesulfonic acid-1-octadecyl-2-heptadecylimidazolium was synthesized.

Note that, a pKa of an acid [nonafluorobutanesulfonic acid] that is a source of a conjugate base in nonafluorobutanesulfonic acid-1-octadecyl-2-heptadecylimidazolium in acetonitrile is 0.7.

### (Comparative Example 7A)

### <Synthesis of hexafluorocyclopropane-1,3-bis(sulfonyl)imide-1-octadecyl-2-heptadecylimidazolium >

For comparison, synthesis of hexafluorocyclopropane-1,3-bis(sulfonyl)imide-1-octadecyl-2-heptadecylimidazolium was performed according to the following scheme.

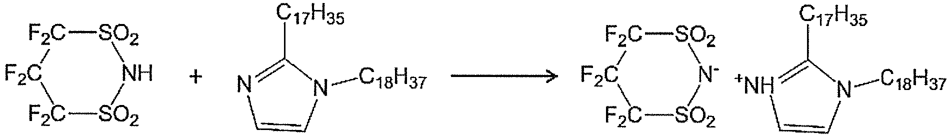

In ethanol, 1-octadecyl-2-heptadecylimidazole (3.46 g) synthesized in Example 8A was dissolved. Then, a product obtained by dissolving hexafluorocyclopropane-1,3-bis(sulfonyl)imide (1.82 g) in ethanol was gradually added dropwise thereto. After the dropping was completed, the resultant was stirred for 30 minutes and was heated to reflux for 1 hour. After the solvent was removed, a mixture solvent of ethanol/n-hexane was used for recrystallization to obtian hexafluorocyclopropane-1,3-disulfonylimide-1-octadecyl-2-heptadecylimidazolium (5.05 g) that was colorless. Yield: 96%.

The assignment of the FTIR spectra of the product is presented below.

Symmetric stretching vibrations of SO₂ were observed at 1,086 cm⁻¹, symmetric stretching vibrations of CF₂ were observed at 1,164 cm⁻¹, asymmetric stretching vibrations of a SO₂ bond were observed at 1,364 cm⁻¹, symmetric stretching vibrations of a C=N bond were observed at 1,469 cm⁻¹, symmetric stretching vibrations of CH₂ were observed at 2,851 cm⁻¹, asymmetric stretching vibrations of CH₂ were observed at 2,920 cm⁻¹, and stretching vibrations of a NH bond were observed at 3,162cm⁻¹ and 3,289 cm⁻¹.

Peaks of the product in deuterated chloroform determined through the proton (¹H)NMR and the carbon (¹³C)NMR are presented below.
¹H-NMR(CDCl₃, δppm);0.853(t,6H,J=6.8Hz), 1.182-1.323(m,58H), 1.652 - 1.833(m,4H), 2.913(t,2H,J=7.5Hz), 4.007(t,2H,J=7.5Hz), 7.126(t,1H,J=1.6Hz), 7.274(t,1H,J=1.6Hz), 11.820(brs,1H)
¹³C-NMR(CDCl₃,δppm);14.065, 22.658, 24.566, 26.321, 27.420, 28.931, 29.297, 29.326, 29.450, 29.496, 29.557, 29.633, 29.679, 30.015, 31.892, 47.888, 119.044, 120.891, 147.159

From these spectra, the product was identified as hexafluorocyclopropane-1,3-bis(sulfonyl)imide-1-octadecyl-2-heptadecylimidazolium

Note that, a pKa of an acid [hexafluorocyclopropane-1,3-bis(sulfonyl)imide] that is a source of a conjugate base in hexafluorocyclopropane-1,3-bis(sulfonyl)imide-1-octadecyl-2-heptadecylimidazolium in acetonitrile is -0.8.

### (Comparative Example 8A)

### <Synthesis of bis(nonafluorobutanesulfonyl)imide-octadecylammonium>

For comparison, synthesis of bis(nonafluorobutanesulfonyl)imide-octadecylammonium was performed according to the following scheme.

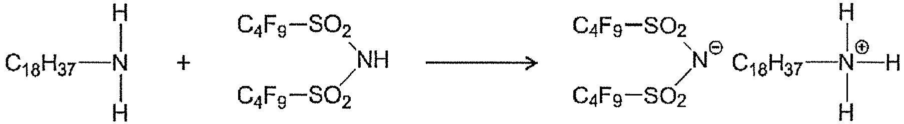

Octadecylamine was dissolved in ethanol and an ethanol solution of equimolar bis(nonafluorobutanesulfonyl)imide was added thereto. The resultant was heated to reflux for 1 hour. After cooling, the solvent was removed. The residue was extracted with dichloromethane and the organic layer was sufficiently washed with water. After the organic layer was dried with anhydrous sodium sulfate, the solvent was removed and recrystallization was performed from a mixture solvent of n-hexane and ethanol to obtain a colorless crystal of bis(nonafluorobutanesulfonyl)imide-octadecylammonium. Yield: 91.1%.

The FTIR absorption of the product and the assignment are presented below.

Asymmetric stretching vibrations of a SNS bond were observed at 1,031 cm⁻¹, symmetric stretching vibrations of a SO₂ bond were observed at 1,088 cm⁻¹, symmetric stretching vibrations of CF₂ were observed at 1,141 cm⁻¹ and 1,200 cm⁻¹, asymmetric stretching vibrations of a SO₂ bond were observed at 1,355 cm⁻¹, bending vibrations of CH₂ were observed at 1,473 cm⁻¹, bending vibrations of NH₃⁺ were observed at 1,616 cm⁻¹, symmetric stretching vibrations of CH₂ were observed at 2,856 cm⁻¹, asymmetric stretching vibrations of CH₂ were observed at 2,926 cm⁻¹, and NH₃⁺ stretching vibrations were observed at 3,248 cm⁻¹.

Peaks of the obtained compound in deuterated methanol (CD₃OD) determined through the proton (¹H)NMR and the carbon (¹³C)NMR are presented below.
¹H-NMR(CD₃OD,δppm);0.889(t/J=6.8Hz,3H), 1.225-1.400(m,30H), 1.635(quint/J=7.5Hz,2H), 2.896(d/J=7.5Hz,2H), 4.867(brs,3H)
¹³C-NMR(CD₃OD,δppm);14.414, 23.709, 27.403, 28.563, 30.196, 30.471, 30.623, 30.776, 33.065, 40.819

From these spectra, the product was identified as bis(nonafluorobutanesulfonyl)imide-octadecylammonium.

Note that, a pKa of an acid [bis(nonafluorobutanesulfonyl)imide] that is a source of a conjugate base in bis(nonafluorobutanesulfonyl)imide-octadecylammonium in acetonitrile is 0.0.

### (Comparative Example 9A)

### <Synthesis of nonafluorobutanesulfonic acid-N,N,N-trimethyloctadecylammonium>

For comparison, synthesis of nonafluorobutanesulfonic acid-N,N,N-trimethyloctadecylammonium was performed according to the following scheme.

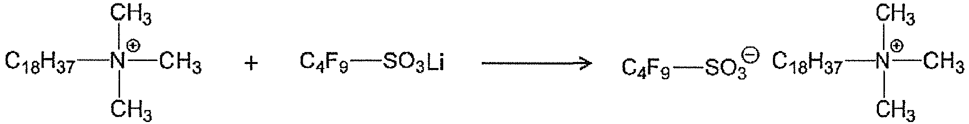

Trimethyloctadecylammonium bromide (5.51 g) was dissolved in water and an aqueous solution of lithium nonafluorobutanesulfonate (4.61 g) was added thereto. The resultant was heated to reflux for 1 hour. After cooling, the precipitated product was extracted with dichloromethane. The organic layer was sufficiently washed with water until a result of the washing liquid became negative through the AgNO₃ test. After the organic layer was dried with anhydrous sodium sulfate, the solvent was removed to obtain a colorless crystal of nonafluorobutanesulfonic acid-trimethyloctadecylammonium (8.14 g). Yield: 94.8%. Recrystallization was performed from a mixture solvent of n-hexane and ethanol.

The FTIR absorption of the product and the assignment are presented below.

Symmetric stretching vibrations of a SO₂ bond were observed at 1,133 cm⁻¹, symmetric stretching vibrations of CF₂ were observed at 1,263 cm⁻¹, asymmetric stretching vibrations of a SO₂ bond were observed at 1,353 cm⁻¹, bending vibrations of CH₂ were observed at 1,485 cm⁻¹, symmetric stretching vibrations of CH₂ were observed at 2,852 cm⁻¹, and asymmetric stretching vibrations of CH₂ were observed at 2,920 cm⁻¹.

Peaks of the obtained compound in CD₃OD determined through the proton (¹H)NMR and the carbon (¹³C)NMR are presented below.
¹H-NMR(CD₃OD, δppm);0.851(t/J=6.8Hz,3H), 1.170-1.340(m,30H), 1.640 - 1.730(m,2H), 3.181(s,9H), 3.291-3.334(m,2H)
¹³C-NMR(CDCl₃,δppm);14.089, 22.666, 23.068, 25.972, 29.067, 29.316, 29.335, 29.412, 29.565, 29.680, 31.904, 53.043, 66.996

From these spectra, the product was identified as nonafluorobutanesulfonic acid-N, N,N-trimethyloctadecylammonium.

Note that, a pKa of an acid (nonafluorobutanesulfonic acid) that is a source of a conjugate base in nonafluorobutanesulfonic acid-N,N,N-trimethyloctadecylammonium in acetonitrile is 0.7.

The ionic liquids synthesized in the above Examples and Comparative Examples are summarized below.

| | |
|---|---|
| **Example 1A** | **Comparative Example 1A** |
| 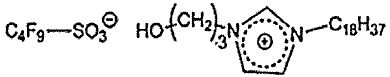 | 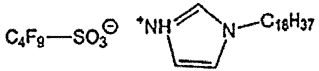 |
| **Example 2A** | **Comparative Example 2A** |
| 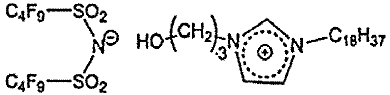 | 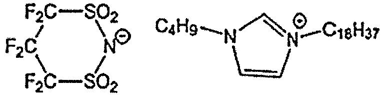 |
| **Example 3A** | **Comparative Example 3A** |
| 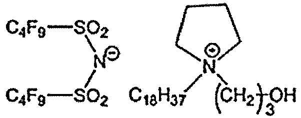 | 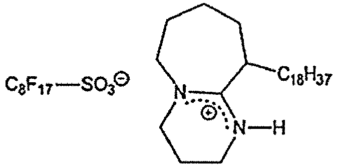 |
| **Example 4A** | **Comparative Example 4A** |
| 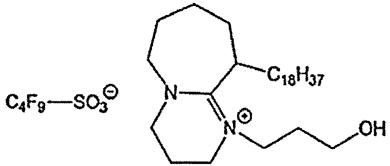 | 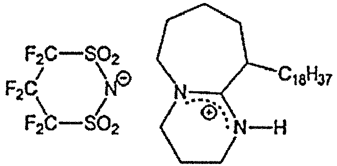 |
| **Example 5A** | **Comparative Example 5A** |
| 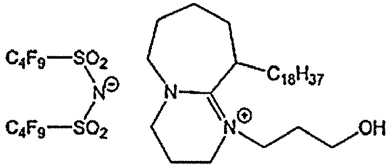 | C₇F₁₅-COO⁻H₃N⁺-C₁₈H₃₇ |
| **Example 6A** | **Comparative Example 6A** |
| 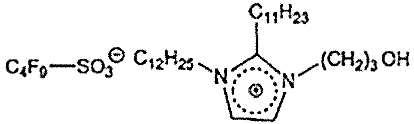 | 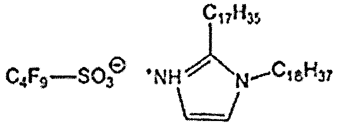 |
| **Example 7A** | **Comparative Example 7A** |
| 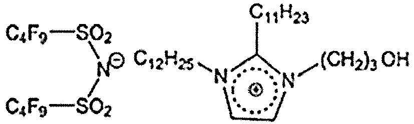 | 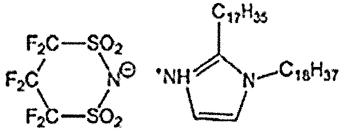 |
| **Example 8A** | **Comparative Example 8A** |
| 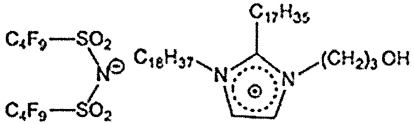 | 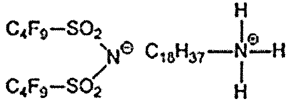 |
| **Example 9A** | **Comparative Example 9A** |
| 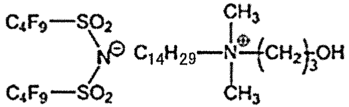 | 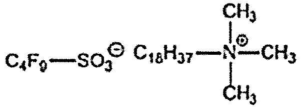 |
| **Example 10A** | |
| 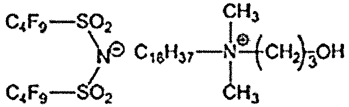 | |

### (Example 1B to Example 5B, Comparative Example 1B to Comparative Example 5B, Comparative Example 10B, and Comparative Example 11B)

### <Measurement results of solubility to fluorine-based solvent>

The ionic liquids synthesized in Examples and Comparative Examples, Z-DOL, and Z-TETRAOL were subjected to the solubility examination using a Vertrel XF [CF₃(CHF)₂CF₂CF₃] available from Du Pont-Mitsui Fluorochemicals Company, Ltd. as a fluorine-based solvent.

To a predetermined mass of Vertrel XF, each of the ionic liquids, Z-DOL, or Z-TETRAOL was added and was irradiated with ultrasonic waves for 5 minutes. Then, the resultant was left to stand for 1 day and solubility thereof was visually observed.

Specifically, to 100 parts by mass of Vertrel XF (25°C), each of the ionic liquids, Z-DOL, or Z-TETRAOL was added in an amount of 1.0 part by mass, 0.5 parts by mass, and 0.1 parts by mass, and was irradiated with ultrasonic waves for 5 minutes. Then, the resultant was left to stand for 1 day and solubility thereof was visually observed, followed by evaluation based on the following evaluation criteria.

Note that, it was judged to be dissolved when the solution was found to be transparent by visual observation. It was judged not to be dissolved (no dissolution) when the solution was found to be opaque or to have an insoluble portion.

Results are presented in Table 2.

### [Evaluation criteria]

- 0.5% by mass or more:
   When 0.5 parts by mass of the ionic liquid was added, dissolution was found.
- 0.1% by mass or more but less than 0.5% by mass:
   When 0.5 parts by mass of the ionic liquid was added, no dissolution was found. However, when 0.1 parts by mass of the ionic liquid was added, dissolution was found.
- Less than 0.1% by mass:
   When 0.5 parts by mass of the ionic liquid was added, no dissolution was found. Moreover, when 0.1 parts by mass of the ionic liquid was added, no dissolution was found.

**Table 2-1**

| Example | Synthesized product | Fluorine-based solvent solubility |
|---|---|---|
| Example 1B | Example 1A | Less than 0.1% by mass |
| Example 2B | Example 2A | 0.1% by mass or more but less than 0.5% by mass |
| Example 3B | Example 3A | 0.5% by mass or more |
| Example 4B | Example 4A | 0.1% by mass or more but less than 0.5% by mass |
| Example 5B | Example 5A | 0.1% by mass or more but less than 0.5% by mass |
| Comparative Example 1B | Comparative Example 1A | Less than 0.1% by mass |
| Comparative Example 2B | Comparative Example 2A | Less than 0.1% by mass |
| Comparative Example 3B | Comparative Example 3A | Less than 0.1% by mass |
| Comparative Example 4B | Comparative Example 4A | Less than 0.1% by mass |
| Comparative Example 5B | Comparative Example 5A | Less than 0.1% by mass |
| Comparative Example 10B | Z-DOL | 0.5% by mass or more |
| Comparative Example 11B | Z-TETRAOL | 0.5% by mass or more |

Solubility of the ionic liquid of Example 1A to the fluorine-based solvent was less than 0.1% by mass.

Solubility of the ionic liquid of Example 2A to the fluorine-based solvent was 0.1% by mass or more but less than 0.5% by mass.

Solubility of the ionic liquid of Example 3A to the fluorine-based solvent was 0.5% by mass or more.

Solubility of the ionic liquid of Example 4A to the fluorine-based solvent was 0.1% by mass or more but less than 0.5% by mass.

Solubility of the ionic liquid of Example 5A to the fluorine-based solvent was 0.1% by mass or more but less than 0.5% by mass.

Solubility of each of the ionic liquids of Comparative Example 1A to Comparative Example 5A to the fluorine-based solvent was less than 0.1% by mass.

Solubility of Z-DOL to the fluorine-based solvent and solubility of Z-TETRAOL to the fluorine-based solvent were 0.5% by mass or more.

As understood from the above, it is found that the ionic liquids used in Examples are improved in solubility to Vertrel XF that is a fluorine-based solvent. The compounds of Examples 2A to 5A are sufficient to be used for producing hard discs.

As understood from Comparative Example 1B and Comparative Example 2B, it is found that the imidazole-based ionic liquids have a low solubility to Vertrel, but the ionic liquid of Example 2B into which a hydroxyl group has been introduced is improved in the solubility. That is, it is found that introduction of a hydroxyl group is effective in solubility to Vertrel as a molecular designing technique.

The ionic liquid having a pyrrolidine skeleton was the compound of Example 3A having bis(nonafluorobutanesulfonyl)imide as an anion, and exhibited the highest solubility of all.

Moreover, even when the same octadecyl-1,8-diazabicyclo[5.4.0]-7-undecene skeleton is included, solubility to Vertrel is low in Comparative Examples 3B and 4B, while solubility to Vertrel is improved in Examples 4B and 5B.

From research results by the present inventors, it was found that introduction of a hydroxyl group into the ionic liquid improved solubility to the fluorine-based solvent. Moreover, many of ones including bis(nonafluorobutanesulfonyl)imide as an anion have a higher solubility than ones including nonafluorobutanesulfonic acid.

### (Example 6B to Example 10B and Comparative Example 6B to Comparative Example 11B)

### <Measurement results of solubility to solvents>

The ionic liquids synthesized in Examples and Comparative Examples, Z-DOL, and Z-TETRAOL were each subjected to the solubility examination using Vertrel XF [CF₃(CHF)₂CF₂CF₃] as a fluorine-based solvent available from Du Pont-Mitsui Fluorochemicals Company, Ltd., n-hexane and ethanol, which are special grade chemicals available from JUNSEI CHEMICAL CO.,LTD..

To a predetermined mass of Vertrel XF, n-hexane, or ethanol, each of the ionic liquids was added and was irradiated with ultrasonic waves for 5 minutes. Then, the resultant was left to stand for 1 day and solubility thereof was visually observed.

Specifically, to 100 parts by mass of Vertrel XF (25°C), 0.2 parts by mass of the ionic liquid was added and was irradiated with ultrasonic waves for 5 minutes. Then, the resultant was left to stand for 1 day and solubility thereof was visually observed, followed by evaluation based on the following evaluation criteria. In the cases of n-hexane and ethanol, each of the ionic liquids (0.5 parts by mass) was added to 100 parts by mass of n-hexane or ethanol at 25°C. The resultant was irradiated with ultrasonic waves for 5 minutes. Then, the resultant was left to stand for 1 day and solubility thereof was visually observed, followed by evaluation based on the following evaluation criteria.

Note that, it was judged to be dissolved when the solution was found to be transparent by visual observation. It was judged not to be dissolved (no dissolution) when the solution was found to be opaque or to have an insoluble portion.

Results are presented in Table 2-2.

### [Evaluation criteria]

### <<Vertrel XF>>

- 0.2% by mass or more:
   When 0.2 parts by mass of the ionic liquid was added, dissolution was found.
- Less than 0.2% by mass:
   When 0.2 parts by mass of the ionic liquid was added, no dissolution was found.

### <<n-Hexane and ethanol>>

- 0.5% by mass or more:
   When 0.5 parts by mass of the ionic liquid was added, dissolution was found.
- Less than 0.5% by mass:
   When 0.5 parts by mass of the ionic liquid was added, no dissolution was found.

**Table 2-2**

| Example | Synthesized product | Solubility | | |
|---|---|---|---|---|
| | | Fluorine-based solvent | n-Hexane | Ethanol |
| Example 6B | Example 6A | 0.2% by mass or more | 0.5% by mass or more | 0.5% by mass or more |
| Example 7B | Example 7A | 0.2% by mass or more | 0.5% by mass or more | 0.5% by mass or more |
| Example 8B | Example 8A | 0.2% by mass or more | 0.5% by mass or more | 0.5% by mass or more |
| Example 9B | Example 9A | 0.2% by mass or more | 0.5% by mass or more | 0.5% by mass or more |
| Example 10B | Example 10A | 0.2% by mass or more | 0.5% by mass or more | 0.5% by mass or more |
| Comparative Example 5B | Comparative Example 5A | Less than 0.2% by mass | 0.5% by mass or more | 0.5% by mass or more |
| Comparative Example 6B | Comparative Example 6A | Less than 0.2% by mass | Less than 0.5% by mass | 0.5% by mass or more |
| Comparative Example 7B | Comparative Example 7A | Less than 0.2% by mass | Less than 0.5% by mass | 0.5% by mass or more |
| Comparative Example 8B | Comparative Example 8A | Less than 0.2% by mass | Less than 0.5% by mass | 0.5% by mass or more |
| Comparative Example 9B | Comparative Example 9A | Less than 0.2% by mass | Less than 0.5% by mass | 0.5% by mass or more |
| Comparative Example 10B | Z-DOL | 0.2% by mass or more | Less than 0.5% by mass | Less than 0.5% by mass |
| Comparative Example 11B | Z-TETRAOL | 0.2% by mass or more | Less than 0.5% by mass | Less than 0.5% by mass |

Solubility of the ionic liquid of Example 6A to the fluorine-based solvent was 0.2% by mass or more, solubility of the ionic liquid of Example 6A to n-hexane was 0.5% by mass or more, and solubility of the ionic liquid of Example 6A to ethanol was 0.5% by mass or more.

Solubility of the ionic liquid of Example 7A to the fluorine-based solvent was 0.2% by mass or more, solubility of the ionic liquid of Example 7A to n-hexane was 0.5% by mass or more, and solubility of the ionic liquid of Example 7A to ethanol was 0.5% by mass or more.

Solubility of the ionic liquid of Example 8A to the fluorine-based solvent was 0.2% by mass or more, solubility of the ionic liquid of Example 8A to n-hexane was 0.5% by mass or more, and solubility of the ionic liquid of Example 8A to ethanol was 0.5% by mass or more.

Solubility of the ionic liquid of Example 9A to the fluorine-based solvent was 0.2% by mass or more, solubility of the ionic liquid of Example 9A to n-hexane was 0.5% by mass or more, and solubility of the ionic liquid of Example 9A to ethanol was 0.5% by mass or more.

Solubility of the ionic liquid of Example 10A to the fluorine-based solvent was 0.2% by mass or more, solubility of the ionic liquid of Example 10A to n-hexane was 0.5% by mass or more, and solubility of the ionic liquid of Example 10A to ethanol was 0.5% by mass or more.

Solubility of each of the ionic liquids of Comparative Example 6A to Comparative Example 9A to ethanol was 0.5% by mass or more. Meanwhile, solubility of each of the ionic liquids of Comparative Example 6A to Comparative Example 9A to the fluorine-based solvent was less than 0.2% by mass and solubility of each of the ionic liquids of Comparative Example 6A to Comparative Example 9A to n-hexane was less than 0.5% by mass. Solubility of the ionic liquid of Comparative Example 5A to the fluorine-based solvent was less than 0.2% by mass. Meanwhile, solubility of the ionic liquid of Comparative Example 5A to n-hexane and solubility of the ionic liquid of Comparative Example 5A to ethanol were 0.5% by mass or more. Solubility of Z-DOL to the fluorine-based solvent and solubility of Z-TETRAOL to the fluorine-based solvent were 0.2% by mass or more. Meanwhile, solubility of Z-DOL to ethanol, solubility of Z-DOL to n-hexane, solubility of Z-TETRAOL to ethanol, and solubility of Z-TETRAOL to n-hexane were less than 0.5% by mass.

As understood from the above, even when the same sulfonate salt having a high polarity is used, it is found that the ionic liquids presented in Comparative Examples, which include a sulfonate salt as a raw material, have a poor solubility to the fluorine-based solvent and the hydrocarbon-based solvent, while the sulfonate salt ionic liquids of Examples are considerably improved in the solubility. Considering that materials widely used as a lubricant are a long-chain fatty acid or an ester thereof, dissolution in hexane means that such ionic liquids can be effectively used as an additive due to its compatibility. Moreover, because the ionic liquids of Examples are dissolved in Vertrel XF that is a fluorine-based solvent, they are sufficient to be used for producing micromachines and hard discs. The onic liquids of Comparative Examples 10B and 11B, which have a perfluoropolyether skeleton, have a high solubility to the fluoride-based solvent, but have a poor solubility to the hydrocarbon-based solvent or alcohol. Therefore, application of such ionic liquids is limited.

Generally, it is difficult to expect solubility because an effect depending on a molecular structure is considerably complex. However, research results by the present inventors demonstrate that the imidazole-based ionic liquids having a long-chain alkyl group have a low solubility to Vertrel and n-hexane as understood from Comparative Example 6B and Comparative Example 7B, but ones into which a hydroxyl group is introduced in Examples 6B and 7B are improved in solubility.

As understood from Comparative Example 8B and Comparative Example 9B, it is found that the ammonium-based lubricants have a poor solubility because of a sulfonate salt and a sulfoimide salt, but such lubricants are improved in solubility by introduction of a hydroxyl group. That is, it is found that introduction of a hydroxyl group is effective in solubility to Vertrel or n-hexane as a molecular designing technique.

From research results by the present inventors, it was found that introduction of a hydroxyl group into the ionic liquid improved solubility to the fluorine-based solvent and the hydrocarbon-based solvent. In addition, many of ones including bis(nonafluorobutanesulfonyl)imide as an anion have higher solubility than ones including nonafluorobutanesulfonic acid.

### (Example 1C)

### <Thermal stability measurement results>

The 5% weight reduction temperature, the 10% weight reduction temperature, and the 20% weight reduction temperature of nonafluorobutanesulfonic acid-1-3'hydroxypropyl-3-octadecylimidazolium were 307.7°C, 357.2°C, and 388.2°C, respectively. It is found that the thermal stability is higher by 140°C or more compared to the commercially available perfluoropolyether Z-DOL (Comparative Example 10C), which was presented in Comparative Examples and is generally known as a lubricant used for magnetic recording media, and the thermal stability is higher by 60°C or more compared to Z-TETRAOL (Comparative Example 11C).

### (Example 2C)

### <Thermal stability measurement results>

The 5% weight reduction temperature, the 10% weight reduction temperature, and the 20% weight reduction temperature of bis(nonafluorobutanesulfonyl)imide1-3'hydroxypropyl-3-octadecylimidazolium were 327.7°C, 361.7°C, and 389.3°C, respectively. It is found that the thermal stability is improved by 150°C or more compared to the commercially available perfluoropolyether Z-DOL (Comparative Example 10C) and the thermal stability is improved by 90°C or more compared to the commercially available Z-TETRAOL (Comparative Example 11C).

### (Example 3C)

### <Thermal stability measurement results>

The 5% weight reduction temperature, the 10% weight reduction temperature, and the 20% weight reduction temperature of bis(nonafluorobutanesulfonyl)imide-N-3'hydroxypropyl-N-octadecylpyrrolidinum were 349.8°C, 370.8°C, and 387.6°C, respectively. It is found that the thermal stability is improved by 160°C or more compared to the commercially available perfluoropolyether Z-DOL (Comparative Example 10C) and the thermal stability is improved by 100°C or more compared to the commercially available Z-TETRAOL (Comparative Example 11C).

### (Example 4C)

### <Thermal stability measurement results>

The 5% weight reduction temperature, the 10% weight reduction temperature, and the 20% weight reduction temperature of nonafluorobutanesulfonic acid-6-octadecyl-8-3'-hydroxypropyl-1,8-diazabicyclo[5.4.0]-7-undecenium were 325.0°C, 356.5°C, and 390.1°C, respectively. It is found that the thermal stability is improved by 160°C or more compared to the commercially available perfluoropolyether Z-DOL (Comparative Example 10C) and the thermal stability is improved by 85°C or more compared to the commercially available Z-TETRAOL (Comparative Example 11C).

### (Example 5C)

### <Thermal stability measurement results>

The 5% weight reduction temperature, the 10% weight reduction temperature, and the 20% weight reduction temperature of bis(nonafluorobutanesulfonyl)imide-6-octadecyl-8-3'-hydroxypropyl-1,8-diazabicyclo[ 5.4.0]-7-undecenium were 326.2°C, 360.8°C, and 387.7°C, respectively. It is found that the thermal stability is higher by 160°C or more compared to the commercially available perfluoropolyether Z-DOL (Comparative Example 10C) and the thermal stability is higher by 85°C or more compared to the commercially available Z-TETRAOL (Comparative Example 11C).

### (Example 6C)

### <Thermal stability measurement results>

The 5% weight reduction temperature, the 10% weight reduction temperature, and the 20% weight reduction temperature of nonafluorobutanesulfonic acid-1-dodecyl-2-undecyl-3-3'hydroxypropylimidazolium were 335.4°C, 368.6°C, and 396.2°C, respectively. It is found that the thermal stability is higher by 170°C or more compared to the commercially available perfluoropolyether Z-DOL (Comparative Example 10C), which was presented in Comparative Examples and is generally known as a lubricant used for magnetic recording media, and the thermal stability is higher by 90°C or more compared to Z-TETRAOL (Comparative Example 11C).

### (Example 7C)

### <Thermal stability measurement results>

The 5% weight reduction temperature, the 10% weight reduction temperature, and the 20% weight reduction temperature of bis(nonafluorobutanesulfonyl)imide-1-dodecyl-2-undecyl-3-3'hydroxypropyloctadecyl imidazolium were 350.9°C, 373.7°C, and 394.8°C, respectively. It is found that the thermal stability is improved by 170°C or more compared to the commercially available perfluoropolyether Z-DOL (Comparative Example 10C) and the thermal stability is improved by 110°C or more compared to the commercially available Z-TETRAOL (Comparative Example 11C).

### (Example 8C)

### <Thermal stability measurement results>

The 5% weight reduction temperature, the 10% weight reduction temperature, and the 20% weight reduction temperature of bis(nonafluorobutanesulfonyl)imide-1-3'hydroxypropyl-2-heptadecyl-3-octadecylimid azolium were 327.8°C, 364.3°C, and 398.8°C°C, respectively. It is found that the thermal stability is improved by 160°C or more compared to the commercially available perfluoropolyether Z-DOL (Comparative Example 10C) and the thermal stability is improved by 85°C or more compared to the commercially available Z-TETRAOL (Comparative Example 11C).

### (Example 9C)

### <Thermal stability measurement results>

The 5% weight reduction temperature, the 10% weight reduction temperature, and the 20% weight reduction temperature of bis(nonafluorobutanesulfonyl)imide-N-3'hydroxypropyl-N,N-dimethyltetradecylam monium were 340.0°C, 359.0°C, and 375.0°C, respectively. It is found that the thermal stability is improved by 145°C or more compared to the commercially available perfluoropolyether Z-DOL (Comparative Example 10C) and the thermal stability is improved by 90°C or more compared to the commercially available Z-TETRAOL (Comparative Example 11C).

### (Example 10C)

### <Thermal stability measurement results>

The 5% weight reduction temperature, the 10% weight reduction temperature, and the 20% weight reduction temperature of bis(nonafluorobutanesulfonyl)imide-N-3'hydroxypropyl-N,N-dimethyloctadecylamm onium were 336.5°C, 359.4°C, and 376.6°C, respectively. It is found that the thermal stability is improved by 150°C or more compared to the commercially available perfluoropolyether Z-DOL (Comparative Example 10C) and the thermal stability is improved by 90°C or more compared to the commercially available Z-TETRAOL (Comparative Example 11C).

### (Comparative Example 1C)

### <Thermal stability measurement results>

The 5% weight reduction temperature, the 10% weight reduction temperature, and the 20% weight reduction temperature of nonafluorobutanesulfonic acid-1-octadecylimidazolium were 349.3°C, 375.0°C, and 397.5°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 2C)

### <Thermal stability measurement results>

The 5% weight reduction temperature, the 10% weight reduction temperature, and the 20% weight reduction temperature of hexafluorocyclopropane-1,3-bis(sulfonyl)imide-1-butyl-3-n-octadecylimidazolium were 347.2°C, 367.0°C, and 387.8°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 3C)

### <Thermal stability measurement results>

The 5% weight reduction temperature, the 10% weight reduction temperature, and the 20% weight reduction temperature of heptadecafluorooctanesulfonic acid-6-octadecyl-1,8-diazabicyclo[5.4.0]-7-undecenium were 361.9°C, 382.7°C, and 403.5°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 4C)

### <Thermal stability measurement results>

The 5% weight reduction temperature, the 10% weight reduction temperature, and the 20% weight reduction temperature of hexafluorocyclopropane-1,3-bis(sulfonyl)imide-6-octadecyl-1,8-diazabicyclo[5.4.0] -7-undecenium were 346.3°C, 384.1°C, and 414.0°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 5C)

### <Thermal stability measurement results>

The 5% weight reduction temperature, the 10% weight reduction temperature, and the 20% weight reduction temperature of pentadecafluorooctanoic acid octadecylammonium were 206.9°C, 215.8°C, and 223.4°C, respectively. Pentadecafluorooctanoic acid octadecylammonium is the ionic liquid. However, because a pKa of an acid thereof is more than 10, a binding force between ions is weak, resulting in poor thermal stability. In the case of this Comparative Example, although it is the ionic liquid, the thermal stability is not considerably improved compared to the commercially available perfluoropolyether Z-DOL (Comparative Example 10C) and Z-TETRAOL (Comparative Example 7C).

### (Comparative Example 6C)

### <Thermal stability measurement results>

The 5% weight reduction temperature, the 10% weight reduction temperature, and the 20% weight reduction temperature of nonafluorobutanesulfonic acid-1-octadecyl-2-heptadecylimidazolium were 338.2°C, 365.9°C, and 390.1°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 7C)

### <Thermal stability measurement results>

The 5% weight reduction temperature, the 10% weight reduction temperature, and the 20% weight reduction temperature of hexafluorocyclopropane-1,3-bis(sulfonyl)imide-1-octadecyl-2-heptadecylimidazolium were 303.7°C, 366.4°C, and 405.0°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 8C)

### <Thermal stability measurement results>

The 5% weight reduction temperature, the 10% weight reduction temperature, and the 20% weight reduction temperature of bis(nonafluorobutanesulfonyl)imide-octadecylammonium were 311.8°C, 329.8°C, and 348.0°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 9C)

### <Thermal stability measurement results>

The 5% weight reduction temperature, the 10% weight reduction temperature, and the 20% weight reduction temperature of nonafluorobutanesulfonic acid-N,N,N-trimethyloctadecylammonium were 340.5°C, 356.2°C, and 371.9°C, respectively. The thermal stability is higher because of the ionic liquid, compared to the commercially available perfluoropolyether Z-DOL (Comparative Example 10C) and Z-TETRAOL (Comparative Example 11C).

### (Comparative Example 10C)

### <Thermal stability measurement results>

When the commercially available perfluoropolyether Z-DOL having a hydroxyl group at the ends thereof and having a molecular weight of about 2,000 was measured as Comparative Example 10, the 5% weight reduction temperature, the 10% weight reduction temperature, and the 20% weight reduction temperature thereof were 165.0°C, 197.0°C, and 226.0°C, respectively. The weight reduction is attributed to evaporation.

### (Comparative Example 11C)

### <Thermal stability measurement results>

Perfluoropolyether (Z-TETRAOL) was used as a lubricant of Comparative Example 11C. Here, perfluoropolyether (Z-TETRAOL) is a commercially available product of the generally used lubricant for magnetic recording media, and has a plurality of hydroxyl groups at the ends thereof and a molecular weight of about 2,000. The 5% weight reduction temperature, the 10% weight reduction temperature, and the 20% weight reduction temperature thereof were 240.0°C, 261.0°C, and 282.0°C, respectively. Similarly to Z-DOL, the weight reduction is attributed to evaporation.

Results of Example 1C to Example 10C and Comparative Example 1C to Comparative Example 11C are summarized in Table 3, together with melting points.

**Table 3**

| Example | Synthesis Example or compound name | Weight reduction temperature (°C) | | | Melting point/°C |
|---|---|---|---|---|---|
| | | 5% | 10% | 20% | |
| Example 1C | Example 1A | 307.7 | 357.2 | 388.2 | 46.9 |
| Example 2C | Example 2A | 327.7 | 361.7 | 389.3 | <25.0 |
| Example 3C | Example 3A | 349.8 | 370.8 | 387.6 | <25.0 |
| Example 4C | Example 4A | 325.0 | 356.5 | 390.1 | <25.0 |
| Example 5C | Example 5A | 326.2 | 360.8 | 387.7 | <25.0 |
| Example 6C | Example 6A | 335.4 | 368.6 | 396.2 | <25.0 |
| Example 7C | Example 7A | 350.9 | 373.7 | 394.8 | <25.0 |
| Example 8C | Example 8A | 327.8 | 364.3 | 398.8 | <25.0 |
| Example 9C | Example 9A | 340.0 | 359.0 | 375.0 | <25.0 |
| Example 10C | Example 10A | 336.5 | 359.4 | 376.6 | <25.0 |
| Comparative Example 1C | Comparative Example 1A | 349.3 | 375.0 | 397.5 | 86.2 |
| Comparative Example 2C | Comparative Example 2A | 347.2 | 367.0 | 387.8 | 55.8 |
| Comparative Example 3C | Comparative Example 3A | 361.9 | 382.7 | 403.5 | 55.5 |
| Comparative Example 4C | Comparative Example 4A | 346.3 | 384.1 | 414.0 | 52.4 |
| Comparative Example 5C | Comparative Example 5A | 206.9 | 215.8 | 223.4 | 61.7 |
| Comparative Example 6C | Comparative Example 6A | 338.2 | 365.9 | 390.1 | 72.3 |
| Comparative Example 7C | Comparative Example 7A | 303.7 | 366.4 | 405.0 | 79.7 |
| Comparative Example 8C | Comparative Example 8A | 311.8 | 329.8 | 348.0 | 118.8 |
| Comparative Example 9C | Comparative Example 9A | 340.5 | 356.2 | 371.9 | 114.3 |
| Comparative Example 10C | Z-DOL | 165.0 | 197.0 | 226.0 | <25.0 |
| Comparative Example 11C | Z-TETRAOL | 240.0 | 261.0 | 282.0 | <25.0 |

As described above, it is found that the ionic liquid lubricants are overwhelmingly excellent in thermal stability other than the lubricant of Comparative Example 5C, compared to the commercially available perfluoropolyethers of Comparative Examples 10C and 11C.

Regarding comparison of the thermal stability in the ionic liquids, the imidazole-based ionic liquids of Examples 1C and 2C are compared with the imidazole-based ionic liquids of Comparative Examples 2C and 3C. The imidazole-based ionic liquids of Examples into which a hydroxyl group is introduced have lower 5% weight reduction temperature and lower 10% weight reduction temperature compared to the imidazole-based ionic liquids of Comparative Examples, and have substantially the same 20% weight reduction temperature. Therefore, it is considered that they have a sufficient thermal stability.

The pyrrolidine-based ionic liquids into which a hydroxyl group is introduced also exhibit substantially the same thermal stability as the imidazole-based ionic liquids.

Regarding ones having an octadecyl-1,8-diazabicyclo[5.4.0]-7-undecene structure, Examples 3C and 4C can be compared with Comparative Examples 3C and 4C. In this case, these Comparative Examples similarly exhibit higher weight reduction temperatures by about 20°C to about 430°C. However, the ionic liquid having an octadecyl-1,8-diazabicyclo[5.4.0]-7-undecene structure has a considerably high weight reduction temperature and has a 20% weight reduction temperature of about 390°C. Therefore, it is considered that such an ionic liquid has a sufficient thermal stability.

The weight reduction temperatures of Examples 6C to 10C and the weight reduction temperatures of Comparative Examples 6C to 9C do not vary significantly and are substantially identical to each other. Therefore, it is considered that such ionic liquids have a sufficient thermal stability.

Introduction of a hydroxyl group has a large effect on lowering a melting point. That is, the ionic liquids presented in Comparative Examples are a solid at 25°C because they have a long-chain alkyl group. However, all the ionic liquids including bis(nonafluorobutanesulfonyl)imide as a conjugate base and including a hydroxyl group, are a liquid at room temperature. Moreover, in the case of imidazole of Example 1A including sulfonate acid as a conjugate base, the melting point is lower compared to ones of Comparative Examples. A lubricant having a low melting point is advantageous because application of the lubricant can be widely expanded to other products other than hard discs. For example, it is expected to realize an increase in output efficiency by using a liquid-type lubricant in a high temperature extrusion·a high temperature forging process (Non-Patent Literature: Tribology Conference 2014, spring, proceedings).

### (Example 1D to Example 10D, Comparative Example 1D to Comparative Example 4D, and Comparative Examples 6D to 9D)

### <Disk durability test>

A lubricant including each of the ionic liquids of Example 1A to Example 10A, Comparative Example 1A to Comparative Example 4A, and Comparative Examples 6A to 9A was coated to prepare a magnetic disk. As presented in Table 4, the CSS measurement of the magnetic disk was more than 50,000 times, and the CSS measurement after the heating test was also more than 50,000 times, exhibiting excellent durability.

### (Comparative Example 5D)

### <Disk durability test>

A lubricant including pentadecafluorooctanoic acid octadecylammonium was used to prepare a magnetic disk as described above. As presented in Table 4, the CSS measurement of the magnetic disk was more than 50,000 times, but the CSS measurement after the heating test was 891 times. Therefore, the durability was deteriorated by the heating test. As presented in Comparative Example 5C, pentadecafluorooctanoic acid octadecylammonium is the ionic acid. However, because a pKa of an acid thereof is more than 10, it is considered that a binding force between ions is weak and thus the thermal stability is decreased, deteriorating the characteristic after the heating test.

### (Comparative Example 10D)

### <Disk durability test>

As described above, a magnetic disk was produced using a lubricant including Z-DOL. As presented in Table 4, the CSS measurement of the magnetic disk was more than 50,000 times, but the CSS measurement after the heating test was 12,000 times. Therefore, the durability was deteriorated by the heating test.

### (Comparative Example 11D)

### <Disk durability test>

As described above, a magnetic disk was produced using a lubricant including Z-TETRAOL. As presented in Table 4, the CSS measurement of the magnetic disk was more than 50,000 times, but the CSS measurement after the heating test was 36,000 times. Therefore, the durability was deteriorated by the heating test.

Results of Example 1D to Example 10D and Comparative Example ID to Comparative Example 11D are summarized in Table 4.

**Table 4**

| Example | Synthesis Example or compound name | CSS durability | | CSS durability after heating | |
|---|---|---|---|---|---|
| Example ID | Example 1A | 25°C, 60%RH | >50,000 | 25°C 60%RH | >50,000 |
| Example 2D | Example 2A | 25°C, 60%RH | >50,000 | 25°C, 60%RH | >50,000 |
| Example 3D | Example 3A | 25°C, 60%RH | >50,000 | 25°C, 60%RH | >50,000 |
| Example 4D | Example 4A | 25°C, 60%RH | >50,000 | 25°C 60%RH | >50,000 |
| Example 5D | Example 5A | 25°C, 60%RH | >50,000 | 25°C, 60%RH | >50,000 |
| Example 6D | Example 6A | 25°C, 60%RH | >50,000 | 25°C, 60%RH | >50,000 |
| Example 7D | Example 7A | 25°C, 60%RH | >50,000 | 25°C, 60%RH | >50,000 |
| Example 8D | Example 8A | 25°C, 60%RH | >50,000 | 25°C, 60%RH | >50,000 |
| Example 9D | Example 9A | 25°C, 60%RH | >50,000 | 25°C 60%RH | >50,000 |
| Example 10D | Example 10A | 25°C, 60%RH | >50,000 | 25°C, 60%RH | >50,000 |
| Comparative Example 1D | Comparative Example 1A | 25°C, 60%RH | >50,000 | 25°C, 60%RH | >50,000 |
| Comparative Example 2D | Comparative Example 2A | 25°C, 60%RH | >50,000 | 25°C, 60%RH | >50,000 |
| Comparative Example 3D | Comparative Example 3A | 25°C, 60%RH | >50,000 | 25°C, 60%RH | >50,000 |
| Comparative Example 4D | Comparative Example 4A | 25°C, 60%RH | >50,000 | 25°C, 60%RH | >50,000 |
| Comparative Example 5D | Comparative Example 5A | 25°C, 60%RH | >50,000 | 25°C, 60%RH | 891 |
| Comparative Example 6D | Comparative Example 6A | 25°C, 60%RH | >50,000 | 25°C, 60%RH | >50,000 |
| Comparative Example 7D | Comparative Example 7A | 25°C, 60%RH | >50,000 | 25°C, 60%RH | >50,000 |
| Comparative Example 8D | Comparative Example 8A | 25°C, 60%RH | >50,000 | 25°C, 60%RH | >50,000 |
| Comparative Example 9D | Comparative Example 9A | 25°C, 60%RH | >50,000 | 25°C, 60%RH | >50,000 |
| Comparative Example 10D | Z-DOL | 25°C, 60%RH | >50,000 | 25°C, 60%RH | 12,000 |
| Comparative Example 11D | Z-Tetraol | 25°C, 60%RH | >50,000 | 25°C, 60%RH | 36,000 |

### (Example 1E to Example 10E and Comparative Example 1E to Comparative Example 11E)

A lubricant including each of the ionic liquids of Examples 1A to 10A, the ionic liquids of Comparative Examples 1A to 9A, the Z-DOL, and the Z-Tetraol was used to prepare a magnetic tape as described above. Then, the following measurements were performed.
- Coefficient of friction of magnetic tape after shuttle run of 100 times:
   In the environment of a temperature of -5°C, or in the environment of a temperature of 40°C and relative humidity of 90%.
- Still durability test:
   In the environment of a temperature of -5°C, or in the environment of a temperature of 40°C and relative humidity of 30%.
- Shuttle durability test:
   In the environment of a temperature of -5°C, or in the environment of a temperature of 40°C and relative humidity of 90%.
- Coefficient of friction of magnetic tape after shuttle run of 100 times after heating test:
   In the environment of a temperature of -5°C, or in the environment of a temperature of 40°C and relative humidity of 90%.
- Still durability test after heating test:
   In the environment of a temperature of -5°C, or in the environment of a temperature of 40°C and relative humidity of 30%.
- Shuttle durability test after heating test:
   In the environment of a temperature of -5°C, or in the environment of a temperature of 40°C and relative humidity of 90%.

Results of Examples 1E to 10E and Comparative Examples 1E to 11E are summarized in Table 5-1 and Table 5-2.

**Table 5-1**

| | Compound | Friction coefficient after 100 runs | | Still durability /min | | Shuttle durability /times | | Friction coefficient after 100 runs after heating | | Still durability after heating/min | | Shuttle durability after heating/times | |
|---|---|---|---|---|---|---|---|---|---|---|---|---|---|
| Example 1E | Example 1A | -5°C | 0.21 | -5°C | >60 | -5°C | >200 | -5°C | 0.22 | -5°C | >60 | -5°C | >200 |
| | | 40°C , 90%RH | 0.24 | 40°C, 30%RH | >60 | 40°C, 90%RH | >200 | 40°C, 90%RH | 0.25 | 40°C, 30%RH | >60 | 40°C, 90%RH | >200 |
| Example 2E | Example 2A | -5°C | 0.22 | -5°C | >60 | -5°C | >200 | -5°C | 0.23 | -5°C | >60 | -5°C | >200 |
| | | 40°C, 90%RH | 0.25 | 40°C, 30%RH | >60 | 40°C, 90%RH | >200 | 40°C, 90%RH | 0.26 | 40°C, 30%RH | >60 | 40°C, 90%RH | >200 |
| Example 3E | Example 3A | -5°C | 0.22 | -5°C | >60 | -5°C | >200 | -5°C | 0.25 | -5°C | >60 | -5°C | >200 |
| | | 40°C, 90%RH | 0.26 | 40°C, 30%RH | >60 | 40°C, 90%RH | >200 | 40°C, 90%RH | 0.28 | 40°C, 30%RH | >60 | 40°c, 90%RH | >200 |
| Example 4E | Example 4A | -5°C | 0.22 | -5°C | >60 | -5°C | >200 | -5°C | 0.24 | -5°C | >60 | -5°C | >200 |
| | | 40°C, 90%RH | 0.25 | 40°C, 30%RH | >60 | 40°C, 90%RH | >200 | 40°C, 90%RH | 0.26 | 40°C, 30%RH | >60 | 40°C, 90%RH | >200 |
| Example 5E | Example 5A | -5°C | 0.24 | -5°C | >60 | -5°C | >200 | -5°C | 0.25 | -5°C | >60 | -5°C | >200 |
| | | 40°C, 90%RH | 0.26 | 40°C, 30%RH | >60 | 40°C, 90%RH | >200 | 40°C, 90%RH | 0.27 | 40°C, 30%RH | >60 | 40°C, 90%RH | >200 |
| Example 6E | Example 6A | -5°C | 0.23 | -5°C | >60 | -5°C | >200 | -5°C | 0.23 | -5°C | >60 | -5°C | >200 |
| | | 40°C, 90%RH | 0.24 | 40°C, 30%RH | >60 | 40°C, 90%RH | >200 | 40°C, 90%RH | 0.25 | 40°C, 30%RH | >60 | 40°C, 90%RH | >200 |
| Example 7E | Example 7A | -5°C | 0.24 | -5°C | >60 | -5°C | >200 | -5°C | 0.24 | -5°C | >60 | -5°C | >200 |
| | | 40°C, 90%RH | 0.25 | 40°C, 30%RH | >60 | 40°C, 90%RH | >200 | 40°C, 90%RH | 0.26 | 40°C, 30%RH | >60 | 40°C, 90%RH | >200 |
| Example 8E | Example 8A | -5°C | 0.21 | -5°C | >60 | -5°C | >200 | -5°C | 0.21 | -5°C | >60 | -5°C | >200 |
| | | 40°C, 90%RH | 0.22 | 40°C, 30%RH | >60 | 40°C, 90%RH | >200 | 40°C, 90%RH | 0.23 | 40°C, 30%RH | >60 | 40°C, 90%RH | >200 |
| Example 9E | Example 9A | -5°C | 0.24 | -5°C | >60 | -5°C | >200 | -5°C | 0.24 | -5°C | >60 | -5°C | >200 |
| | | 40°C. 90%RH | 0.25 | 40°C, 30%RH | >60 | 40°C, 90%RH | >200 | 40°C, 90%RH | 0.26 | 40°C, 30%RH | >60 | 40°C, 90%RH | >200 |
| Example 10E | Example 10A | -5°C | 0.19 | -5°C | >60 | -5°C | >200 | -5°C | 0.20 | -5°C | >60 | -5°C | >200 |
| | | 40°C, 90%RH | 0.21 | 40°C, 30%RH | >60 | 40°C, 90%RH | >200 | 40°C, 90%RH | 0.21 | 40°C. 30%RH | >60 | 40°C, 90%RH | >200 |

**Table 5-2**

| | Compound | Friction coefficient after 100 runs | | Still durability /min | | Shuttle durability /times | | Friction coefficient after 100 runs after heating | | Still durability after heating/min | | Shuttle durability after heating/times | |
|---|---|---|---|---|---|---|---|---|---|---|---|---|---|
| Comparative Example 1E | Comparative Example 1A | -5°C | 0.20 | -5°C | >60 | -5°C | >200 | -5°C | 0.20 | -5°C | >60 | -5°C | >200 |
| | | 40°C. 90%RH | 0.22 | 40°C, 30%RH | >60 | 40°C, 90%RH | >200 | 40°C, 90%RH | 0.23 | 40°C, 30%RH | >60 | 40°C, 90%RH | >200 |
| Comparative Example 2E | Comparative Example 2A | -5°C | 0.23 | -5°C | >60 | -5°C | >200 | -5°C | 0.25 | -5°C | >60 | -5°C | >200 |
| | | 40°C, 90%RH | 0.25 | 40°C, 30%RH | >60 | 40°C, 90%RH | >200 | 40°C, 90%RH | 0.27 | 40°C, 30%RH | >60 | 40°C, 90%RH | >200 |
| Comparative Example 3E | Comparative Example 3A | -5°C | 0.22 | -5°C | >60 | -5°C | >200 | -5°C | 0.24 | -5°C | >60 | -5°C | >200 |
| | | 40°C, 90%RH | 0.26 | 40°C, 30%RH | >60 | 40°C, 90%RH | >200 | 40°C, 90%RH | 0.26 | 40°C, 30%RH | >60 | 40°C, 90%RH | >200 |
| Comparative Example 4E | Comparative Example 4A | -5°C | 0.23 | -5°C | >60 | -5°C | >200 | -5°C | 0.24 | -5°C | >60 | -5°C | >200 |
| | | 40°C, 90%RH | 0.26 | 40°C, 30%RH | >60 | 40°C, 90%RH | >200 | 40°C, 90%RH | 0.27 | 40°C, 30%RH | >60 | 40°C, 90%RH | >200 |
| Comparative Example 5E | Comparative Example 5A | -5°C | 0.21 | -5°C | >60 | -5°C | >200 | -5°C | 0.45 | -5°C | 12 | -5°C | 30 |
| | | 40°C, 90%RH | 0.25 | 40°C, 30%RH | >60 | 40°C, 90%RH | >200 | 40°C, 90%RH | 0.51 | 40°C, 30%RH | 16 | 40°C, 90%RH | 23 |
| Comparative Example 6E | Comparative Example 6A | -5°C | 0.20 | -5°C | >60 | -5°C | >200 | -5°C | 0.21 | -5°C | >60 | -5°C | >200 |
| | | 40°C, 90%RH | 0.22 | 40°C, 30%RH | >60 | 40°C, 90%RH | >200 | 40°C, 90%RH | 0.23 | 40°C, 30%RH | >60 | 40°C, 90%RH | >200 |
| Comparative Example 7E | Comparative Example 7A | -5°C | 0.20 | -5°C | >60 | -5°C | >200 | -5°C | 0.22 | -5°C | >60 | -5°C | >200 |
| | | 40°C, 90%RH | 0.22 | 40°C, 30%RH | >60 | 40°C, 90%RH | >200 | 40°C, 90%RH | 0.23 | 40°C, 30%RH | >60 | 40°C, 90%RH | >200 |
| Comparative Example 8E | Comparative Example 8A | -5°C | 0.19 | -5°C | >60 | -5°C | >200 | -5°C | 0.20 | -5°C | >60 | -5°C | >200 |
| | | 40°C, 90%RH | 0.20 | 40°C, 30%RH | >60 | 40°C, 90%RH | >200 | 40°C, 90%RH | 0.20 | 40°C, 30%RH | >60 | 40°C, 90%RH | >200 |
| Comparative Example 9E | Comparative Example 9A | -5°C | 0.18 | -5°C | >60 | -5°C | >200 | -5°C | 0.19 | -5°C | >60 | -5°C | >200 |
| | | 40°C, 90%RH | 0.19 | 40°C, 30%RH | >60 | 40°C, 90%RH | >200 | 40°C, 90%RH | 0.19 | 40°C, 30%RH | >60 | 40°C, 90%RH | >200 |
| Comparative Example 10E | Z-DOL | -5°C | 0.25 | -5°C | 12 | -5°C | 59 | -5°C | 0.32 | -5°C | 12 | -5°C | 46 |
| | | 40°C, 90%RH | 0.30 | 40°C, 30%RH | 48 | 40°C, 90%RH | 124 | 40°C, 90%RH | 0.35 | 40°C, 30%RH | 15 | 40°C, 90%RH | 58 |
| Comparative Example 11E | Z-Tetraol | -5°C | 0.22 | -5°C | 25 | -5°C | 65 | -5°C | 0.28 | -5°C | 23 | -5°C | 55 |
| | | 40°C, 90%RH | 0.26 | 40°C, 30%RH | 35 | 40°C, 90%RH | 156 | 40°C, 90%RH | 0.32 | 40°C, 30%RH | 31 | 40°C, 90%RH | 126 |

In Tables, ">60" of the still durability denotes more than 60 minutes.

In Tables, ">200" of the shuttle durability denotes more than 200 times.

The following facts can be confirmed.

It was found that the magnetic tape to which the lubricant including each of the ionic liquids of Example 1A to Example 10A was applied had excellent friction properties, still durability, and shuttle durability.

It was found that the magnetic tape to which the lubricant including each of the ionic liquids of Comparative Example 1A to Comparative Example 4A and Comparative Examples 6A to 9A was applied had excellent friction properties, still durability, and shuttle durability. The lubricants of Comparative Examples were each the ionic liquid and thus exhibited an excellent magnetic tape durability after the heating test.

It was found that the magnetic tape to which the lubricant including the ionic liquid of Comparative Example 5A was applied had excellent friction properties, still durability, and shuttle durability. The lubricant of this Comparative Example was considerably deteriorated in the magnetic tape durability after the heating test.

It was found that the magnetic tape to which Z-DOL was applied was considerably deteriorated in still durability and shuttle durability.

It was found that the magnetic tape to which Z-Tetraol was applied was considerably deteriorated in still durability and shuttle durability.

From Table 5-1 and Table 5-2, it was found that when an ionic liquid lubricant that includes an ionic liquid including a conjugate base and a conjugate acid, where the conjugate acid includes a group including a hydroxyl group and a group including a straight-chain hydrocarbon group having 6 or greater carbon atoms, and a pKa of an acid that is a source of the conjugate base in acetonitrile is 10 or less, was used, it is possible to obtain excellent thermal resistance and durability in the magnetic tape and the magnetic disk. Moreover, the ionic liquid lubricants are excellent in thermal resistance and durability of the magnetic recording media. In addition, some of the ionic liquid lubricants are dissolved in Vertrel that is a fluorine-based solvent. Therefore, particularly, when application to hard discs is considered, such ionic liquid lubricants do not have a problem in the production process.

As is clear from the above description, an ionic liquid lubricant that includes an ionic liquid including a conjugate base and a conjugate acid, where the conjugate acid includes a group including a hydroxyl group and a group including a straight-chain hydrocarbon group having 6 or greater carbon atoms, and a pKa of an acid that is a source of the conjugate base in acetonitrile is 10 or less, has high decomposition temperature, high 5% weight reduction temperature, high 10% weight reduction temperature, and high 20% weight reduction temperature, and has an excellent thermal stability. Even under high temperature conditions, the ionic liquid lubricant can maintain excellent lubricity and can maintain lubricity for a long period of time, compared to the conventional perfluoropolyether. Therefore, a magnetic recording medium using the lubricant including the ionic liquid can attain considerably excellent running performances, abrasion resistance, and durability.

### Reference Sings List

11: substrate
12: base layer
13: magnetic layer
14: carbon protective layer
15: lubricant layer
21: substrate
22: magnetic layer
23: carbon protective layer
24: lubricant layer
25: back-coating layer

## Claims

1. A lubricant comprising:
an ionic liquid including a conjugate base and a conjugate acid,
wherein the conjugate acid includes a group including a hydroxyl group and a group including a straight-chain hydrocarbon group having 6 or greater carbon atoms, and a pKa of an acid that is a source of the conjugate base in acetonitrile is 10 or less.

2. The lubricant according to claim 1, wherein the conjugate acid is represented by General Formula (A) below, General Formula (B) below, General Formula (C) below, or General Formula (D) below: 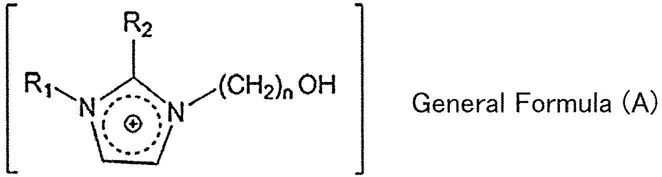 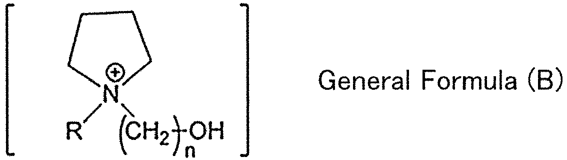 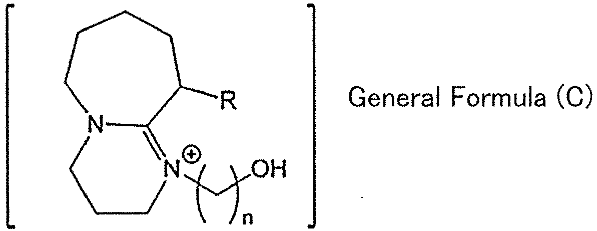 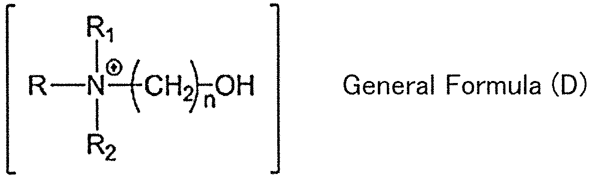 where in the General Formula (A), R₁ and R₂ each independently represent a hydrogen atom or a group including a straight-chain hydrocarbon group having 6 or greater carbon atoms, n is an integer of 1 or greater, and at least one of R₁ and R₂ is a group including a straight-chain hydrocarbon group having 6 or greater carbon atoms,
in the General Formula (B), R represents a group including a straight-chain hydrocarbon group having 6 or greater carbon atoms, and n is an integer of 1 or greater,
in the General Formula (C), R represents a group including a straight-chain hydrocarbon group having 6 or greater carbon atoms, and n is an integer of 1 or greater, and
in the General Formula (D), R represents a group including a straight-chain hydrocarbon group having 6 or greater carbon atoms, R₁ and R₂ each independently represent a hydrogen atom or a hydrocarbon group, and n is an integer of 1 or greater.

3. The lubricant according to claim 1 or 2, wherein the conjugate base is represented by General Formula (X) below or General Formula (Y) below:
CₗF₂ₗ₊₁-SO₃^{Θ} General Formula (X) 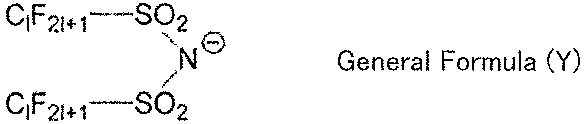
where in the General Formula (X), 1 is an integer of 1 or greater but 12 or less, and
in the General Formula (Y), 1 is an integer of 1 or greater but 12 or less.

4. A magnetic recording medium comprising:
a non-magnetic support;
a magnetic layer disposed on the non-magnetic support; and
the lubricant according to any one of claims 1 to 3, disposed on the magnetic layer.

5. An ionic liquid comprising:
a conjugate base; and
a conjugate acid,
wherein the conjugate acid includes a group including a hydroxyl group and a group including a straight-chain hydrocarbon group having 6 or greater carbon atoms, and a pKa of an acid that is a source of the conjugate base in acetonitrile is 10 or less.

6. The ionic liquid according to claim 5, wherein the conjugate acid is represented by General Formula (A) below, General Formula (B) below, General Formula (C) below, or General Formula (D) below: 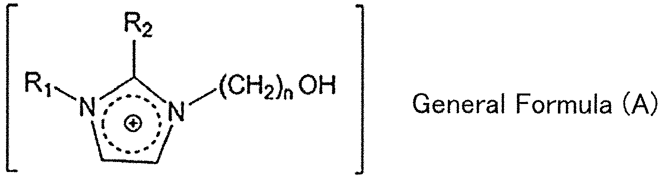 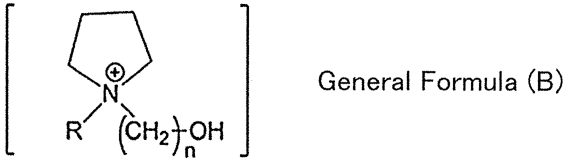 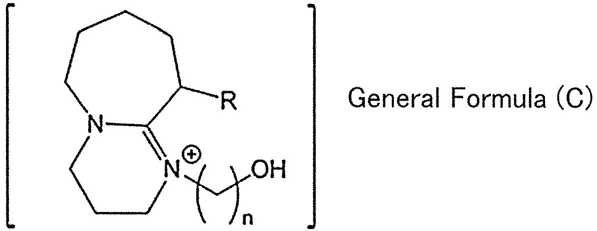 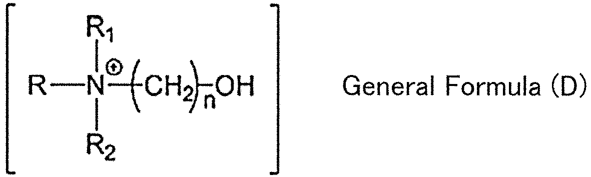 where in the General Formula (A), R₁ and R₂ each independently represent a hydrogen atom or a group including a straight-chain hydrocarbon group having 6 or greater carbon atoms, n is an integer of 1 or greater, and at least one of R₁ and R₂ is a group including a straight-chain hydrocarbon group having 6 or greater carbon atoms,
in the General Formula (B), R represents a group including a straight-chain hydrocarbon group having 6 or greater carbon atoms, and n is an integer of 1 or greater,
in the General Formula (C), R represents a group including a straight-chain hydrocarbon group having 6 or greater carbon atoms, and n is an integer of 1 or greater, and
in the General Formula (D), R represents a group including a straight-chain hydrocarbon group having 6 or greater carbon atoms, R₁ and R₂ each independently represent a hydrogen atom or a hydrocarbon group, and n is an integer of 1 or greater.

7. The ionic liquid according to claim 5 or 6, wherein the conjugate base is represented by General Formula (X) below or General Formula (Y) below:
CₗF₂ₗ₊₁-SO₃^{Θ} General Formula (X) 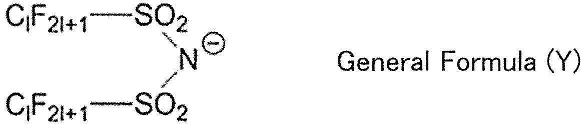
where in the General Formula (X), 1 is an integer of 1 or greater but 12 or less, and
in the General Formula (Y), 1 is an integer of 1 or greater but 12 or less.
